# EUROPEAN PATENT SPECIFICATION

(11) **EP 2 164 993 B1**
(45) Date of publication and mention of the grant of the patent: **21.09.2016**
(21) Application number: 08769761.1
(22) Date of filing: 28.05.2008
(51) Int. Cl.: C12Q 1/68, C12N 15/00, A61K 39/21

(54) **METHODS FOR PRODUCTION AND USES OF MULTIPOTENT CELL POPULATIONS, PLURIPOTENT CELL POPULATIONS, DIFFERENTIATED CELL POPULATIONS, AND HIV-RESISTANT CELL POPULATIONS**
HERSTELLUNGSVERFAHREN UND VERWENDUNGEN FÜR MULTIPOTENTE ZELLPOPULATIONEN, PLURIPOTENTE ZELLPOPULATIONEN, AUSDIFFERENZIERTE ZELLPOPULATIONEN UND HIV-RESISTENTE ZELLPOPULATIONEN
PROCÉDÉS DE PRODUCTION ET D'UTILISATIONS DE POPULATIONS DE CELLULES MULTIPOTENTES, PLURIPOTENTES, DIFFÉRENTIÉES ET RÉSISTANTES AU VIH

(30) Priority: 29.05.2007 US 932020 P; 05.06.2007 US 933133 P; 08.06.2007 US 933670 P; 14.01.2008 US 6449 P; 25.03.2008 US 64761 P
(43) Date of publication of application: 24.03.2010
(73) Proprietor: Reid, Christopher B., Los Angeles, CA 90012 (US)
(72) Inventor: Reid, Christopher B., Los Angeles, CA 90012 (US)
(74) Representative: Lapienis, Juozas
(86) International application number: PCT/US2008/065007
(87) International publication number: WO 2008/150814

(56) References cited:
- US-A1- 2003 012 769
- US-A1- 2006 269 518
- US-B2- 6 733 993
- VERDI JOSEPH M ET AL: "Distinct human NUMB isoforms regulate differentiation vs. proliferation in the neuronal lineage", PROCEEDINGS OF THE NATIONAL ACADEMY OF SCIENCES OF THE UNITED STATES OF AMERICA, vol. 96, no. 18, 31 August 1999 (1999-08-31), pages 10472-10476, XP002614311, ISSN: 0027-8424
- BANI-YAGHOUB MAHMUD ET AL: "A switch in numb Isoforms is a critical step in cortical development", DEVELOPMENTAL DYNAMICS, vol. 236, no. 3, March 2007 (2007-03), pages 696-705, XP002614312, ISSN: 1058-8388
- KAOMEI GUAN ET AL: "Embryonic stem cell differentiation models: cardiogenesis, myogenesis, neurogenesis, epithelial and vascular smooth muscle cell differentiation in vitro", CYTOTECHNOLOGY, KLUWER ACADEMIC PUBLISHERS, DO, vol. 30, no. 1-3, 1 May 1999 (1999-05-01), pages 211-226, XP019236607, ISSN: 1573-0778, DOI: DOI:10.1023/A:1008041420166
- CHAPMAN GAVIN ET AL: "High levels of Notch signaling down-regulate Numb and Numblike", JOURNAL OF CELL BIOLOGY, vol. 175, no. 4, November 2006 (2006-11), pages 535-540, ISSN: 0021-9525

## Description

### BACKGROUND OF THE INVENTION

The transcendent challenge for medicine in the 21 st century will be replacing damaged, worn-out or genetically-compromised cells. Transcription factors binding specifically to DNA play a vital role in regulating gene expression. It is the particular complement of transcription factors within an individual cell, that determine which cellular programs are active and which are turned off. In this capacity transcription factors play a decisive role in determining and maintaining cellular identity, as well as determining cellular vulnerability.

A report written by VERDI JOSEPH M ET AL: "Distinct human NUMB isoforms regulate differentiation vs. proliferation in the neuronal lineage", published on 31 August 1999, discloses four different isoforms of Numb, and their differential expression during neural development. Each isoform was separately expressed in three cell lines (pluripotent murine P19 embryonic carcinoma cells and immortalized neural crest stem cells (MONC-1), and primary neural crest stem cells) to determine their distinct functions. The long (PRR^{L}) Numb isoforms were found to promote proliferation of undifferentiated, stem/progenitor cells, but not their differentiation.

A report written by CHAPMAN GAVIN ET AL: "High levels of Notch signaling down-regulate Numb and Numblike", published on November 2006, demonstrates a complex relationship between Notch and the two vertebrate Numb homologues Numb and Numblike. Although Numb and Numblike at low levels of Notch signaling negatively regulated Notch, high levels of Notch signaling conversely led to a reduction of Numb and Numblike protein levels in cultured cells and in the developing chick central nervous system. However, Chapman et al., 2006 is exclusively directed to Numblike and short PRR- Numb, but is silent with respect to long PRR+ Numb

A report written by BANI-YAGHOUB MAHMUD ET AL: "A switch in numb Isoforms is a critical step in cortical development", published in March 2007, discloses the switch from long (PRR+) to short (PRR-) Numb expression in cortical progenitor cells. It also discloses that the long Numb isoforms were inactivated by antisense morpholino in neuronal stem cell cultures. The expression of the long Numb isoforms resulted in a significant reduction of neural differentiation, correlating with an expansion of the cortical progenitor pool.

### SUMMARY OF THE INVENTION

The ability to derive proliferating, self-renewing, multipotent and pluripotent cell population(s) from otherwise non-pluripotent, non-self renewing cells may have significant positive implications for all fields utilizing cellular therapies. These fields include bone marrow transplantation, transfusion medicine, and gene therapy and enable the production of patient-specific stem cells and other desired cell types. Likewise, the ability to initiate differentiation of cells into neural, muscle, and various other desirable cell populations is and will also be of significant value to medicine and commercial processes involving animals. Accordingly, the present invention provides methods for genetic production and uses of multipotent cell populations, pluripotent cell populations, neuronal cell populations, muscle cell populations, and other desired cell populations such as, for example, HIV resistant cell populations.

It is a proposition of the present invention that the efficient introduction or overexpression of specific transcription factors, alone or in combination with other cell fate determinants (such as notch, numb and numblike), enables the interconversion of what have been considered transitory (multipotent, pluripotent, and/or self-renewing) or fixed (differentiated or somatic) cellular phenotypes. The ability to reliably induce phenotypic conversion or cellular reprogramming allows the production of stem cells, replacement cells, tissues, and organs that match individual patients. In conjunction with gene therapy techniques and cell culture techniques, cell type interconversion also provides for the production of disease-resistant and genetically-repaired cells that are suitable for transplantation.

It is an object of this invention to provide various manners of generating proliferating, self-renewing, multipotent and/or pluripotent cell population(s), as well as other desirable cell populations, from either dividing or non-dividing cells without the use of oncogenes. Differentiating cell populations comprise cells expressing some, but not all markers associated with specific cell type categorization. It is disclosed herein that appropriate Numb isoform expression in combination with other transgenes (especially transcription factors) enables the production of dividing, pluripotent cell populations or differentiating cell populations.

The invention may be used with any suitable cells, including vertebrate cells, and including fish, mammalian, avian, amphibian, and reptilian cells.

### DETAILED DESCRIPTION

A nucleic acid sequence (e.g., a nucleic acid seqeuence encoding a polypeptide) is termed "operably linked" to another nucleic acid sequence (e.g., a promoter) when the first nucleic acid sequence is placed in a functional relationship with the second nuceleic acid sequence. For example, a promoter is operably linked to a coding sequence if the promoter affects the transcription or expression of the coding sequence. As used herein, the term "driven by" refers to a gene or coding sequence that is operably linked to a promoter sequence, and that the promoter sequence affects the transcription or expression of the coding sequence.

A theoretical basis for the embodiments of the invention is described herein, however, this discussion is not in any way to be considered as binding or limiting on the present invention. Those of skill in the art will understand that the various embodiments of the invention may be practiced regardless of the model used to describe the theoretical underpinnings of the invention.

In a preferred embodiment, cells are "selected" from accessible, dividing or non-dividing cell populations for the purpose of generating the desired a) proliferating, multipotent or pluripotent cell population, differentiating b) populations of neuronal cells c) muscle cells, d) and/or any other desired cell population; moreover the desired cell population may be capable of further differentiation in vitro, in vivo, and/or tissue-appropriate and regionally-appropriate differentiation in vivo.

### Sources of cells selected for use in the invention:

Selected cells may include any cell practicable in the present invention. Cells selected for use in the present invention (herein termed "selected cells") may originate as endogenous cells of the patient --including cells derived from other organ systems; or from exogenous sources (including those derived from cell lines, cryopreserved sources, banked sources, and donors). Cells may also be selected from cells genetically-modified with synthetic or natural nucleic acid sequences. The term "selected cells" as used herein does not include human embryonic stem cells.

In embodiments of the present invention, in order that they may be isolated without the involvement of invasive procedures, selected cells will preferably be easily accessible cells (e.g. peripheral blood leukocytes, circulating hematopoietic stem cells, epithelial cells (e.g. buccal cheek cells), adipose tissue, umbilical cord blood cells, etc.). However, bone marrow stem cells, , primordial germ cells (PGCs), stem cells isolated from amniotic membranes, amniotic fluid, as well as cells isolated from the skin etc., are also covered by the present invention. Such cells can be isolated from the tissues in which they reside by any means known to the art.

The selected cells may be genetically-modified cells, especially cells that have been genetically modified by any means known to the art, to encode therapeutic or commercially useful deoxyribonucleic acid (DNA) or ribonucleic acid (RNA) sequences.

In accordance with an aspect of the present invention, there is provided a method of producing a desired cell population (e.g. pluripotent, neuronal, muscle, etc.) from the selected cells.

### Achieving multipotent, pluripotent, and/or self renewing cell populations:

In order to achieve a) a population of proliferating, self renewing pluripotent cells, the selected cell(s) and/or their progeny are transfected with nucleotide sequence(s) encoding the "long" (PRR insert +) isoform(s) of the mammalian numb gene and with additional nucleotide sequence encoding Notch. Then they are cultured under conditions which promote growth of the selected cells at an optimal growth rate. Selected cells are maintained under these conditions for the period of time sufficient to achieve the desired cell number.

The cells are grown at the (optimal) rate of growth achieved by incubation in a growth medium comp[rising cytokines selected from EGF, bFGF, LIF, steel factor, and/or equipotent concentrations of IL-6, hyper IL-6, IL-7, oncostatin-M and/or cardiotrophin-1; or that growth rate achieved in the presence of other growth enhancing cytokines (e.g. those conditions described for culturing pluripotent cells e.g. Guan et al., 2006). The growth rate is determined from the doubling times of the selected cells in said growth culture medium. Likewise, culture conditions such as those described in US Patents 6432711 and 5453357 may also be suitable for the propagation and expansion, at an optimal growth rate, of cells transfected with the long (PRR+) Numb isioform(s). Other appropriate protocols and reference cytokine concentrations have been taught by Koshimizu et al., 1996; Keller et al., 1996; Piquet-Pellorce, 1994; Rose et al., 1994; Park and Han, 2000; Guan et al., 2006; Dykstra et al., 2006; Zhang et al., 2007). However the practice of the present invention is not limited to the details of these teachings.

In a separate preferred embodiment, other nucleic acid(s) or protein(s) are utilized in concert with the nucleic acid(s) or protein(s) corresponding to Long (PRR+) Numb isoforms so long as a population of multipotent, pluripotent, and/or self-renewing cells is produced from the selected cells and the method is electroporation, liposomes, nanocapsules, nanovaults, and/or another approach avoiding retroviral/lentiviral integration or other random alteration of the cell's genome.

It is to be understood that any combination of nucleic acid or protein sequences described herein can be modified by excluding those corresponding to Numb and/or Numblike so long as the desired cell population or behavior is achieved.

In a preferred embodiment, the transfection steps described herein represent transient transfection.

Such transient transfection is accomplished using viral vectors that do not integrate into the host genome.

In another preferred embodiment, such transient transfection is accomplished using standard transfection techniques (electroporation, chemically mediated transfection, fusogenic or non-fusogenic liposomes, nanocapsules, nanovaults, etc.).

Over time, other gene combinations differing from those described herein may be described or discovered capable of causing cells to become multipotent, pluripotent, capable of self-renewal or to begin differentiating. However this patent application also covers the genetic reprogramming of any nucleated cell utilizing nucleic acid or protein electroporation (for example methods see Gagne et al., 1991; Saito et al., 2001; Yuan, 2008; Huang et al., 2007; Xia and Zhang, 2007; Cemazar and Sersa 2007; Isaka and Imai, 2007; Luxembourg et al., 2007; Van Tendeloos, 2007; Takahashi, 2007; etc.) electroporation, liposomes, nanocapsules, nanovaults, and/or another approach avoiding viral integration or other random alteration of the cell's genome as such means increase safety and efficiency.

### Assessment of Potency and Differentiation

Pluripotency and multipotency can be assessed by any means known to the art including 1) transplantation, 2) culture under conditions promoting embryoid body formation, 3) injection of cells into animal blastocyst stage embryos with subsequent development, and 4) RNA expression assays (e.g. RT-PCR and microarray based analyses) for gene expression associated with differentiation, multipotency, pluripotency, etc. (see Guan et al., 2006), 5) colony-formation, as well as by ES-like morphology. One approach disclosed herein for detecting pluripotency in selected cells and/or their progeny involves transfection with a reporter construct comprising the Nanog promoter operably linked to a fluorescent protein gene. This allows identification and enrichment of Nanog expressing cells using Fluorescence Activated Cell Sorting (FACS), etc.

In a preferred embodiment, endogenous cells (e.g. cells surrounding a burn or injury site) are transfected in vivo with genetic vectors encoding the long (PRR+) numb isoform(s) alone or in conjuction with other transgenes named herein to transiently promote renewed or increased cell proliferation. This approach can also be utilized clinically in the setting of hypoplastic tissues, disorders where stem/progenitor cells are abnormally depleted, and other disorders where the approach can be shown to be beneficial.

### Achieving Differentiating Cell Populations

In order to achieve b) neural c) muscle d) and other cell populations capable of further environmentally-regulated differentiation in vivo, selected cell(s) and/or their progeny are optionally transfected with long (PRR+) Numb isoform sequence(s) and/or synthetic oligonucleotide sequences and expanded by growth for sufficient time to achieve the desirable number of cell progeny in vitro (as described above).

Following this optional step, the selected cells and/or their progeny are washed free of the cytokines and agents comprising the expansion/optimal growth media, and are optionally transfected with the nucleotide sequence(s) encoding synthetic oligonucleotides targeting the long (PRR+) isoforms, etc. , then cultured under conditions which promote differentiation of the selected cells into the desired cell type(s).

### Achieving Neuronal or Neural Cell Populations

When the desired cell population is a neural cell population, the successfully transfected cells are cultured under conditions that promote growth at a rate which is less than the optimal rate and in the presence of agent(s) promoting differentiation of the cells into neural cells. Conditions promoting differentiation into neurons have been described in numerous publications including (Benninger et al., 2003; Chung et al. 2005; Harkany et al., 2004; Ikeda et al., 2004; Ikeda et al., 2005; Wernig et al., 2002; and Wernig et al., 2004). Furthermore, combining retinoic acid exposure with the presence of additional cytokines favors specific neuronal cell type differentiation in vitro (e.g. Soundararajan et al., 2006; Soundararajan et al., 2007; U.S. Patent 6432711).

In a preferred embodiment, in vitro differentiation of neurons or neural cells occurs in the presence of 50 ng/mL nerve growth factor (NGF).

### Achieving Muscle Cell Populations

When the desired cell population is a muscle population, the successfully transfected cells are cultured in the presence of an agent promoting differentiation of the cells into muscle cells and growth at a rate less than the optimal rate. Conditions promoting differentiation into muscle cells have also been described previously (Nakamura et al., 2003; Pal and Khanna, 2005; Pipes et al., 2005; Albilez et al., 2006; Pal and Khanna, 2007; Behfar et al., 2007; U.S. Patent 6432711). Furthermore, exposure of selected cells and/or their progeny to hexamethylene bis-acrylamide or dimethylsulfoxide in the presence of additional cytokines favors the initiation of muscle type differentiation in vitro.

In another preferred embodiment, when a cardiac muscle cell population is the desired population, the cells are also transfected with nucleotide sequences including ones selected from those sequences encoding Gata 4, Gata 5, and Gata 6.

Simultaneous transfection with any subset of these distinct transgene sequences listed above can be accomplished by any means known to the art including the use of multiple genetic vectors, serial transfection as well as selection based on distinct marker proteins and/or antibiotic resistance.

When the desired cell population is a hematopoietic cell population, the differentiation medium includes specific agents at concentrations promoting differentiation into hematopoietic progenitor cells (e.g. vascular endothelial growth factor (VEGF), thrombopoietin, etc. (e.g. Ohmizono, 1997; Wang et al., 2005; Srivastava et al., 2007; Gupta et al., 2007) or differentiated hematopoietic cell types (according to methods known to the art for providing differentiated hematopoietic cell types from undifferentiated or pluripotent cells).

When the desired cell population is a germ cell population, the differentiation medium includes specific agents at concentrations promoting differentiation into germ cells (e.g. Nayernia et al. 2006a, 2006b).

When the desired cell population is an endoderm and pancreatic islet cell population, the differentiation media includes specific agents at concentrations promoting differentiation into endoderm and pancreatic islet cells (e.g. Xu et al., 2006; Denner et al., 2007; Shim et al., 2007; Jiang et al., 2007).

In embodiments, a single vector will be utilized which controls the expression of nucleotide sequence(s) encoding the "long" (PRR+) isoform(s) of the mammalian numb gene under one regulable promoter (e.g. a tetracycline-regulated promoter), while the Numblike and short Numb isoforms (and/or synthetic oligonucleotides targeting the long (PRR+) isoforms) are expressed under the control of another, distinct, but also regulable promoter. Thus, the long (PRR+) numb isoform(s) can be expressed (and/or short isoforms repressed) when expansion of the selected cells is desired and an inducing agent (e.g. tetracycline) is added to the growth medium; later numblike and the short isoforms can be expressed (and/or long (PRR+) numb isoform(s) repressed) when differentiation is desired.

Alternatively, proteins and peptides corresponding to Numb isoforms, Notch, listed herein may be applied in analogous fashion to selected cells and/or their progeny via electroporation (e.g. Koken et al., 1994; Ritchie and Gilroy, 1998), using nano particles, cationic lipids, fusogenic liposomes (e.g. Yoshikawa et al., 2005; 2007), etc. in lieu of, or in combination with genetic transfection. Generally, electroporation allows for high transfection efficiency (and efficient production of the desired cells) without genomic integration of the transgene and is therefore associated with increased safety.

The DNA or RNA encoding protein(s) or polypeptide(s) promoting proliferation, multipotentiality, pluripotentiality or differentiation of the selected cells may be isolated in accordance with standard genetic engineering techniques (for example, by isolating such DNA from a cDNA library of the specific cell line) and placing it into an appropriate expression vector, which then is transfected into the selected cells.

In a preferred embodiment, the genetic vectors encoding the long Numb isoforms (such as those described herein) are introduced transiently or under the control of a regulable promoter, into endogenous cells in vivo in order to cause those cells proliferate transiently.

In a preferred embodiment, endogenous cells (e.g. ependymal zone cells of the central nervous system) are transfected in vivo with genetic vectors encoding the long numb isoform(s) and/or other transgenes named herein, in order to transiently promote renewed or increased stem cell proliferation (with subsequent differentiation of progeny cells). This renewal or increase is measured in terms of the number of cells showing new-onset expression of marlers associated with dividing progenitors. This may be accomplished by introduction of the genetic vectors into the organ system using methods suitable for that purpose (see examples).

In a separate preferred embodiment, other nucleic acid(s) or protein(s) can be utilized in concert with the nucleic acid(s) or protein(s) corresponding to a single gene, or portion thereof, (particularly those named herein, discovered according to methods described herein, discovered according to other published methods; and/or known to be capable of initiating the desirable manner of differentiation) so long as a population of differentiating cells is produced from the selected cells and the method utilized is electroporation, liposomes, nanocapsules, nanovaults, and/or another approach avoiding retroviral/lentiviral integration or other random alteration of the cell's genome.

It is to be understood that any combination of nucleic acid or protein sequences described herein can be modified by excluding those corresponding to Numb and/or Numblike so long as the desired cell population or behavior is achieved.

Similarly, it should be understood that the methods described herein (or elsewhere) for initiating differentiation are applicable to any induced or non-induced multipotent, pluripotent, or self-renewing stem cells, or other selected cells, not only those obtained in the manner described herein.

### Sources of Selected cells

The population of selected cells may derive from somatic cells. However somatic cells lacking nuclei (e.g. mature, human red blood cells) are specifically excluded. Selected stem cells may be derived from existing cell lines or isolated from stored, banked, or cryopreserved sources. Typical sources of stem cells include bone marrow, peripheral blood, placental blood, amniotic fluid (e.g. De Coppi et al., 2007), umbilical cord blood (e.g. Zhao, et al., 2006; Tian et al., 2007), adipose tissue (e.g. Gimble et al., 2007; Ma et al., 2007), non-human embryos, and others. Circulating leukocytes and other non-stem cells may likewise be selected and subjected to the same culture conditions as described above effective that they acquire multipotency, pluripotency and/or self-renewal as a result. Examples of other accessible somatic cells useful in this invention include lymphocytes and epithelial (e.g. buccal cheek) cells. Isolation and collection of cells selected for use within the present invention may be performed by any method known to the art.

In a preferred embodiment, the selected cells are cultured in a three-dimensional format.

For instance, pluripotent, multipotent, and/or differentiating cells produced or treated according to the methods desribed herein (or other published methods) may be grown in association with three-dimesnisonal or two-dimensional scaffoldings engineered to replicate normal tissue structure and/or organ structures (e.g.Yarlagada et al., 2005; Kim et al, 1998; WO/2003/070084; EP1482871; WO03070084; US Patents 2395698; 7297540; 6995013; 6800753; Isenberg et al., 2006).

Similarly, scaffoldings to be occupied by the pluripotent, multipotent, and/or differentiating cells may be derived from cadaveric organ(s) or tissue(s) after the cadaveric organs or tissues (e.g. bone, heart, kidney, liver, lung, etc.) may be treated in such away that the host immune cells resident in that tissue, and other undesirable or ancillary host cells, are eliminated (e.g. by ionizing radiation, sterilization (e.g. Mroz et al., 2006), and/or various methods of decellularization (United States Patents 6734018; 6962814; 6479064; 6376244; U.S. Pat. Nos. 5032508; 4902508; 4956178; 5281422, 5554389; 6099567; and 6206931; 4361552 and 6576618; 6753181; U.S. application Ser. No. 11/162,715; WO/2001/048153; WO/2002/024244; WO003002165; WO/2001/049210; WO/2007/025233; European Patents EP1482871; EP1246903; EP1244396; EP0987998; EP1244396; EP1333870; Riederetal., 2004; Ott et al., 2008; Taylor et al., 1998)).

Likewise, it is anticipated that the pluripotent, multipotent, and/or differentiating cells of the present invention may be used in applications utilizing inkjet-style printing for tissue engineering (e.g. Boland et al., 2006. Xu et al., 2006; Campbell et al., 2007). Therefore such use of the cells produced or treated according to the methods described herein is covered.

In another preferred embodiment, the selected cells are cultured in hanging drops.

In accordance with another aspect of the present invention, selected cells may be modified genetically beforehand.

In accordance with another aspect of the present invention, selected cells may be modified with DNA or RNA encoding protein(s) or polypeptide(s) promoting differentiation of the cell into a desired cell population.

The present invention further discloses the directed differentiation of cells transfected with the therapeutic vector(s) into desired cell types by further incubation in media containing the appropriate cytokines and growth factors such as colony stimulating factors such as M-CSF (CSF-1), GM-CSF, IL-7, any cytokine promoting CD4+ T cell differentiation, etc.

### Transfection

Genetic modification of selected cells and target cells, whether they be exogenous cells or endogenous cells can be performed according to any published or unpublished method known to the art (e.g. U.S. Pat. Nos. 6,432,711, U.S. 05,593,875, U.S. 05,783,566, U.S. 5,928,944, U.S. 05,910,488, U.S. 05,824,547, etc.) or by other generally accepted means. Suitable methods for transforming host cells can be found in Sambrook et al. (Molecular Cloning: A Laboratory Manual, 2nd Edition, Cold Spring Harbor Laboratory press (1989)), and other laboratory textbooks.

Successfully transfected cells can be identified by selection protocols involving markers such as antibiotic resistance genes in addition to RNA expression assays and morphological analyses. Clones from successfully transfected cells, expressing the appropriate exogenous DNA at appropriate levels, can be preserved as cell lines by cryopreservation (utilizing any appropriate method of cryopreservation known to the art).

Selectable markers (e.g., antibiotics resistance genes) may include those which confer resistance to drugs, such as G418, hygromycin, ampicillin and blasticidin, etc. Cells containing the gene of interest can be identified by drug selection where cells that have incorporated the selectable marker gene survive, and others die.

A theoretical basis for the embodiments of the invention is described herein, however, this discussion is not in any way to be considered as binding or limiting on the present invention. Those of skill in the art will understand that the various embodiments of the invention may be practiced regardless of the model used to describe the theoretical underpinnings of the invention.

The invention will now be described and illustrated with respect to the following examples; however, the scope of the present invention is not intended to be limited thereby.

### Example 1: Growth Medium for selected cells

Selected cells can be expanded / grown in Dulbecco's modified Minimal Essential Medium (DMEM) supplemented with glutamine, beta.-mercaptoethanol, 10% (by volume) horse serum, and human recombinant Leukemia Inhibitory Factor (LIF). LIF replaces the need for maintaining selected cells on feeder layers of cells, (which may also be employed) and is essential for maintaining selected cells in an undifferentiated, multipotent, or pluripotent state, such cells can be maintained in Dulbecco's modified Minimal Essential Medium (DMEM) supplemented with glutamine, beta.-mercaptoethanol, 10% (by volume) horse serum, and human recombinant Leukemia Inhibitory Factor (LIF). The LIF replaces the need for maintaining cells on feeder layers of cells, (which may also be employed) and is essential for maintaining cells in an undifferentiated state (per US Patent 6432711).

In order to initiate the differentiation of the selected cells into neuronal cells, the cells are trypsinized and washed free of LIF, and placed in DMEM supplemented with 10% fetal bovine serum (FBS). After resuspension in DMEM and 10% FBS, 1X10⁶ cells are plated in 5 ml DMEM, 10% FBS, 0.5 microM retinoic acid in a 60 mm Fisher bacteriological grade Petri dishes, where the cells are expected to form small aggregates. Aggregation aids in proper cell differentiation. High efficiency transfection with appropriate neuronal transcription factors can occur before or after plating in DMEM, FBS, and retinoic acid. (See U.S. Patents 6432711 and 5453357 for additional details).

Example 2: HLA matching. Selected cells (e.g. umbilical cord blood or cells from any other suitable source and/or their progeny), can be screened, genetically-modified (optional), expanded, and induced to begin differentiating into the desired cell type(s) (optional). The cells are then transplanted according to standard stem cell transplantation protocols. In certain instances, cells may be transplanted into patients without HLA matching.

### Example 3: Genetic Modification of Selected Cells.

In vitro genetic modification of exogenous cells or patient's endogenous cells can be performed according to any published or unpublished method known to the art (e.g. U.S. Pat. Nos. 6,432,711, U.S. 05,593,875, U.S. 05,783,566, U.S. 5,928,944, U.S. 05,910,488, U.S. 05,824,547, etc.) or by other generally accepted means. Suitable methods for transforming host cells can be found in Sambrook et al. (Molecular Cloning: A Laboratory Manual, 2nd Edition, Cold Spring Harbor Laboratory press (1989)), and other laboratory textbooks.

Successfully transfected cells are identified by selection protocols involving markers such as antibiotic resistance genes in addition to RNA expression assays and morphological analyses. Clones from successfully transfected cells, expressing the appropriate exogenous DNA at appropriate levels, can be preserved as cell lines by cryopreservation (utilizing any appropriate method of cryopreservation known to the art).

Selectable markers (e.g., antibiotics resistance genes) may include those conferring resistance to drugs, such as G418, hygromycin and methotrexate. Cells containing the gene of interest can be identified by drug selection where cells that have incorporated the selectable marker gene survive, and others die.

The current invention discloses the selection of genetically-modified cells as "selected cells" of the invention. The term genetic modification refers to alteration of the cellular genotype by introducing natural or synthetic nucleic acids into selected cells and/or their progeny or immortalized cell lines and/or their progeny by any means known to the art. Alternatively culture conditions that induce permanent changes in gene expression patterns are considered herein to represent genetic modification. Modification of stem cells, whether they be derived from the host brain, endogenous donor sources, exogenous donor sources, or cell lines, represents a feasible approach to the treatment of certain human diseases, especially those of the human nervous system.

Genetic modifications covered by this disclosure include, but are not limited to: genetic modifications performed in vivo; modifications that alter the activity or amount of metabolic enzymes expressed by endogenous or exogenous selected cells and/or their progeny; modifications which alter the activity, amount, or antigenicity of cellular proteins; modifications which alter the activity or amount of proteins involved in signal transduction pathways; modifications which alter HLA type; modifications which alter cellular differentiation; modifications which alter neoplastic potential; modifications which alter cellular differentiation; modifications which alter the amount or activity of structural proteins; modifications which alter the amount or activity of membrane associated proteins (structural or enzymatic); modifications which alter the activity or amount of proteins involved in DNA repair and chromosome maintenance; modifications which alter the activity or amount of proteins involved in cellular transport; modifications which alter the activity or amount of enzymes; modifications which alter the activity or amount of proteins involved in synapse formation and maintenance; modifications which alter the activity or amount of proteins involved in neurite outgrowth or axon outgrowth and formation; modifications altering the amount or activity of antioxidant producing enzymes within the cell; modifications which lead to altered post-translational modification of cellular proteins; modifications which alter the activity or amount of proteins involved in other aspects of cellular repair, and alterations which increase the lifespan of the cell (such as production of telomerase). Such proteins as those mentioned above may be encoded for by DNA or RNA derived from the human genome or other animal, plant, viral, or bacterial genomes. This invention also covers sequences designed de novo.

In addition, this invention relates to the in situ, genetic modification of selected cells and/or their progeny cells for the treatment of disease. Endogenous stem cells may be modified in situ by direct injection or application of DNA or RNA vectors, including viruses, retroviruses, liposomes, etc, into the substance of the tissue or into the appropriate portion of the ventricular system of the brain. Since 1992, we have modified thousands of stem/progenitor cells and many thousand progeny cells in this manner. Our data shows that this manner of modifying progenitor cells results in a tremendous variety of modified cell types throughout the nervous system, and has never resulted in adverse effects.

### Example4: Examples of expressed or targeted transgenes utilized in the present invention.

Any transgene sequences effective in fulfilling the present invention is suitable for use in the present invention. Suitable nucleotide sequences may be drawn from any species so long as the desired cells or behavior is achieved. Likewise the method of naming such sequences, either in lower case or upper case letters herein, does not imply a particular species. The following sequences stored in the NCBI database (listed by accession number) represent examples of sequences referenced above in the present application. They are also examples of specific transgene encoding sequences (cds) suitable for use in the present invention, but do not in any way limit the practice of the invention:
cardiotrophin1:U43030 (SEQ ID NO: 18):
IL6:BC015511 (SEQ ID NO: 21):
IL6R:NM_00565 (SEQ ID NO: 23):
IL11:NM_133519 (SEQ ID NO: 24):
LIFR:NM_002310 (SEQ ID NO: 26):

### Notch

Notch1:NM_017617 (SEQ ID NO: 72):
NOTCH2:NM_024408; NM_010928.
NOTCH3:NM_000435 (SEQ ID NO: 73):

### SEQUENCE LISTING

<110> REID, CHRISTOPHER B.
<120> METHODS FOR PRODUCTION AND USES OF MULTIPOTENT CELL POPULATIONS, PLURIPOTENT CELL POPULATIONS, NEURONAL CELL POPULATIONS, AND MUSCLE CELL POPULATIONS
<130> CBR-002PCT
<140> PCT/US2008/065007
   <141> 2008-05-28
<150> 61/064,761
   <151> 2008-03-25
<150> 61/006,449
   <151> 2008-01-14
<150> 60/933,670
   <151> 2007-06-08
<150> 60/933,133
   <151> 2007-06-05
<150> 60/932,020
   <151> 2007-05-29
<160> 109
<170> PatentIn Ver. 3.3
<210> 1
   <211> 8069
   <212> DNA
   <213> Artificial Sequence
<220>
   <223> Description of Artificial Sequence: Synthetic polynucleotide
<400> 1
   aatgtagtct tatgcaatac tcttgtagtc ttgcaacatg gtaacgatga gttagcaaca 60
   tgccttacaa ggagagaaaa agcaccgtgc atgccgattg gtggaagtaa ggtggtacga 120
   tcgtgcctta ttaggaaggc aacagacggg tctgacatgg attggacgaa ccactgaatt 180
   gccgcattgc agagatattg tatttaagtg cctagctcga tacataaacg ggtctctctg 240
   gttagaccag atctgagcct gggagctctc tggctaacta gggaacccac tgcttaagcc 300
   tcaataaagc ttgccttgag tgcttcaagt agtgtgtgcc cgtctgttgt gtgactctgg 360
   taactagaga tccctcagac ccttttagtc agtgtggaaa atctctagca gtggcgcccg 420
   aacagggact tgaaagcgaa agggaaacca gaggagctct ctcgacgcag gactcggctt 480
   gctgaagcgc gcacggcaag aggcgagggg cggcgactgg tgagtacgcc aaaaattttg 540
   actagcggag gctagaagga gagagatggg tgcgagagcg tcagtattaa gcgggggaga 600
   attagatcgc gatgggaaaa aattcggtta aggccagggg gaaagaaaaa atataaatta 660
   aaacatatag tatgggcaag cagggagcta gaacgattcg cagttaatcc tggcctgtta 720
   gaaacatcag aaggctgtag acaaatactg ggacagctac aaccatccct tcagacagga 780
   tcagaagaac ttagatcatt atataataca gtagcaaccc tctattgtgt gcatcaaagg 840
   atagagataa aagacaccaa ggaagcttta gacaagatag aggaagagca aaacaaaagt 900
   aagaccaccg cacagcaagc ggccgctgat cttcagacct ggaggaggag atatgaggga 960
   caattggaga agtgaattat ataaatataa agtagtaaaa attgaaccat taggagtagc 1020
   acccaccaag gcaaagagaa gagtggtgca gagagaaaaa agagcagtgg gaataggagc 1080
   tttgttcctt gggttcttgg gagcagcagg aagcactatg ggcgcagcgt caatgacgct 1140
   gacggtacag gccagacaat tattgtctgg tatagtgcag cagcagaaca atttgctgag 1200
   ggctattgag gcgcaacagc atctgttgca actcacagtc tggggcatca agcagctcca 1260
   ggcaagaatc ctggctgtgg aaagatacct aaaggatcaa cagctcctgg ggatttgggg 1320
   ttgctctgga aaactcattt gcaccactgc tgtgccttgg aatgctagtt ggagtaataa 1380
   atctctggaa cagatttgga atcacacgac ctggatggag tgggacagag aaattaacaa 1440
   ttacacaagc ttaatacact ccttaattga agaatcgcaa aaccagcaag aaaagaatga 1500
   acaagaatta ttggaattag ataaatgggc aagtttgtgg aattggttta acataacaaa 1560
   ttggctgtgg tatataaaat tattcataat gatagtagga ggcttggtag gtttaagaat 1620
   agtttttgct gtactttcta tagtgaatag agttaggcag ggatattcac cattatcgtt 1680
   tcagacccac ctcccaaccc cgaggggacc cgacaggccc gaaggaatag aagaagaagg 1740
   tggagagaga gacagagaca gatccattcg attagtgaac ggatctcgac ggtatcgatg 1800
   tcgacgttaa cgctagtgat atcaactttg tatagaaaag ttgaacgaga aacgtaaaat 1860
   gatataaata tcaatatatt aaattagatt ttgcataaaa aacagactac ataatactgt 1920
   aaaacacaac atatccagtc actatggcgg ccgcattagg caccccaggc tttacacttt 1980
   atgcttccgg ctcgtataat gtgtggattt tgagttagga tccgtcgaga ttttcaggag 2040
   ctaaggaagc taaaatggag aaaaaaatca ctggatatac caccgttgat atatcccaat 2100
   ggcatcgtaa agaacatttt gaggcatttc agtcagttgc tcaatgtacc tataaccaga 2160
   ccgttcagct ggatattacg gcctttttaa agaccgtaaa gaaaaataag cacaagtttt 2220
   atccggcctt tattcacatt cttgcccgcc tgatgaatgc tcatccggaa ttccgtatgg 2280
   caatgaaaga cggtgagctg gtgatatggg atagtgttca cccttgttac accgttttcc 2340
   atgagcaaac tgaaacgttt tcatcgctct ggagtgaata ccacgacgat ttccggcagt 2400
   ttctacacat atattcgcaa gatgtggcgt gttacggtga aaacctggcc tatttcccta 2460
   aagggtttat tgagaatatg tttttcgtct cagccaatcc ctgggtgagt ttcaccagtt 2520
   ttgatttaaa cgtggccaat atggacaact tcttcgcccc cgttttcacc atgggcaaat 2580
   attatacgca aggcgacaag gtgctgatgc cgctggcgat tcaggttcat catgccgttt 2640
   gtgatggctt ccatgtcggc agaatgctta atgaattaca acagtactgc gatgagtggc 2700
   agggcggggc gtaaagatct ggatccggct tactaaaagc cagataacag tatgcgtatt 2760
   tgcgcgctga tttttgcggt ataagaatat atactgatat gtatacccga agtatgtcaa 2820
   aaagaggtat gctatgaagc agcgtattac agtgacagtt gacagcgaca gctatcagtt 2880
   gctcaaggca tatatgatgt caatatctcc ggtctggtaa gcacaaccat gcagaatgaa 2940
   gcccgtcgtc tgcgtgccga acgctggaaa gcggaaaatc aggaagggat ggctgaggtc 3000
   gcccggttta ttgaaatgaa cggctctttt gctgacgaga acagggactg gtgaaatgca 3060
   gtttaaggtt tacacctata aaagagagag ccgttatcgt ctgtttgtgg atgtacagag 3120
   tgatattatt gacacgcccg ggcgacggat ggtgatcccc ctggccagtg cacgtctgct 3180
   gtcagataaa gtctcccgtg aactttaccc ggtggtgcat atcggggatg aaagctggcg 3240
   catgatgacc accgatatgg ccagtgtgcc ggtctccgtt atcggggaag aagtggctga 3300
   tctcagccac cgcgaaaatg acatcaaaaa cgccattaac ctgatgttct ggggaatata 3360
   aatgtcaggc tccgttatac acagccagtc tgcaggtcga ccatagtgac tggatatgtt 3420
   gtgttttaca gtattatgta gtctgttttt tatgcaaaat ctaatttaat atattgatat 3480
   ttatatcatt ttacgtttct cgttcagctt tcttgtacaa agtggttgat atccagcaca 3540
   gtggcggccg ctcgagtcta gagggcccgc ggttcgaagg taagcctatc cctaaccctc 3600
   tcctcggtct cgattctacg cgtaccggtt agtaatgagt ttggaattaa ttctgtggaa 3660
   tgtgtgtcag ttagggtgtg gaaagtcccc aggctcccca gcaggcagaa gtatgcaaag 3720
   catgcatctc aattagtcag caaccaggtg tggaaagtcc ccaggctccc cagcaggcag 3780
   aagtatgcaa agcatgcatc tcaattagtc agcaaccata gtcccgcccc taactccgcc 3840
   catcccgccc ctaactccgc ccagttccgc ccattctccg ccccatggct gactaatttt 3900
   ttttatttat gcagaggccg aggccgcctc tgcctctgag ctattccaga agtagtgagg 3960
   aggctttttt ggaggcctag gcttttgcaa aaagctcccg ggagcttgta tatccatttt 4020
   cggatctgat cagcacgtgt tgacaattaa tcatcggcat agtatatcgg catagtataa 4080
   tacgacaagg tgaggaacta aaccatggcc aagcctttgt ctcaagaaga atccaccctc 4140
   attgaaagag caacggctac aatcaacagc atccccatct ctgaagacta cagcgtcgcc 4200
   agcgcagctc tctctagcga cggccgcatc ttcactggtg tcaatgtata tcattttact 4260
   gggggacctt gtgcagaact cgtggtgctg ggcactgctg ctgctgcggc agctggcaac 4320
   ctgacttgta tcgtcgcgat cggaaatgag aacaggggca tcttgagccc ctgcggacgg 4380
   tgccgacagg tgcttctcga tctgcatcct gggatcaaag ccatagtgaa ggacagtgat 4440
   ggacagccga cggcagttgg gattcgtgaa ttgctgccct ctggttatgt gtgggagggc 4500
   taagcacaat tcgagctcgg tacctttaag accaatgact tacaaggcag ctgtagatct 4560
   tagccacttt ttaaaagaaa aggggggact ggaagggcta attcactccc aacgaagaca 4620
   agatctgctt tttgcttgta ctgggtctct ctggttagac cagatctgag cctgggagct 4680
   ctctggctaa ctagggaacc cactgcttaa gcctcaataa agcttgcctt gagtgcttca 4740
   agtagtgtgt gcccgtctgt tgtgtgactc tggtaactag agatccctca gaccctttta 4800
   gtcagtgtgg aaaatctcta gcagtagtag ttcatgtcat cttattattc agtatttata 4860
   acttgcaaag aaatgaatat cagagagtga gaggaacttg tttattgcag cttataatgg 4920
   ttacaaataa agcaatagca tcacaaattt cacaaataaa gcattttttt cactgcattc 4980
   tagttgtggt ttgtccaaac tcatcaatgt atcttatcat gtctggctct agctatcccg 5040
   cccctaactc cgcccatccc gcccctaact ccgcccagtt ccgcccattc tccgccccat 5100
   ggctgactaa ttttttttat ttatgcagag gccgaggccg cctcggcctc tgagctattc 5160
   cagaagtagt gaggaggctt ttttggaggc ctagggacgt acccaattcg ccctatagtg 5220
   agtcgtatta cgcgcgctca ctggccgtcg ttttacaacg tcgtgactgg gaaaaccctg 5280
   gcgttaccca acttaatcgc cttgcagcac atcccccttt cgccagctgg cgtaatagcg 5340
   aagaggcccg caccgatcgc ccttcccaac agttgcgcag cctgaatggc gaatgggacg 5400
   cgccctgtag cggcgcatta agcgcggcgg gtgtggtggt tacgcgcagc gtgaccgcta 5460
   cacttgccag cgccctagcg cccgctcctt tcgctttctt cccttccttt ctcgccacgt 5520
   tcgccggctt tccccgtcaa gctctaaatc gggggctccc tttagggttc cgatttagtg 5580
   ctttacggca cctcgacccc aaaaaacttg attagggtga tggttcacgt agtgggccat 5640
   cgccctgata gacggttttt cgccctttga cgttggagtc cacgttcttt aatagtggac 5700
   tcttgttcca aactggaaca acactcaacc ctatctcggt ctattctttt gatttataag 5760
   ggattttgcc gatttcggcc tattggttaa aaaatgagct gatttaacaa aaatttaacg 5820
   cgaattttaa caaaatatta acgcttacaa tttaggtggc acttttcggg gaaatgtgcg 5880
   cggaacccct atttgtttat ttttctaaat acattcaaat atgtatccgc tcatgagaca 5940
   ataaccctga taaatgcttc aataatattg aaaaaggaag agtatgagta ttcaacattt 6000
   ccgtgtcgcc cttattccct tttttgcggc attttgcctt cctgtttttg ctcacccaga 6060
   aacgctggtg aaagtaaaag atgctgaaga tcagttgggt gcacgagtgg gttacatcga 6120
   actggatctc aacagcggta agatccttga gagttttcgc cccgaagaac gttttccaat 6180
   gatgagcact tttaaagttc tgctatgtgg cgcggtatta tcccgtattg acgccgggca 6240
   agagcaactc ggtcgccgca tacactattc tcagaatgac ttggttgagt actcaccagt 6300
   cacagaaaag catcttacgg atggcatgac agtaagagaa ttatgcagtg ctgccataac 6360
   catgagtgat aacactgcgg ccaacttact tctgacaacg atcggaggac cgaaggagct 6420
   aaccgctttt ttgcacaaca tgggggatca tgtaactcgc cttgatcgtt gggaaccgga 6480
   gctgaatgaa gccataccaa acgacgagcg tgacaccacg atgcctgtag caatggcaac 6540
   aacgttgcgc aaactattaa ctggcgaact acttactcta gcttcccggc aacaattaat 6600
   agactggatg gaggcggata aagttgcagg accacttctg cgctcggccc ttccggctgg 6660
   ctggtttatt gctgataaat ctggagccgg tgagcgtggg tctcgcggta tcattgcagc 6720
   actggggcca gatggtaagc cctcccgtat cgtagttatc tacacgacgg ggagtcaggc 6780
   aactatggat gaacgaaata gacagatcgc tgagataggt gcctcactga ttaagcattg 6840
   gtaactgtca gaccaagttt actcatatat actttagatt gatttaaaac ttcattttta 6900
   atttaaaagg atctaggtga agatcctttt tgataatctc atgaccaaaa tcccttaacg 6960
   tgagttttcg ttccactgag cgtcagaccc cgtagaaaag atcaaaggat cttcttgaga 7020
   tccttttttt ctgcgcgtaa tctgctgctt gcaaacaaaa aaaccaccgc taccagcggt 7080
   ggtttgtttg ccggatcaag agctaccaac tctttttccg aaggtaactg gcttcagcag 7140
   agcgcagata ccaaatactg ttcttctagt gtagccgtag ttaggccacc acttcaagaa 7200
   ctctgtagca ccgcctacat acctcgctct gctaatcctg ttaccagtgg ctgctgccag 7260
   tggcgataag tcgtgtctta ccgggttgga ctcaagacga tagttaccgg ataaggcgca 7320
   gcggtcgggc tgaacggggg gttcgtgcac acagcccagc ttggagcgaa cgacctacac 7380
   cgaactgaga tacctacagc gtgagctatg agaaagcgcc acgcttcccg aagggagaaa 7440
   ggcggacagg tatccggtaa gcggcagggt cggaacagga gagcgcacga gggagcttcc 7500
   agggggaaac gcctggtatc tttatagtcc tgtcgggttt cgccacctct gacttgagcg 7560
   tcgatttttg tgatgctcgt caggggggcg gagcctatgg aaaaacgcca gcaacgcggc 7620
   ctttttacgg ttcctggcct tttgctggcc ttttgctcac atgttctttc ctgcgttatc 7680
   ccctgattct gtggataacc gtattaccgc ctttgagtga gctgataccg ctcgccgcag 7740
   ccgaacgacc gagcgcagcg agtcagtgag cgaggaagcg gaagagcgcc caatacgcaa 7800
   accgcctctc cccgcgcgtt ggccgattca ttaatgcagc tggcacgaca ggtttcccga 7860
   ctggaaagcg ggcagtgagc gcaacgcaat taatgtgagt tagctcactc attaggcacc 7920
   ccaggcttta cactttatgc ttccggctcg tatgttgtgt ggaattgtga gcggataaca 7980
   atttcacaca ggaaacagct atgaccatga ttacgccaag cgcgcaatta accctcacta 8040
   aagggaacaa aagctggagc tgcaagctt                                   8069
<210> 2
   <211> 22460
   <212> DNA
   <213> Artificial Sequence
<220>
   <223> Description of Artificial Sequence: Synthetic polynucleotide
<400> 2
   aatgtagtct tatgcaatac tcttgtagtc ttgcaacatg gtaacgatga gttagcaaca 60
   tgccttacaa ggagagaaaa agcaccgtgc atgccgattg gtggaagtaa ggtggtacga 120
   tcgtgcctta ttaggaaggc aacagacggg tctgacatgg attggacgaa ccactgaatt 180
   gccgcattgc agagatattg tatttaagtg cctagctcga tacataaacg ggtctctctg 240
   gttagaccag atctgagcct gggagctctc tggctaacta gggaacccac tgcttaagcc 300
   tcaataaagc ttgccttgag tgcttcaagt agtgtgtgcc cgtctgttgt gtgactctgg 360
   taactagaga tccctcagac ccttttagtc agtgtggaaa atctctagca gtggcgcccg 420
   aacagggact tgaaagcgaa agggaaacca gaggagctct ctcgacgcag gactcggctt 480
   gctgaagcgc gcacggcaag aggcgagggg cggcgactgg tgagtacgcc aaaaattttg 540
   actagcggag gctagaagga gagagatggg tgcgagagcg tcagtattaa gcgggggaga 600
   attagatcgc gatgggaaaa aattcggtta aggccagggg gaaagaaaaa atataaatta 660
   aaacatatag tatgggcaag cagggagcta gaacgattcg cagttaatcc tggcctgtta 720
   gaaacatcag aaggctgtag acaaatactg ggacagctac aaccatccct tcagacagga 780
   tcagaagaac ttagatcatt atataataca gtagcaaccc tctattgtgt gcatcaaagg 840
   atagagataa aagacaccaa ggaagcttta gacaagatag aggaagagca aaacaaaagt 900
   aagaccaccg cacagcaagc ggccgctgat cttcagacct ggaggaggag atatgaggga 960
   caattggaga agtgaattat ataaatataa agtagtaaaa attgaaccat taggagtagc 1020
   acccaccaag gcaaagagaa gagtggtgca gagagaaaaa agagcagtgg gaataggagc 1080
   tttgttcctt gggttcttgg gagcagcagg aagcactatg ggcgcagcgt caatgacgct 1140
   gacggtacag gccagacaat tattgtctgg tatagtgcag cagcagaaca atttgctgag 1200
   ggctattgag gcgcaacagc atctgttgca actcacagtc tggggcatca agcagctcca 1260
   ggcaagaatc ctggctgtgg aaagatacct aaaggatcaa cagctcctgg ggatttgggg 1320
   ttgctctgga aaactcattt gcaccactgc tgtgccttgg aatgctagtt ggagtaataa 1380
   atctctggaa cagatttgga atcacacgac ctggatggag tgggacagag aaattaacaa 1440
   ttacacaagc ttaatacact ccttaattga agaatcgcaa aaccagcaag aaaagaatga 1500
   acaagaatta ttggaattag ataaatgggc aagtttgtgg aattggttta acataacaaa 1560
   ttggctgtgg tatataaaat tattcataat gatagtagga ggcttggtag gtttaagaat 1620
   agtttttgct gtactttcta tagtgaatag agttaggcag ggatattcac cattatcgtt 1680
   tcagacccac ctcccaaccc cgaggggacc cgacaggccc gaaggaatag aagaagaagg 1740
   tggagagaga gacagagaca gatccattcg attagtgaac ggatctcgac ggtatcgatg 1800
   tcgacgttaa cgctagtgat atcaactttg tatagaaaag ttgaacgaga aacgtaaaat 1860
   gatataaata tcaatatatt aaattagatt ttgcataaaa aacagactac ataatactgt 1920
   aaaacacaac atatccagtc actatgggac ggatcgggag atctcccgat cccctatggt 1980
   gcactctcag tacaatctgc tctgatgccg catagttaag ccagtatctg ctccctgctt 2040
   gtgtgttgga ggtcgctgag tagtgcgcga gcaaaattta agctacaaca aggcaaggct 2100
   tgaccgacaa ttgcatgaag aatctgctta gggttaggcg ttttgcgctg cttcgcgatg 2160
   tacgggccag atatacgcgt tgacattgat tattgactag ttattaatag taatcaatta 2220
   cggggtcatt agttcatagc ccatatatgg agttccgcgt tacataactt acggtaaatg 2280
   gcccgcctgg ctgaccgccc aacgaccccc gcccattgac gtcaataatg acgtatgttc 2340
   ccatagtaac gccaataggg actttccatt gacgtcaatg ggtggagtat ttacggtaaa 2400
   ctgcccactt ggcagtacat caagtgtatc atatgccaag tacgccccct attgacgtca 2460
   atgacggtaa atggcccgcc tggcattatg cccagtacat gaccttatgg gactttccta 2520
   cttggcagta catctacgta ttagtcatcg ctattaccat ggtgatgcgg ttttggcagt 2580
   acatcaatgg gcgtggatag cggtttgact cacggggatt tccaagtctc caccccattg 2640
   acgtcaatgg gagtttgttt tggaaccaaa atcaacggga ctttccaaaa tgtcgtaaca 2700
   actccgcccc attgacgcaa atgggcggta ggcgtgtacg gtgggaggtc tatataagca 2760
   gagctctccc tatcagtgat agagatctcc ctatcagtga tagagatcgt cgacgagctc 2820
   gtttagtgaa ccgtcagatc gcctggagac gccatccacg ctgttttgac ctccatagaa 2880
   gacaccggga ccgatccagc ctccggactc tagcgtttaa acttaagctt accatgcctt 2940
   cgcaagccct catttcacca ggcccccggc ttggggcgcc ttccttcccc atggcgggac 3000
   acctggcttc ggatttcgcc ttctcgcccc ctccaggtgg tggaggtgat gggccagggg 3060
   ggccggagcc gggctgggtt gatcctcgga cctggctaag cttccaaggc cctcctggag 3120
   ggccaggaat cgggccgggg gttgggccag gctctgaggt gtgggggatt cccccatgcc 3180
   ccccgccgta tgagttctgt ggggggatgg cgtactgtgg gccccaggtt ggagtggggc 3240
   tagtgcccca aggcggcttg gagacctctc agcctgaggg cgaagcagga gtcggggtgg 3300
   agagcaactc cgatggggcc tccccggagc cctgcaccgt cacccctggt gccgtgaagc 3360
   tggagaagga gaagctggag caaaacccgg aggagtccca ggacatcaaa gctctgcaga 3420
   aagaactcga gcaatttgcc aagctcctga agcagaagag gatcaccctg ggatatacac 3480
   aggccgatgt ggggctcacc ctgggggttc tatttgggaa ggtattcagc caaacgacca 3540
   tctgccgctt tgaggctctg cagcttagct tcaagaacat gtgtaagctg cggcccttgc 3600
   tgcagaagtg ggtggaggaa gctgacaaca atgaaaatct tcaggagata tgcaaagcag 3660
   aaaccctcgt gcaggcccga aagagaaagc gaaccagtat cgagaaccga gtgagaggca 3720
   acctggagaa tttgttcctg cagtgcccga aacccacact gcagcagatc agccacatcg 3780
   cccagcagct tgggctcgag aaggatgtgg tccgagtgtg gttctgtaac cggcgccaga 3840
   agggcaagcg atcaagcagc gactatgcac aacgagagga ttttgaggct gctgggtctc 3900
   ctttctcagg gggaccagtg tcctttcctc tggccccagg gccccatttt ggtaccccag 3960
   gctatgggag ccctcacttc actgcactgt actcctcggt ccctttccct gagggggaag 4020
   cctttccccc tgtctccgtc accactctgg gctctcccat gcattcaaac tgaggtgcct 4080
   gcccttctag gaatggggga cagggggagg ggaggagcta gggaaagaaa acctggagtt 4140
   tgtgccaggg tttttgggat taagttcttc attcactaag gaaggaattg ggaacacaaa 4200
   gggtgggggc aggggagttt ggggcaactg gttggaggga aggtgaagtt caatgatgct 4260
   cttgatttta atcccacatc atgtatcact tttttcttaa ataaagaagc ctgggacaca 4320
   gtagatagac acacttaaaa aaaaaaacct cgactgtgcc ttctagttgc cagccatctg 4380
   ttgtttgccc ctcccccgtg ccttccttga ccctggaagg tgccactccc actgtccttt 4440
   cctaataaaa tgaggaaatt gcatcgcatt gtctgagtag gtgtcattct attctggggg 4500
   gtggggtggg gcaggacagc aagggggagg attgggaaga caatagcagg catgctgggg 4560
   atgcggtggg ctctatggga cggatcggga gatctcccga tcccctatgg tgcactctca 4620
   gtacaatctt gctctgatgc cgcatagtta agccagtatc tgctccctgc ttgtgtgttg 4680
   gaggtcgctg agtagtgcgc gagcaaaatt taagctacaa caaggcaagg cttgaccgac 4740
   aattgcatga agaatctgct tagggttagg cgttttgcgc tgcttcgcga tgtacgggcc 4800
   agatatacgc gttgacattg attattgact agttattaat agtaatcaat tacggggtca 4860
   ttagttcata gcccatatat ggagttccgc gttacataac ttacggtaaa tggcccgcct 4920
   ggctgaccgc ccaacgaccc ccgcccattg acgtcaataa tgacgtatgt tcccatagta 4980
   acgccaatag ggactttcca ttgacgtcaa tgggtggagt atttacggta aactgcccac 5040
   ttggcagtac atcaagtgta tcatatgcca agtacgcccc ctattgacgt caatgacggt 5100
   aaatggcccg cctggcatta tgcccagtac atgaccttat gggactttcc tacttggcag 5160
   tacatctacg tattagtcat cgctattacc atggtgatgc ggttttggca gtacatcaat 5220
   gggcgtggat agcggtttga ctcacgggga tttccaagtc tccaccccat tgacgtcaat 5280
   gggagtttgt tttggaacca aaatcaacgg gactttccaa aatgtcgtaa caactccgcc 5340
   ccattgacgc aaatgggcgg taggcgtgta cggtgggagg tctatataag cagagctctc 5400
   cctatcagtg atagagatct ccctatcagt gatagagatc gtcgacgagc tcgtttagtg 5460
   aaccgtcaga tcgcctggag acgccatcca cgctgttttg acctccatag aagacaccgg 5520
   gaccgatcca gcctccggac tctagcgttt aaacttaagc ttaccatgct attaacttgt 5580
   tcaaaaaagt atcaggagtt gtcaaggcag agaagagagt gtttgcaaaa gggggaaagt 5640
   agtttgctgc ctctttaaga ctaggactga gagaaagaag aggagagaga aagaaaggga 5700
   gagaagtttg agccccaggc ttaagccttt ccaaaaaata ataataacaa tcatcggcgg 5760
   cggcaggatc ggccagagga ggagggaagc gctttttttg atcctgattc cagtttgcct 5820
   ctctcttttt ttcccccaaa ttattcttcg cctgattttc ctcgcggagc cctgcgctcc 5880
   cgacaccccc gcccgcctcc cctcctcctc tccccccgcc cgcgggcccc ccaaagtccc 5940
   ggccgggccg agggtcggcg gccgccggcg ggccgggccc gcgcacagcg cccgcatgta 6000
   caacatgatg gagacggagc tgaagccgcc gggcccgcag caaacttcgg ggggcggcgg 6060
   cggcaactcc accgcggcgg cggccggcgg caaccagaaa aacagcccgg accgcgtcaa 6120
   gcggcccatg aatgccttca tggtgtggtc ccgcgggcag cggcgcaaga tggcccagga 6180
   gaaccccaag atgcacaact cggagatcag caagcgcctg ggcgccgagt ggaaactttt 6240
   gtcggagacg gagaagcggc cgttcatcga cgaggctaag cggctgcgag cgctgcacat 6300
   gaaggagcac ccggattata aataccggcc ccggcggaaa accaagacgc tcatgaagaa 6360
   ggataagtac acgctgcccg gcgggctgct ggcccccggc ggcaatagca tggcgagcgg 6420
   ggtcggggtg ggcgccggcc tgggcgcggg cgtgaaccag cgcatggaca gttacgcgca 6480
   catgaacggc tggagcaacg gcagctacag catgatgcag gaccagctgg gctacccgca 6540
   gcacccgggc ctcaatgcgc acggcgcagc gcagatgcag cccatgcacc gctacgacgt 6600
   gagcgccctg cagtacaact ccatgaccag ctcgcagacc tacatgaacg gctcgcccac 6660
   ctacagcatg tcctactcgc agcagggcac ccctggcatg gctcttggct ccatgggttc 6720
   ggtggtcaag tccgaggcca gctccagccc ccctgtggtt acctcttcct cccactccag 6780
   ggcgccctgc caggccgggg acctccggga catgatcagc atgtatctcc ccggcgccga 6840
   ggtgccggaa cccgccgccc ccagcagact tcacatgtcc cagcactacc agagcggccc 6900
   ggtgcccggc acggccatta acggcacact gcccctctca cacatgtgag ggccggacag 6960
   cgaactggag gggggagaaa ttttcaaaga aaaacgaggg aaatgggagg ggtgcaaaag 7020
   aggagagtaa gaaacagcat ggagaaaacc cggtacgctc aaaaagaaaa aggaaaaaaa 7080
   aaaatcccat cacccacagc aaatgacagc tgcaaaagag aacaccaatc ccatccacac 7140
   tcacgcaaaa accgcgatgc cgacaagaaa acttttatga gagagatcct ggacttcttt 7200
   ttgggggact atttttgtac agagaaaacc tggggagggt ggggagggcg ggggaatgga 7260
   ccttgtatag atctggagga aagaaagcta cgaaaaactt tttaaaagtt ctagtggtac 7320
   ggtaggagct ttgcaggaag tttgcaaaag tctttaccaa taatatttag agctagtctc 7380
   caagcgacga aaaaaatgtt ttaatatttg caagcaactt ttgtacagta tttatcgaga 7440
   taaacatggc aatcaaaatg tccattgttt ataagctgag aatttgccaa tatttttcaa 7500
   ggagaggctt cttgctgaat tttgattctg cagctgaaat ttaggacagt tgcaaacgtg 7560
   aaaagaagaa aattattcaa atttggacat tttaattgtt taaaaattgt acaaaaggaa 7620
   aaaattagaa taagtactgg cgaaccatct ctgtggtctt gtttaaaaag ggcaaaagtt 7680
   ttagactgta ctaaatttta taacttactg ttaaaagcaa aaatggccat gcaggttgac 7740
   accgttggta atttataata gcttttgttc gatcccaact ttccattttg ttcagataaa 7800
   aaaaaccatg aaattactgt gtttgaaata ttttcttatg gtttgtaata tttctgtaaa 7860
   tttattgtga tattttaagg ttttcccccc tttattttcc gtagttgtat tttaaaagat 7920
   tcggctctgt attatttgaa tcagtctgcc gagaatccat gtatatattt gaactaatat 7980
   catccttata acaggtacat tttcaactta agtttttact ccattatgca cagtttgaga 8040
   taaataaatt tttgaaatat ggacactgaa aaaaaaaaaa aaaaaacctc gactgtgcct 8100
   tctagttgcc agccatctgt tgtttgcccc tcccccgtgc cttccttgac cctggaaggt 8160
   gccactccca ctgtcctttc ctaataaaat gaggaaattg catcgcattg tctgagtagg 8220
   tgtcattcta ttctgggggg tggggtgggg caggacagca agggggagga ttgggaagac 8280
   aatagcaggc atgctgggga tgcggtgggc tctatgggac ggatcgggag atctcccgat 8340
   cccctatggt gcactctcag tacaatctgc tctgatgccg catagttaag ccagtatctg 8400
   ctccctgctt gtgtgttgga ggtcgctgag tagtgcgcga gcaaaattta agctacaaca 8460
   aggcaaggct tgaccgacaa ttgcatgaag aatctgctta gggttaggcg ttttgcgctg 8520
   cttcgcgatg tacgggccag atatacgcgt tgacattgat tattgactag ttattaatag 8580
   taatcaatta cggggtcatt agttcatagc ccatatatgg agttccgcgt tacataactt 8640
   acggtaaatg gcccgcctgg ctgaccgccc aacgaccccc gcccattgac gtcaataatg 8700
   acgtatgttc ccatagtaac gccaataggg actttccatt gacgtcaatg ggtggagtat 8760
   ttacggtaaa ctgcccactt ggcagtacat caagtgtatc atatgccaag tacgccccct 8820
   attgacgtca atgacggtaa atggcccgcc tggcattatg cccagtacat gaccttatgg 8880
   gactttccta cttggcagta catctacgta ttagtcatcg ctattaccat ggtgatgcgg 8940
   ttttggcagt acatcaatgg gcgtggatag cggtttgact cacggggatt tccaagtctc 9000
   caccccattg acgtcaatgg gagtttgttt tggaaccaaa atcaacggga ctttccaaaa 9060
   tgtcgtaaca actccgcccc attgacgcaa atgggcggta ggcgtgtacg gtgggaggtc 9120
   tatataagca gagctctccc tatcagtgat agagatctcc ctatcagtga tagagatcgt 9180
   cgacgagctc gtttagtgaa ccgtcagatc gcctggagac gccatccacg ctgttttgac 9240
   ctccatagaa gacaccggga ccgatccagc ctccggactc tagcgtttaa acttaagctt 9300
   accatggttg tcatggggga ggtggtggcg cttggtggcc actggcggcc gaggtagagg 9360
   cagtggcgct tgagttggtc gggggcagcg gcagatttga ggcttaagca acttcttccg 9420
   gggaagagtg ccagtgcagc cactgttaca attcaagatc ttgatctata tccatagatt 9480
   ggaatattgg tgggccagca atcctcagac gcctcactta ggacaaatga ggaaactgag 9540
   gcttggtgaa gttacgaaac ttgtccaaaa tcacacaact tgtaaagggc acagccaaga 9600
   ttcagagcca ggctgtaaaa attaaaatga acaaattacg gcaaagtttt aggagaaaga 9660
   aggatgttta tgttccagag gccagtcgtc cacatcagtg gcagacagat gaagaaggcg 9720
   ttcgcaccgg aaaatgtagc ttcccggtta agtaccttgg ccatgtagaa gttgatgaat 9780
   caagaggaat gcacatctgt gaagatgctg taaaaagatt gaaagctgaa aggaagttct 9840
   tcaaaggctt ctttggaaaa actggaaaga aagcagttaa agcagttctg tgggtctcag 9900
   cagatggact cagagttgtg gatgaaaaaa ctaaggacct catagttgac cagacgatag 9960
   agaaagtttc tttctgtgcc ccagacagga actttgatag agccttttct tacatatgcc 10020
   gtgatggcac cactcgtcgc tggatctgtc actgcttcat ggctgtcaag gacacaggtg 10080
   aaaggttgag ccatgcagta ggctgtgctt ttgcagcctg tttagagcgc aagcagaagc 10140
   gggagaagga atgtggagtg actgctactt ttgatgctag tcggaccact tttacaagag 10200
   aaggatcatt ccgtgtcaca acagccactg aacaagcaga aagagaggag atcatgaaac 10260
   aaatgcaaga tgccaagaaa gctgaaacag ataagatagt cgttggttca tcagttgccc 10320
   ctggcaacac tgccccatcc ccatcctctc ccacctctcc tacttctgat gccacgacct 10380
   ctctggagat gaacaatcct catgccatcc cacgccggca tgctccaatt gaacagcttg 10440
   ctcgccaagg ctctttccga ggttttcctg ctcttagcca gaagatgtca ccctttaaac 10500
   gccaactatc cctacgcatc aatgagttgc cttccactat gcagaggaag actgatttcc 10560
   ccattaaaaa tgcagtgcca gaagtagaag gggaggcaga gagcatcagc tccctgtgct 10620
   cacagatcac caatgccttc agcacacctg aggacccctt ctcatctgct ccgatgacca 10680
   aaccagtgac agtggtggca ccacaatctc ctaccttcca agctaatggc actgactcag 10740
   ccttccatgt gcttgctaag ccagcccata ctgctctagc acccgtagca atgcctgtgc 10800
   gtgaaaccaa cccttgggcc catgcccctg atgctgctaa caaggaaatt gcagccacat 10860
   gttcggggac cgagtggggt caatcttctg gtgctgcctc tccaggtctc ttccaggccg 10920
   gtcatagacg tactccctct gaggccgacc gatggttaga agaggtgtct aagagcgtcc 10980
   gggctcagca gccccaggcc tcagctgctc ctctgcagcc agttctccag cctcctccac 11040
   ccactgccat ctcccagcca gcatcacctt tccaagggaa tgcattcctc acctctcagc 11100
   ctgtgccagt gggtgtggtc ccagccctgc aaccagcctt tgtccctgcc cagtcctatc 11160
   ctgtggccaa tggaatgccc tatccagccc ctaatgtgcc tgtggtgggc atcactccct 11220
   cccagatggt ggccaacgta tttggcactg caggccaccc tcaggctgcc catccccatc 11280
   agtcacccag cctggtcagg cagcagacat tccctcacta cgaggcaagc agtgctacca 11340
   ccagtccctt ctttaagcct cctgctcagc acctcaacgg ttctgcagct ttcaatggtg 11400
   tagatgatgg caggttggcc tcagcagaca ggcatacaga ggttcctaca ggcacctgcc 11460
   cagtggatcc ttttgaagcc cagtgggctg cattagaaaa taagtccaag cagcgtacta 11520
   atccctcccc taccaaccct ttctccagtg acttacagaa gacgtttgaa attgaacttt 11580
   aagcaatcat tatggctatg tatcttgtcc ataccagaca gggagcaggg ggtagcggtc 11640
   aaaggagcaa aacagacttt gtctcctgat tagtactctt ttcactaatc ccaaaggtcc 11700
   caaggaacaa gtccaggccc agagtactgt gaggggtgat tttgaaagac atgggaaaaa 11760
   gcattcctag agaaaagctg ccttgcaatt aggctaaaga agtcaaggaa atgttgcttt 11820
   ctgtactccc tcttccctta cccccttaca aatctctggc aacagagagg caaagtatct 11880
   gaacaagaat ctatattcca agcacattta ctgaaatgta aaacacaaca ggaagcaaag 11940
   caatctccct ttgtttttca ggccattcac ctgcctcctg tcagtagtgg cctgtattag 12000
   agatcaagaa gagtggtttg tgctcaggct ggggaacaga gaggcacgct atgctgccag 12060
   aattcccagg agggcatatc agcaactgcc cagcagagct atattttggg ggagaagttg 12120
   agcttccatt ttgagtaaca gaataaatat tatatatatc aaaagccaaa atctttattt 12180
   ttatgcattt agaatatttt aaatagttct cagatattaa gaagttgtat gagttgtaag 12240
   taatcttgcc aaaggtaaag gggctagttg taagaaattg tacataagat tgatttatca 12300
   ttgatgccta ctgaaataaa aagaggaaag gctggaagct gcagacagga tccctagctt 12360
   gttttctgtc agtcattcat tgtaagtagc acattgcaac aacaatcatg cttatgacca 12420
   atacagtcac taggttgtag ttttttttaa ataaaggaaa agcagtattg tcctggtttt 12480
   aaacctatga tggaattcta atgtcattat tttaatggaa tcaatcgaaa tatgctctat 12540
   agagaatata tcttttatat attgctgcag tttccttatg ttaatccttt aacactaagg 12600
   taacatgaca taatcatacc atagaaggga acacaggtta ccatattggt ttgtaatatg 12660
   ggtcttggtg ggttttgttt tatcctttaa attttgttcc catgagtttt gtggggatgg 12720
   ggattctggt tttattagct ttgtgtgtgt cctcttcccc caaaccccct tttggtgaga 12780
   acatcccctt gacagttgca gcctcttgac ctcggataac aataagagag ctcatctcat 12840
   ttttactttt gaacgttggc cttacaatca aatgtaagtt atatatattt gtactgatga 12900
   aaatttataa tctgctttaa caaaaataaa tgttcatggt agaagctttt aaaaaaaaaa 12960
   aaacctcgac tgtgccttct agttgccagc catctgttgt ttgcccctcc cccgtgcctt 13020
   ccttgaccct ggaaggtgcc actcccactg tcctttccta ataaaatgag gaaattgcat 13080
   cgcattgtct gagtaggtgt cattctattc tggggggtgg ggtggggcag gacagcaagg 13140
   gggaggattg ggaagacaat agcaggcatg ctggggatgc ggtgggctct atgggacgga 13200
   tcgggagatc tcccgatccc ctatggtgca ctctcagtac aatctgctct gatgccgcat 13260
   agttaagcca gtatctgctc cctgcttgtg tgttggaggt cgctgagtag tgcgcgagca 13320
   aaatttaagc tacaacaagg caaggcttga ccgacaattg catgaagaat ctgcttaggg 13380
   ttaggcgttt tgcgctgctt cgcgatgtac gggccagata tacgcgttga cattgattat 13440
   tgactagtta ttaatagtaa tcaattacgg ggtcattagt tcatagccca tatatggagt 13500
   tccgcgttac ataacttacg gtaaatggcc cgcctggctg accgcccaac gacccccgcc 13560
   cattgacgtc aataatgacg tatgttccca tagtaacgcc aatagggact ttccattgac 13620
   gtcaatgggt ggagtattta cggtaaactg cccacttggc agtacatcaa gtgtatcata 13680
   tgccaagtac gccccctatt gacgtcaatg acggtaaatg gcccgcctgg cattatgccc 13740
   agtacatgac cttatgggac tttcctactt ggcagtacat ctacgtatta gtcatcgcta 13800
   ttaccatggt gatgcggttt tggcagtaca tcaatgggcg tggatagcgg tttgactcac 13860
   ggggatttcc aagtctccac cccattgacg tcaatgggag tttgttttgg aaccaaaatc 13920
   aacgggactt tccaaaatgt cgtaacaact ccgccccatt gacgcaaatg ggcggtaggc 13980
   gtgtacggtg ggaggtctat ataagcagag ctctccctat cagtgataga gatctcccta 14040
   tcagtgatag agatcgtcga cgagctcgtt tagtgaaccg tcagatcgcc tggagacgcc 14100
   atccacgctg ttttgacctc catagaagac accgggaccg atccagcctc cggactctag 14160
   cgtttaaact taagcttacc atggtgagca agggcgagga gctgttcacc ggggtggtgc 14220
   ccatcctggt cgagctggac ggcgacgtaa acggccacaa gttcagcgtg tccggcgagg 14280
   gcgagggcga tgccacctac ggcaagctga ccctgaagtt catctgcacc accggcaagc 14340
   tgcccgtgcc ctggcccacc ctcgtgacca ccttcaccta cggcgtgcag tgcttcgccc 14400
   gctaccccga ccacatgaag cagcacgact tcttcaagtc cgccatgccc gaaggctacg 14460
   tccaggagcg caccatcttc ttcaaggacg acggcaacta caagacccgc gccgaggtga 14520
   agttcgaggg cgacaccctg gtgaaccgca tcgagctgaa gggcatcgac ttcaaggagg 14580
   acggcaacat cctggggcac aagctggagt acaactacaa cagccacaag gtctatatca 14640
   ccgccgacaa gcagaagaac ggcatcaagg tgaacttcaa gacccgccac aacatcgagg 14700
   acggcagcgt gcagctcgcc gaccactacc agcagaacac ccccatcggc gacggccccg 14760
   tgctgctgcc cgacaaccac tacctgagca cccagtccgc cctgagcaaa gaccccaacg 14820
   agaagcgcga tcacatggtc ctgctggagt tcgtgaccgc cgccgggatc actctcggca 14880
   tggacgagct gtacaagtaa cctcgactgt gccttctagt tgccagccat ctgttgtttg 14940
   cccctccccc gtgccttcct tgaccctgga aggtgccact cccactgtcc tttcctaata 15000
   aaatgaggaa attgcatcgc attgtctgag taggtgtcat tctattctgg ggggtggggt 15060
   ggggcaggac agcaaggggg aggattggga agacaatagc aggcatgctg gggatgcggt 15120
   gggctctatg ggacggatcg ggagatctcc cgatccccta tggtgcactc tcagtacaat 15180
   ctgctctgat gccgcatagt taagccagta tctgctccct gcttgtgtgt tggaggtcgc 15240
   tgagtagtgc gcgagcaaaa tttaagctac aacaaggcaa ggcttgaccg acaattgcat 15300
   gaagaatctg cttagggtta ggcgttttgc gctgcttcgc gatgtacggg ccagatatac 15360
   gcgttgacat tgattattga ctagttatta atagtaatca attacggggt cattagttca 15420
   tagcccatat atggagttcc gcgttacata acttacggta aatggcccgc ctggctgacc 15480
   gcccaacgac ccccgcccat tgacgtcaat aatgacgtat gttcccatag taacgccaat 15540
   agggactttc cattgacgtc aatgggtgga gtatttacgg taaactgccc acttggcagt 15600
   acatcaagtg tatcatatgc caagtacgcc ccctattgac gtcaatgacg gtaaatggcc 15660
   cgcctggcat tatgcccagt acatgacctt atgggacttt cctacttggc agtacatcta 15720
   cgtattagtc atcgctatta ccatggtgat gcggttttgg cagtacatca atgggcgtgg 15780
   atagcggttt gactcacggg gatttccaag tctccacccc attgacgtca atgggagttt 15840
   gttttggaac caaaatcaac gggactttcc aaaatgtcgt aacaactccg ccccattgac 15900
   gcaaatgggc ggtaggcgtg tacggtggga ggtctatata agcagagctc gtgagtttgg 15960
   ggacccttga ttgttctttc tttttcgcta ttgtaaaatt catgttatat ggagggggca 16020
   aagttttcag ggtgttgttt agaatgggaa gatgtccctt gtatcaccat ggaccctcat 16080
   gataattttg tttctttcac tttctactct gttgacaacc attgtctcct cttattttct 16140
   tttcattttc tgtaactttt tcgttaaact ttagcttgca tttgtaacga atttttaaat 16200
   tcacttttgt ttatttgtca gattgtaagt actttctcta atcacttttt tttcaaggca 16260
   atcagggtat attatattgt acttcagcac agttttagag aacaattgtt ataattaaat 16320
   gataaggtag aatatttctg catataaatt ctggctggcg tggaaatatt cttattggta 16380
   gaaacaacta catcctggtc atcatcctgc ctttctcttt atggttacaa tgatatacac 16440
   tgtttgagat gaggataaaa tactctgagt ccaaaccggg cccctctgct aaccatgttc 16500
   atgccttctt ctttttccta cagctcctgg gcaacgtgct ggttattgtg ctgtctcatc 16560
   attttggcaa agaattgtaa tacgactcac tatagggcga attgatatgt ctagattaga 16620
   taaaagtaaa gtgattaaca gcgcattaga gctgcatgtc tagattagat aaaagtaaag 16680
   tgattaacag cgcattagag ctgcttaatg aggtcggaat cgaaggttta acaacccgta 16740
   aactcgccca gaagctaggt gtagagcagc ctacattgta ttggcatgta aaaaataagc 16800
   gggctttgct cgacgcctta gccattgaga tgttagatag gcaccatact cacttttgcc 16860
   ctttagaagg ggaaagctgg caagattttt tacgtaataa cgctaaaagt tttagatgtg 16920
   ctttactaag tcatcgcgat ggagcaaaag tacatttagg tacacggcct acagaaaaac 16980
   agtatgaaac tctcgaaaat caattagcct ttttatgcca acaaggtttt tcactagaga 17040
   atgcattata tgcactcagc gctgtggggc attttacttt aggttgcgta ttggaagatc 17100
   aagagcatca agtcgctaaa gaagaaaggg aaacacctac tactgatagt atgccgccat 17160
   tattacgaca agctatcgaa ttatttgatc accaaggtgc agagccagcc ttcttattcg 17220
   gccttgaatt gatcatatgc ggattagaaa aacaacttaa atgtgaaagt gggtccgcgt 17280
   acagcggatc ccgggaattc agatcttatt aaagcagaac ttgtttattg cagcttataa 17340
   tggttacaaa taaagcaata gcatcacaaa tttcacaaat aaagcatttt tttcactgca 17400
   ttctagttgt ggtttgtcca aactcatcaa tgtatcttat catgtctggt caatgtgtgt 17460
   cagttagggt gtggaaagtc cccaggctcc ccagcaggca gaagtatgca aagcatgcat 17520
   ctcaattagt cagcaaccag gtgtggaaag tccccaggct ccccagcagg cagaagtatg 17580
   caaagcatgc atctcaatta gtcagcaacc atagtcccgc ccctaactcc gcccatcccg 17640
   cccctaactc cgcccagttc cgcccattct ccgccccatg gctgactaat tttttttatt 17700
   tatgcagagg ccgaggccgc ctctgcctct gagctattcc agaagtagtg aggaggcttt 17760
   tttggaggcc taggcttttg caaaaagctc cccatagtga ctggatatgt tgtgttttac 17820
   agtattatgt agtctgtttt ttatgcaaaa tctaatttaa tatattgata tttatatcat 17880
   tttacgtttc tcgttcagct ttcttgtaca aagtggttga tatccagcac agtggcggcc 17940
   gctcgagtct agagggcccg cggttcgaag gtaagcctat ccctaaccct ctcctcggtc 18000
   tcgattctac gcgtaccggt tagtaatgag tttggaatta attctgtgga atgtgtgtca 18060
   gttagggtgt ggaaagtccc caggctcccc agcaggcaga agtatgcaaa gcatgcatct 18120
   caattagtca gcaaccaggt gtggaaagtc cccaggctcc ccagcaggca gaagtatgca 18180
   aagcatgcat ctcaattagt cagcaaccat agtcccgccc ctaactccgc ccatcccgcc 18240
   cctaactccg cccagttccg cccattctcc gccccatggc tgactaattt ttttatttta 18300
   tgcagaggcc gaggccgcct ctgcctctga gctattccag aagtagtgag gaggcttttt 18360
   tggaggccta ggcttttgca aaaagctccc gggagcttgt atatccattt tcggatctga 18420
   tcagcacgtg ttgacaatta atcatcggca tagtatatcg gcatagtata atacgacaag 18480
   gtgaggaact aaaccatggc caagcctttg tctcaagaag aatccaccct cattgaaaga 18540
   gcaacggcta caatcaacag catccccatc tctgaagact acagcgtcgc cagcgcagct 18600
   ctctctagcg acggccgcat cttcactggt gtcaatgtat atcattttac tgggggacct 18660
   tgtgcagaac tcgtggtgct gggcactgct gctgctgcgg cagctggcaa cctgacttgt 18720
   atcgtcgcga tcggaaatga gaacaggggc atcttgagcc cctgcggacg gtgccgacag 18780
   gtgcttctcg atctgcatcc tgggatcaaa gccatagtga aggacagtga tggacagccg 18840
   acggcagttg ggattcgtga attgctgccc tctggttatg tgtgggaggg ctaagcacaa 18900
   ttcgagctcg gtacctttaa gaccaatgac ttacaaggca gctgtagatc ttagccactt 18960
   tttaaaagaa aaggggggac tggaagggct aattcactcc caacgaagac aagatctgct 19020
   ttttgcttgt actgggtctc tctggttaga ccagatctga gcctgggagc tctctggcta 19080
   actagggaac ccactgctta agcctcaata aagcttgcct tgagtgcttc aagtagtgtg 19140
   tgcccgtctg ttgtgtgact ctggtaacta gagatccctc agaccctttt agtcagtgtg 19200
   gaaaatctct agcagtagta gttcatgtca tcttattatt cagtatttat aacttgcaaa 19260
   gaaatgaata tcagagagtg agaggaactt gtttattgca gcttataatg gttacaaata 19320
   aagcaatagc atcacaaatt tcacaaataa agcatttttt tcactgcatt ctagttgtgg 19380
   tttgtccaaa ctcatcaatg tatcttatca tgtctggctc tagctatccc gcccctaact 19440
   ccgcccatcc cgcccctaac tccgcccagt tccgcccatt ctccgcccca tggctgacta 19500
   atttttttta tttatgcaga ggccgaggcc gcctcggcct ctgagctatt ccagaagtag 19560
   tgaggaggct tttttggagg cctagggacg tacccaattc gccctatagt gagtcgtatt 19620
   acgcgcgctc actggccgtc gttttacaac gtcgtgactg ggaaaaccct ggcgttaccc 19680
   aacttaatcg ccttgcagca catccccctt tcgccagctg gcgtaatagc gaagaggccc 19740
   gcaccgatcg cccttcccaa cagttgcgca gcctgaatgg cgaatgggac gcgccctgta 19800
   gcggcgcatt aagcgcggcg ggtgtggtgg ttacgcgcag cgtgaccgct acacttgcca 19860
   gcgccctagc gcccgctcct ttcgctttct tcccttcctt tctcgccacg ttcgccggct 19920
   ttccccgtca agctctaaat cgggggctcc ctttagggtt ccgatttagt gctttacggc 19980
   acctcgaccc caaaaaactt gattagggtg atggttcacg tagtgggcca tcgccctgat 20040
   agacggtttt tcgccctttg acgttggagt ccacgttctt taatagtgga ctcttgttcc 20100
   aaactggaac aacactcaac cctatctcgg tctattcttt tgatttataa gggattttgc 20160
   cgatttcggc ctattggtta aaaaatgagc tgatttaaca aaaatttaac gcgaatttta 20220
   acaaaatatt aacgcttaca atttaggtgg cacttttcgg ggaaatgtgc gcggaacccc 20280
   tatttgttta tttttctaaa tacattcaaa tatgtatccg ctcatgagac aataaccctg 20340
   ataaatgctt caataatatt gaaaaaggaa gagtatgagt attcaacatt tccgtgtcgc 20400
   ccttattccc ttttttgcgg cattttgcct tcctgttttt gctcacccag aaacgctggt 20460
   gaaagtaaaa gatgctgaag atcagttggg tgcacgagtg ggttacatcg aactggatct 20520
   caacagcggt aagatccttg agagttttcg ccccgaagaa cgttttccaa tgatgagcac 20580
   ttttaaagtt ctgctatgtg gcgcggtatt atcccgtatt gacgccgggc aagagcaact 20640
   cggtcgccgc atacactatt ctcagaatga cttggttgag tactcaccag tcacagaaaa 20700
   gcatcttacg gatggcatga cagtaagaga attatgcagt gctgccataa ccatgagtga 20760
   taacactgcg gccaacttac ttctgacaac gatcggagga ccgaaggagc taaccgcttt 20820
   tttgcacaac atgggggatc atgtaactcg ccttgatcgt tgggaaccgg agctgaatga 20880
   agccatacca aacgacgagc gtgacaccac gatgcctgta gcaatggcaa caacgttgcg 20940
   caaactatta actggcgaac tacttactct agcttcccgg caacaattaa tagactggat 21000
   ggaggcggat aaagttgcag gaccacttct gcgctcggcc cttccggctg gctggtttat 21060
   tgctgataaa tctggagccg gtgagcgtgg gtctcgcggt atcattgcag cactggggcc 21120
   agatggtaag ccctcccgta tcgtagttat ctacacgacg gggagtcagg caactatgga 21180
   tgaacgaaat agacagatcg ctgagatagg tgcctcactg attaagcatt ggtaactgtc 21240
   agaccaagtt tactcatata tactttagat tgatttaaaa cttcattttt aatttaaaag 21300
   gatctaggtg aagatccttt ttgataatct catgaccaaa atcccttaac gtgagttttc 21360
   gttccactga gcgtcagacc ccgtagaaaa gatcaaagga tcttcttgag atcctttttt 21420
   tctgcgcgta atctgctgct tgcaaacaaa aaaaccaccg ctaccagcgg tggtttgttt 21480
   gccggatcaa gagctaccaa ctctttttcc gaaggtaact ggcttcagca gagcgcagat 21540
   accaaatact gttcttctag tgtagccgta gttaggccac cacttcaaga actctgtagc 21600
   accgcctaca tacctcgctc tgctaatcct gttaccagtg gctgctgcca gtggcgataa 21660
   gtcgtgtctt accgggttgg actcaagacg atagttaccg gataaggcgc agcggtcggg 21720
   ctgaacgggg ggttcgtgca cacagcccag cttggagcga acgacctaca ccgaactgag 21780
   atacctacag cgtgagctat gagaaagcgc cacgcttccc gaagggagaa aggcggacag 21840
   gtatccggta agcggcaggg tcggaacagg agagcgcacg agggagcttc cagggggaaa 21900
   cgcctggtat ctttatagtc ctgtcgggtt tcgccacctc tgacttgagc gtcgattttt 21960
   gtgatgctcg tcaggggggc ggagcctatg gaaaaacgcc agcaacgcgg cctttttacg 22020
   gttcctggcc ttttgctggc cttttgctca catgttcttt cctgcgttat cccctgattc 22080
   tgtggataac cgtattaccg cctttgagtg agctgatacc gctcgccgca gccgaacgac 22140
   cgagcgcagc gagtcagtga gcgaggaagc ggaagagcgc ccaatacgca aaccgcctct 22200
   ccccgcgcgt tggccgattc attaatgcag ctggcacgac aggtttcccg actggaaagc 22260
   gggcagtgag cgcaacgcaa ttaatgtgag ttagctcact cattaggcac cccaggcttt 22320
   acactttatg cttccggctc gtatgttgtg tggaattgtg agcggataac aatttcacac 22380
   aggaaacagc tatgaccatg attacgccaa gcgcgcaatt aaccctcact aaagggaaca 22440
   aaagctggag ctgcaagctt                                             22460
<210> 3
   <211> 1751
   <212> DNA
   <213> Artificial Sequence
<220>
   <223> Description of Artificial Sequence: Synthetic polynucleotide
<400> 3
   gtcgaccagt ggatcctgga ggcttgctga aggctgtatg ctgatcgggt gtaaactgag 60
   cttggttttg gccactgact gaccaagctc attacacccg atcaggacac aaggcctgtt 120
   actagcactc acatggaaca aatggcccag atcctggagg cttgctgaag gctgtatgct 180
   gataccaggc aggataaggc cagttttggc cactgactga ctggccttac tgcctggtat 240
   caggacacaa ggcctgttac tagcactcac atggaacaaa tggcccagat cctggaggct 300
   tgctgaaggc tgtatgctgt gaccaggatg accaatccat gttttggcca ctgactgaca 360
   tggattgcat cctggtcaca ggacacaagg cctgttacta gcactcacat ggaacaaatg 420
   gcccagatcc tggaggcttg ctgaaggctg tatgctgata gcttggtcca acctgttagt 480
   tttggccact gactgactaa caggtgacca agctatcagg acacaaggcc tgttactagc 540
   actcacatgg aacaaatggc ccagatctcc ccagtggaaa gacgcgcagg caaaacgcac 600
   cacgtgacgg agcgtgaccg cgcgccgagc gcgcgccaag gtcgggcagg aagagggcct 660
   atttcccatg attccttcat atttgcatat acgatacaag gctgttagag agataattag 720
   aattaatttg actgtaaaca caaagatatt agtacaaaat acgtgacgta gaaagtaata 780
   atttcttggg tagtttgcag ttttaaaatt atgttttaaa atggactatc atatgcttac 840
   cgtaacttga aagtatttcg atttcttggg tttatatatc ttgtggaaag gacggtgctc 900
   gcttcggcag cacgtcgtgc tagggttctt gggttttctc gcaacagcag gttctgcaat 960
   gggcgcggcg tccctgaccg tgtcggctca gtcccggact ttactggccg ggatagtgca 1020
   gcaacagcaa cagctgttgg acgtggtcaa gagacaacaa gaactgttgc gactgaccgt 1080
   ctggggaacg aaaaacctcc aggcaagagt cactgctata gagaagtacc tacaggacca 1140
   ggcgcggcta aattcatggg gatgtctaga cctagagcgg acttcggtcc gctttttccc 1200
   cagtggaaag acgcgcaggc aaaacgcacc acgtgacgga gcgtgaccgc gcgccgagcg 1260
   cgcgccaagg tcgggcagga agagggccta tttcccatga ttccttcata tttgcatata 1320
   cgatacaagg ctgttagaga gataattaga attaatttga ctgtaaacac aaagatatta 1380
   gtacaaaata cgtgacgtag aaagtaataa tttcttgggt agtttgcagt tttaaaatta 1440
   tgttttaaaa tggactatca tatgcttacc gtaacttgaa agtatttcga tttcttgggt 1500
   ttatatatct tgtggaaagg acggtgctcg cttcggcagc acgtcggtcg ctctgcggag 1560
   aggctggcag attgagccct gggaggttct ctccagcact agcaggtaga gcctgggtgt 1620
   tccctgctag actctcacca gtgcttggcc ggcactgggc agacggctcc acgcttgctt 1680
   gcttaaagac ctcttaataa agctgctcta gacctagagc ggacttcggt ccgctttttt 1740
   acgtactcga g                                                      1751
<210> 4
   <211> 64
   <212> DNA
   <213> Artificial Sequence
<220>
   <223> Description of Artificial Sequence: Synthetic oligonucleotide
<400> 4
   tgctgatacc aggcaggata aggccagttt tggccactga ctgactggcc ttactgcctg 60
   gtat                                                              64
<210> 5
   <211> 64
   <212> DNA
   <213> Artificial Sequence
<220>
   <223> Description of Artificial Sequence: Synthetic oligonucleotide
<400> 5
   cctgatacca ggcagtaagg ccagtcagtc agtggccaaa actggcctta tcctgcctgg 60
   tatc 64
<210> 6
   <211> 64
   <212> DNA
   <213> Artificial Sequence
<220>
   <223> Description of Artificial Sequence: Synthetic oligonucleotide
<400> 6
   tgctgtgacc aggatgacca atccatgttt tggccactga ctgacatgga ttgcatcctg 60
   gtca 64
<210> 7
   <211> 64
   <212> DNA
   <213> Artificial Sequence
<220>
   <223> Description of Artificial Sequence: Synthetic oligonucleotide
<400> 7
   cctgtgacca ggatgcaatc catgtcagtc agtggccaaa acatggattg gtcatcctgg 60
   tcac 64
<210> 8
   <211> 64
   <212> DNA
   <213> Artificial Sequence
<220>
   <223> Description of Artificial Sequence: Synthetic oligonucleotide
<400> 8
   tgctgatcgg gtgtaaactg agcttggttt tggccactga ctgaccaagc tcattacacc 60
   cgat 64
<210> 9
   <211> 64
   <212> DNA
   <213> Artificial Sequence
<220>
   <223> Description of Artificial Sequence: Synthetic oligonucleotide
<400> 9
   cctgatcggg tgtaatgagc ttggtcagtc agtggccaaa accaagctca gtttacaccc 60
   gatc 64
<210> 10
   <211> 64
   <212> DNA
   <213> Artificial Sequence
<220>
   <223> Description of Artificial Sequence: Synthetic oligonucleotide
<400> 10
   tgctgatagc ttggtccaac ctgttagttt tggccactga ctgactaaca ggtgaccaag 60
   ctat 64
<210> 11
   <211> 64
   <212> DNA
   <213> Artificial Sequence
<220>
   <223> Description of Artificial Sequence: Synthetic oligonucleotide
<400> 11
   cctgatagct tggtcacctg ttagtcagtc agtggccaaa actaacaggt tggaccaagc 60
   tatc 64
<210> 12
   <211> 160
   <212> DNA
   <213> Artificial Sequence
<220>
   <223> Description of Artificial Sequence: Synthetic polynucleotide
<400> 12
   gtcgctctgc ggagaggctg gcagattgag ccctgggagg ttctctccag cactagcagg 60
   tagagcctgg gtgttccctg ctagactctc accagtgctt ggccggcact gggcagacgg 120
   ctccacgctt gcttgcttaa agacctctta ataaagctgc 160
<210> 13
   <211> 247
   <212> DNA
   <213> Artificial Sequence
<220>
   <223> Description of Artificial Sequence: Synthetic polynucleotide
<400> 13
   tgctagggtt cttgggtttt ctcgcaacag caggttctgc aatgggcgcg gcgtccctga 60
   ccgtgtcggc tcagtcccgg actttactgg ccgggatagt gcagcaacag caacagctgt 120
   tggacgtggt caagagacaa caagaactgt tgcgactgac cgtctgggga acgaaaaacc 180
   tccaggcaag agtcactgct atagagaagt acctacagga ccaggcgcgg ctaaattcat 240
   ggggatg                                                           247
<210> 14
   <211> 25
   <212> DNA
   <213> Artificial Sequence
<220>
   <223> Description of Combined DNA/RNA Molecule: Synthetic oligonucleotide
<220>
   <223> Description of Artificial Sequence: Synthetic oligonucleotide
<400> 14
   gugcucgcuu cggcagcacg tcgac 25
<210> 15
   <211> 26
   <212> RNA
   <213> Artificial Sequence
<220>
   <223> Description of Artificial Sequence: Synthetic olgionucleotide
<400> 15
   ucuagagcgg acuucggucc gcuuuu 26
<210> 16
   <211> 25
   <212> DNA
   <213> Artificial Sequence
<220>
   <223> Description of Combined DNA/RNA Molecule: Synthetic oligonucleotide
<220>
   <223> Description of Artificial Sequence: Synthetic oligonucleotide
<400> 16
   gugcucgcuu cggcagcacg tcgac 25
<210> 17
   <211> 26
   <212> RNA
   <213> Artificial Sequence
<220>
   <223> Description of Artificial Sequence: Synthetic oligonucleotide
<400> 17
   ucuagagcgg acuucggucc gcuuuu 26
<210> 18
   <211> 606
   <212> DNA
   <213> Homo sapiens
<400> 18
   atgagccgga gggagggaag tctggaagac ccccagactg attcctcagt ctcacttctt 60
   ccccacttgg aggccaagat ccgtcagaca cacagccttg cgcacctcct caccaaatac 120
   gctgagcagc tgctccagga atatgtgcag ctccagggag accccttcgg gctgcccagc 180
   ttctcgccgc cgcggctgcc ggtggccggc ctgagcgccc cggctccgag ccacgcgggg 240
   ctgccagtgc acgagcggct gcggctggac gcggcggcgc tggccgcgct gcccccgctg 300
   ctggacgcag tgtgtcgccg ccaggccgag ctgaacccgc gcgcgccgcg cctgctgcgc 360
   cgcctggagg acgcggcgcg ccaggcccgg gccctgggcg ccgccgtgga ggccttgctg 420
   gccgcgctgg gcgccgccaa ccgcgggccc cgggccgagc cccccgccgc caccgcctca 480
   gccgcctccg ccaccggggt cttccccgcc aaggtgctgg ggctccgcgt ttgcggcctc 540
   taccgcgagt ggctgagccg caccgagggc gacctgggcc agctgctgcc cgggggctcg 600
   gcctga                                                            606
<210> 19
   <211> 603
   <212> DNA
   <213> Homo sapiens
<400> 19
   atggctttca cagagcattc accgctgacc cctcaccgtc gggacctctg tagccgctct 60
   atctggctag caaggaagat tcgttcagac ctgactgctc ttacggaatc ctatgtgaag 120
   catcagggcc tgaacaagaa catcaacctg gactctgcgg atgggatgcc agtggcaagc 180
   actgatcagt ggagtgagct gaccgaggca gagcgactcc aagagaacct tcaagcttat 240
   cgtaccttcc atgttttgtt ggccaggctc ttagaagacc agcaggtgca ttttacccca 300
   accgaaggtg acttccatca agctatacat acccttcttc tccaagtcgc tgcctttgca 360
   taccagatag aggagttaat gatactcctg gaatacaaga tcccccgcaa tgaggctgat 420
   gggatgccta ttaatgttgg agatggtggt ctctttgaga agaagctgtg gggcctaaag 480
   gtgctgcagg agctttcaca gtggacagta aggtccatcc atgaccttcg tttcatttct 540
   tctcatcaga ctgggatccc agcacgtggg agccattata ttgctaacaa caagaaaatg 600
   tag                                                               603
<210> 20
   <211> 990
   <212> DNA
   <213> Homo sapiens
<400> 20
   atgttgacgt tgcagacttg gctagtgcaa gccttgttta ttttcctcac cactgaatct 60
   acaggtgaac ttctagatcc atgtggttat atcagtcctg aatctccagt tgtacaactt 120
   cattctaatt tcactgcagt ttgtgtgcta aaggaaaaat gtatggatta ttttcatgta 180
   aatgctaatt acattgtctg gaaaacaaac cattttacta ttcctaagga gcaatatact 240
   atcataaaca gaacagcatc cagtgtcacc tttacagata tagcttcatt aaatattcag 300
   ctcacttgca acattcttac attcggacag cttgaacaga atgtttatgg aatcacaata 360
   atttcaggct tgcctccaga aaaacctaaa aatttgagtt gcattgtgaa cgaggggaag 420
   aaaatgaggt gtgagtggga tggtggaagg gaaacacact tggagacaaa cttcacttta 480
   aaatctgaat gggcaacaca caagtttgct gattgcaaag caaaacgtga cacccccacc 540
   tcatgcactg ttgattattc tactgtgtat tttgtcaaca ttgaagtctg ggtagaagca 600
   gagaatgccc ttgggaaggt tacatcagat catatcaatt ttgatcctgt atataaagtg 660
   aagcccaatc cgccacataa tttatcagtg atcaactcag aggaactgtc tagtatctta 720
   aaattgacat ggaccaaccc aagtattaag agtgttataa tactaaaata taacattcaa 780
   tataggacca aagatgcctc aacttggagc cagattcctc ctgaagacac agcatccacc 840
   cgatcttcat tcactgtcca agaccttaaa ccttttacag aatatgtgtt taggattcgc 900
   tgtatgaagg aagatggtaa gggatactgg agtgactgga gtgaagaagc aagtgggatc 960
   acctatgaag ataacattgc ctccttttga                                  990
<210> 21
   <211> 639
   <212> DNA
   <213> Homo sapiens
<400> 21
   atgaactcct tctccacaag cgccttcggt ccagttgcct tctccctggg gctgctcctg 60
   gtgttgcctg ctgccttccc tgccccagta cccccaggag aagattccaa agatgtagcc 120
   gccccacaca gacagccact cacctcttca gaacgaattg acaaacaaat tcggtacatc 180
   ctcgacggca tctcagccct gagaaaggag acatgtaaca agagtaacat gtgtgaaagc 240
   agcaaagagg cactggcaga aaacaacctg aaccttccaa agatggctga aaaagatgga 300
   tgcttccaat ctggattcaa tgaggagact tgcctggtga aaatcatcac tggtcttttg 360
   gagtttgagg tatacctaga gtacctccag aacagatttg agagtagtga ggaacaagcc 420
   agagctgtgc agatgagtac aaaagtcctg atccagttcc tgcagaaaaa ggcaaagaat 480
   ctagatgcaa taaccacccc tgacccaacc acaaatgcca gcctgctgac gaagctgcag 540
   gcacagaacc agtggctgca ggacatgaca actcatctca ttctgcgcag ctttaaggag 600
   ttcctgcagt ccagcctgag ggctcttcgg caaatgtag                        639
<210> 22
   <211> 756
   <212> DNA
   <213> Homo sapiens
<400> 22
   atggctatga gttctttttt gatcaactca aactatgtcg accccaagtt ccctccatgc 60
   gaggaatatt cacagagcga ttacctaccc agcgaccact cgcccgggta ctacgccggc 120
   ggccagaggc gagagagcag cttccagccg gaggcgggct tcgggcggcg cgcggcgtgc 180
   accgtgcagc gctacgcggc ctgccgggac cctgggcccc cgccgcctcc gccaccaccc 240
   ccgccgcccc cgccaccgcc cggtctgtcc cctcgggctc ctgcgccgcc acccgccggg 300
   gccctcctcc cggagcccgg ccagcgctgc gaggcggtca gcagcagccc cccgccgcct 360
   ccctgcgccc agaaccccct gcaccccagc ccgtcccact ccgcgtgcaa agagcccgtc 420
   gtctacccct ggatgcgcaa agttcacgtg agcacggtaa accccaatta cgccggcggg 480
   gagcccaagc gctctcggac cgcctacacg cgccagcagg tcttggagct ggagaaggaa 540
   tttcactaca accgctacct gacacggcgc cggagggtgg agatcgccca cgcgctctgc 600
   ctctccgagc gccagatcaa gatctggttc cagaaccggc gcatgaagtg gaaaaagagc 660
   cacaagttgc ccaacaccaa gatccgctcg ggtggtgcgg caggctcagc cggagggccc 720
   cctggccggc ccaatggagg cccccgcgcg ctctag                           756
<210> 23
   <211> 1407
   <212> DNA
   <213> Homo sapiens
<400> 23
   atgctggccg tcggctgcgc gctgctggct gccctgctgg ccgcgccggg agcggcgctg 60
   gccccaaggc gctgccctgc gcaggaggtg gcgagaggcg tgctgaccag tctgccagga 120
   gacagcgtga ctctgacctg cccgggggta gagccggaag acaatgccac tgttcactgg 180
   gtgctcagga agccggctgc aggctcccac cccagcagat gggctggcat gggaaggagg 240
   ctgctgctga ggtcggtgca gctccacgac tctggaaact attcatgcta ccgggccggc 300
   cgcccagctg ggactgtgca cttgctggtg gatgttcccc ccgaggagcc ccagctctcc 360
   tgcttccgga agagccccct cagcaatgtt gtttgtgagt ggggtcctcg gagcacccca 420
   tccctgacga caaaggctgt gctcttggtg aggaagtttc agaacagtcc ggccgaagac 480
   ttccaggagc cgtgccagta ttcccaggag tcccagaagt tctcctgcca gttagcagtc 540
   ccggagggag acagctcttt ctacatagtg tccatgtgcg tcgccagtag tgtcgggagc 600
   aagttcagca aaactcaaac ctttcagggt tgtggaatct tgcagcctga tccgcctgcc 660
   aacatcacag tcactgccgt ggccagaaac ccccgctggc tcagtgtcac ctggcaagac 720
   ccccactcct ggaactcatc tttctacaga ctacggtttg agctcagata tcgggctgaa 780
   cggtcaaaga cattcacaac atggatggtc aaggacctcc agcatcactg tgtcatccac 840
   gacgcctgga gcggcctgag gcacgtggtg cagcttcgtg cccaggagga gttcgggcaa 900
   ggcgagtgga gcgagtggag cccggaggcc atgggcacgc cttggacaga atccaggagt 960
   cctccagctg agaacgaggt gtccaccccc atgcaggcac ttactactaa taaagacgat 1020
   gataatattc tcttcagaga ttctgcaaat gcgacaagcc tcccagtgca agattcttct 1080
   tcagtaccac tgcccacatt cctggttgct ggagggagcc tggccttcgg aacgctcctc 1140
   tgcattgcca ttgttctgag gttcaagaag acgtggaagc tgcgggctct gaaggaaggc 1200
   aagacaagca tgcatccgcc gtactctttg gggcagctgg tcccggagag gcctcgaccc 1260
   accccagtgc ttgttcctct catctcccca ccggtgtccc ccagcagcct ggggtctgac 1320
   aatacctcga gccacaaccg accagatgcc agggacccac ggagccctta tgacatcagc 1380
   aatacagact acttcttccc cagatag 1407
<210> 24
   <211> 600
   <212> DNA
   <213> Rattus norvegicus
<400> 24
   atgaactgtg tttgtcgcct ggtcctggtg gtgctgagcc tctggccaga tagagtcgtt 60
   gcccctgggc caccagctgg ctcccctcga gtgtcttcag accctcgtgc agatctggat 120
   agcgctgtcc tcttgaccag gtccctcctg gcagacacac ggcaactagc tgcacagatg 180
   agagacaaat tcccagctga tggagaccac aatctggact ccctacctac cttggccatg 240
   agcgctggga cactgggatc tttgcagctt cctggagtgc tgacaaggct tcgagtagac 300
   ttaatgtcct acttccgaca tgtacagtgg ttgcgccggg cagctggtcc ttccctaaag 360
   actctggagc cagagctggg tgccctgcaa gcccgactgg aacggctact tcgtcgctta 420
   cagctcttga tgtctcgcct agccttgccc caggcagccc cggaccaacc tgcggtccct 480
   ctgggccctc ctgcctcggc ctggggaagc atccgggcag ctcatgccat cctaggaggg 540
   ctgcacctga ccttggactg ggccgtgcgg ggcctgctgt tgttaaagac tcggctgtaa 600
<210> 25
   <211> 609
   <212> DNA
   <213> Homo sapiens
<400> 25
   atgaaggtct tggcggcagg agttgtgccc ctgctgttgg ttctgcactg gaaacatggg 60
   gcggggagcc ccctccccat cacccctgtc aacgccacct gtgccatacg ccacccatgt 120
   cacaacaacc tcatgaacca gatcaggagc caactggcac agctcaatgg cagtgccaat 180
   gccctcttta ttctctatta cacagcccag ggggagccgt tccccaacaa cctggacaag 240
   ctatgtggcc ccaacgtgac ggacttcccg cccttccacg ccaacggcac ggagaaggcc 300
   aagctggtgg agctgtaccg catagtcgtg taccttggca cctccctggg caacatcacc 360
   cgggaccaga agatcctcaa ccccagtgcc ctcagcctcc acagcaagct caacgccacc 420
   gccgacatcc tgcgaggcct ccttagcaac gtgctgtgcc gcctgtgcag caagtaccac 480
   gtgggccatg tggacgtgac ctacggccct gacacctcgg gtaaggatgt cttccagaag 540
   aagaagctgg gctgtcaact cctggggaag tataagcaga tcatcgccgt gttggcccag 600
   gccttctag                                                         609
<210> 26
   <211> 3294
   <212> DNA
   <213> Homo sapiens
<400> 26
   atgatggata tttacgtatg tttgaaacga ccatcctgga tggtggacaa taaaagaatg 60
   aggactgctt caaatttcca gtggctgtta tcaacattta ttcttctata tctaatgaat 120
   caagtaaata gccagaaaaa gggggctcct catgatttga agtgtgtaac taacaatttg 180
   caagtgtgga actgttcttg gaaagcaccc tctggaacag gccgtggtac tgattatgaa 240
   gtttgcattg aaaacaggtc ccgttcttgt tatcagttgg agaaaaccag tattaaaatt 300
   ccagctcttt cacatggtga ttatgaaata acaataaatt ctctacatga ttttggaagt 360
   tctacaagta aattcacact aaatgaacaa aacgtttcct taattccaga tactccagag 420
   atcttgaatt tgtctgctga tttctcaacc tctacattat acctaaagtg gaacgacagg 480
   ggttcagttt ttccacaccg ctcaaatgtt atctgggaaa ttaaagttct acgtaaagag 540
   agtatggagc tcgtaaaatt agtgacccac aacacaactc tgaatggcaa agatacactt 600
   catcactgga gttgggcctc agatatgccc ttggaatgtg ccattcattt tgtggaaatt 660
   agatgctaca ttgacaatct tcatttttct ggtctcgaag agtggagtga ctggagccct 720
   gtgaagaaca tttcttggat acctgattct cagactaagg tttttcctca agataaagtg 780
   atacttgtag gctcagacat aacattttgt tgtgtgagtc aagaaaaagt gttatcagca 840
   ctgattggcc atacaaactg ccccttgatc catcttgatg gggaaaatgt tgcaatcaag 900
   attcgtaata tttctgtttc tgcaagtagt ggaacaaatg tagtttttac aaccgaagat 960
   aacatatttg gaaccgttat ttttgctgga tatccaccag atactcctca acaactgaat 1020
   tgtgagacac atgatttaaa agaaattata tgtagttgga atccaggaag ggtgacagcg 1080
   ttggtgggcc cacgtgctac aagctacact ttagttgaaa gtttttcagg aaaatatgtt 1140
   agacttaaaa gagctgaagc acctacaaac gaaagctatc aattattatt tcaaatgctt 1200
   ccaaatcaag aaatatataa ttttactttg aatgctcaca atccgctggg tcgatcacaa 1260
   tcaacaattt tagttaatat aactgaaaaa gtttatcccc atactcctac ttcattcaaa 1320
   gtgaaggata ttaattcaac agctgttaaa ctttcttggc atttaccagg caactttgca 1380
   aagattaatt ttttatgtga aattgaaatt aagaaatcta attcagtaca agagcagcgg 1440
   aatgtcacaa tcaaaggagt agaaaattca agttatcttg ttgctctgga caagttaaat 1500
   ccatacactc tatatacttt tcggattcgt tgttctactg aaactttctg gaaatggagc 1560
   aaatggagca ataaaaaaca acatttaaca acagaagcca gtccttcaaa ggggcctgat 1620
   acttggagag agtggagttc tgatggaaaa aatttaataa tctattggaa gcctttaccc 1680
   attaatgaag ctaatggaaa aatactttcc tacaatgtat cgtgttcatc agatgaggaa 1740
   acacagtccc tttctgaaat ccctgatcct cagcacaaag cagagatacg acttgataag 1800
   aatgactaca tcatcagcgt agtggctaaa aattctgtgg gctcatcacc accttccaaa 1860
   atagcgagta tggaaattcc aaatgatgat ctcaaaatag aacaagttgt tgggatggga 1920
   aaggggattc tcctcacctg gcattacgac cccaacatga cttgcgacta cgtcattaag 1980
   tggtgtaact cgtctcggtc ggaaccatgc cttatggact ggagaaaagt tccctcaaac 2040
   agcactgaaa ctgtaataga atctgatgag tttcgaccag gtataagata taattttttc 2100
   ctgtatggat gcagaaatca aggatatcaa ttattacgct ccatgattgg atatatagaa 2160
   gaattggctc ccattgttgc accaaatttt actgttgagg atacttctgc agattcgata 2220
   ttagtaaaat gggaagacat tcctgtggaa gaacttagag gctttttaag aggatatttg 2280
   ttttactttg gaaaaggaga aagagacaca tctaagatga gggttttaga atcaggtcgt 2340
   tctgacataa aagttaagaa tattactgac atatcccaga agacactgag aattgctgat 2400
   cttcaaggta aaacaagtta ccacctggtc ttgcgagcct atacagatgg tggagtgggc 2460
   ccggagaaga gtatgtatgt ggtgacaaag gaaaattctg tgggattaat tattgccatt 2520
   ctcatcccag tggcagtggc tgtcattgtt ggagtggtga caagtatcct ttgctatcgg 2580
   aaacgagaat ggattaaaga aaccttctac cctgatattc caaatccaga aaactgtaaa 2640
   gcattacagt ttcaaaagag tgtctgtgag ggaagcagtg ctcttaaaac attggaaatg 2700
   aatccttgta ccccaaataa tgttgaggtt ctggaaactc gatcagcatt tcctaaaata 2760
   gaagatacag aaataatttc cccagtagct gagcgtcctg aagatcgctc tgatgcagag 2820
   cctgaaaacc atgtggttgt gtcctattgt ccacccatca ttgaggaaga aataccaaac 2880
   ccagccgcag atgaagctgg agggactgca caggttattt acattgatgt tcagtcgatg 2940
   tatcagcctc aagcaaaacc agaagaagaa caagaaaatg accctgtagg aggggcaggc 3000
   tataagccac agatgcacct ccccattaat tctactgtgg aagatatagc tgcagaagag 3060
   gacttagata aaactgcggg ttacagacct caggccaatg taaatacatg gaatttagtg 3120
   tctccagact ctcctagatc catagacagc aacagtgaga ttgtctcatt tggaagtcca 3180
   tgctccatta attcccgaca atttttgatt cctcctaaag atgaagactc tcctaaatct 3240
   aatggaggag ggtggtcctt tacaaacttt tttcagaaca aaccaaacga ttaa       3294
<210> 27
   <211> 2310
   <212> DNA
   <213> Homo sapiens
<400> 27
   atggcccaat ggaatcagct acagcagctt gacacacggt acctggagca gctccatcag 60
   ctctacagtg acagcttccc aatggagctg cggcagtttc tggccccttg gattgagagt 120
   caagattggg catatgcggc cagcaaagaa tcacatgcca ctttggtgtt tcataatctc 180
   ctgggagaga ttgaccagca gtatagccgc ttcctgcaag agtcgaatgt tctctatcag 240
   cacaatctac gaagaatcaa gcagtttctt cagagcaggt atcttgagaa gccaatggag 300
   attgcccgga ttgtggcccg gtgcctgtgg gaagaatcac gccttctaca gactgcagcc 360
   actgcggccc agcaaggggg ccaggccaac caccccacag cagccgtggt gacggagaag 420
   cagcagatgc tggagcagca ccttcaggat gtccggaaga gagtgcagga tctagaacag 480
   aaaatgaaag tggtagagaa tctccaggat gactttgatt tcaactataa aaccctcaag 540
   agtcaaggag acatgcaaga tctgaatgga aacaaccagt cagtgaccag gcagaagatg 600
   cagcagctgg aacagatgct cactgcgctg gaccagatgc ggagaagcat cgtgagtgag 660
   ctggcggggc ttttgtcagc gatggagtac gtgcagaaaa ctctcacgga cgaggagctg 720
   gctgactgga agaggcggca acagattgcc tgcattggag gcccgcccaa catctgccta 780
   gatcggctag aaaactggat aacgtcatta gcagaatctc aacttcagac ccgtcaacaa 840
   attaagaaac tggaggagtt gcagcaaaaa gtttcctaca aaggggaccc cattgtacag 900
   caccggccga tgctggagga gagaatcgtg gagctgttta gaaacttaat gaaaagtgcc 960
   tttgtggtgg agcggcagcc ctgcatgccc atgcatcctg accggcccct cgtcatcaag 1020
   accggcgtcc agttcactac taaagtcagg ttgctggtca aattccctga gttgaattat 1080
   cagcttaaaa ttaaagtgtg cattgacaaa gactctgggg acgttgcagc tctcagagga 1140
   tcccggaaat ttaacattct gggcacaaac acaaaagtga tgaacatgga agaatccaac 1200
   aacggcagcc tctctgcaga attcaaacac ttgaccctga gggagcagag atgtgggaat 1260
   gggggccgag ccaattgtga tgcttccctg attgtgactg aggagctgca cctgatcacc 1320
   tttgagaccg aggtgtatca ccaaggcctc aagattgacc tagagaccca ctccttgcca 1380
   gttgtggtga tctccaacat ctgtcagatg ccaaatgcct gggcgtccat cctgtggtac 1440
   aacatgctga ccaacaatcc caagaatgta aactttttta ccaagccccc aattggaacc 1500
   tgggatcaag tggccgaggt cctgagctgg cagttctcct ccaccaccaa gcgaggactg 1560
   agcatcgagc agctgactac actggcagag aaactcttgg gacctggtgt gaattattca 1620
   gggtgtcaga tcacatgggc taaattttgc aaagaaaaca tggctggcaa gggcttctcc 1680
   ttctgggtct ggctggacaa tatcattgac cttgtgaaaa agtacatcct ggccctttgg 1740
   aacgaagggt acatcatggg ctttatcagt aaggagcggg agcgggccat cttgagcact 1800
   aagcctccag gcaccttcct gctaagattc agtgaaagca gcaaagaagg aggcgtcact 1860
   ttcacttggg tggagaagga catcagcggt aagacccaga tccagtccgt ggaaccatac 1920
   acaaagcagc agctgaacaa catgtcattt gctgaaatca tcatgggcta taagatcatg 1980
   gatgctacca atatcctggt gtctccactg gtctatctct atcctgacat tcccaaggag 2040
   gaggcattcg gaaagtattg tcggccagag agccaggagc atcctgaagc tgacccaggc 2100
   gctgccccat acctgaagac caagtttatc tgtgtgacac caacgacctg cagcaatacc 2160
   attgacctgc cgatgtcccc ccgcacttta gattcattga tgcagtttgg aaataatggt 2220
   gaaggtgctg aaccctcagc aggagggcag tttgagtccc tcacctttga catggagttg 2280
   acctcggagt gcgctacctc ccccatgtga                                  2310
<210> 28
   <211> 1956
   <212> DNA
   <213> Homo sapiens
<400> 28
   atgaacaaat tacggcaaag ttttaggaga aagaaggatg tttatgttcc agaggccagt 60
   cgtccacatc agtggcagac agatgaagaa ggcgttcgca ccggaaaatg tagcttcccg 120
   gttaagtacc ttggccatgt agaagttgat gaatcaagag gaatgcacat ctgtgaagat 180
   gctgtaaaaa gattgaaagc tgaaaggaag ttcttcaaag gcttctttgg aaaaactgga 240
   aagaaagcag ttaaagcagt tctgtgggtc tcagcagatg gactcagagt tgtggatgaa 300
   aaaactaagg acctcatagt tgaccagacg atagagaaag tttctttctg tgccccagac 360
   aggaactttg atagagcctt ttcttacata tgccgtgatg gcaccactcg tcgctggatc 420
   tgtcactgct tcatggctgt caaggacaca ggtgaaaggt tgagccatgc agtaggctgt 480
   gcttttgcag cctgtttaga gcgcaagcag aagcgggaga aggaatgtgg agtgactgct 540
   acttttgatg ctagtcggac cacttttaca agagaaggat cattccgtgt cacaacagcc 600
   actgaacaag cagaaagaga ggagatcatg aaacaaatgc aagatgccaa gaaagctgaa 660
   acagataaga tagtcgttgg ttcatcagtt gcccctggca acactgcccc atccccatcc 720
   tctcccacct ctcctacttc tgatgccacg acctctctgg agatgaacaa tcctcatgcc 780
   atcccacgcc ggcatgctcc aattgaacag cttgctcgcc aaggctcttt ccgaggtttt 840
   cctgctctta gccagaagat gtcacccttt aaacgccaac tatccctacg catcaatgag 900
   ttgccttcca ctatgcagag gaagactgat ttccccatta aaaatgcagt gccagaagta 960
   gaaggggagg cagagagcat cagctccctg tgctcacaga tcaccaatgc cttcagcaca 1020
   cctgaggacc ccttctcatc tgctccgatg accaaaccag tgacagtggt ggcaccacaa 1080
   tctcctacct tccaagctaa tggcactgac tcagccttcc atgtgcttgc taagccagcc 1140
   catactgctc tagcacccgt agcaatgcct gtgcgtgaaa ccaacccttg ggcccatgcc 1200
   cctgatgctg ctaacaagga aattgcagcc acatgttcgg ggaccgagtg gggtcaatct 1260
   tctggtgctg cctctccagg tctcttccag gccggtcata gacgtactcc ctctgaggcc 1320
   gaccgatggt tagaagaggt gtctaagagc gtccgggctc agcagcccca ggcctcagct 1380
   gctcctctgc agccagttct ccagcctcct ccacccactg ccatctccca gccagcatca 1440
   cctttccaag ggaatgcatt cctcacctct cagcctgtgc cagtgggtgt ggtcccagcc 1500
   ctgcaaccag cctttgtccc tgcccagtcc tatcctgtgg ccaatggaat gccctatcca 1560
   gcccctaatg tgcctgtggt gggcatcact ccctcccaga tggtggccaa cgtatttggc 1620
   actgcaggcc accctcaggc tgcccatccc catcagtcac ccagcctggt caggcagcag 1680
   acattccctc actacgaggc aagcagtgct accaccagtc ccttctttaa gcctcctgct 1740
   cagcacctca acggttctgc agctttcaat ggtgtagatg atggcaggtt ggcctcagca 1800
   gacaggcata cagaggttcc tacaggcacc tgcccagtgg atccttttga agcccagtgg 1860
   gctgcattag aaaataagtc caagcagcgt actaatccct cccctaccaa ccctttctcc 1920
   agtgacttac agaagacgtt tgaaattgaa ctttaa 1956
<210> 29
   <211> 1812
   <212> DNA
   <213> Homo sapiens
<400> 29
   atgaacaaat tacggcaaag ttttaggaga aagaaggatg tttatgttcc agaggccagt 60
   cgtccacatc agtggcagac agatgaagaa ggcgttcgca ccggaaaatg tagcttcccg 120
   gttaagtacc ttggccatgt agaagttgat gaatcaagag gaatgcacat ctgtgaagat 180
   gctgtaaaaa gattgaaagc tgaaaggaag ttcttcaaag gcttctttgg aaaaactgga 240
   aagaaagcag ttaaagcagt tctgtgggtc tcagcagatg gactcagagt tgtggatgaa 300
   aaaactaagg acctcatagt tgaccagacg atagagaaag tttctttctg tgccccagac 360
   aggaactttg atagagcctt ttcttacata tgccgtgatg gcaccactcg tcgctggatc 420
   tgtcactgct tcatggctgt caaggacaca ggtgaaaggt tgagccatgc agtaggctgt 480
   gcttttgcag cctgtttaga gcgcaagcag aagcgggaga aggaatgtgg agtgactgct 540
   acttttgatg ctagtcggac cacttttaca agagaaggat cattccgtgt cacaacagcc 600
   actgaacaag cagaaagaga ggagatcatg aaacaaatgc aagatgccaa gaaagctgaa 660
   acagataaga tagtcgttgg ttcatcagtt gcccctggca acactgcccc atccccatcc 720
   tctcccacct ctcctacttc tgatgccacg acctctctgg agatgaacaa tcctcatgcc 780
   atcccacgcc ggcatgctcc aattgaacag cttgctcgcc aaggctcttt ccgaggtttt 840
   cctgctctta gccagaagat gtcacccttt aaacgccaac tatccctacg catcaatgag 900
   ttgccttcca ctatgcagag gaagactgat ttccccatta aaaatgcagt gccagaagta 960
   gaaggggagg cagagagcat cagctccctg tgctcacaga tcaccaatgc cttcagcaca 1020
   cctgaggacc ccttctcatc tgctccgatg accaaaccag tgacagtggt ggcaccacaa 1080
   tctcctacct tccaagggac cgagtggggt caatcttctg gtgctgcctc tccaggtctc 1140
   ttccaggccg gtcatagacg tactccctct gaggccgacc gatggttaga agaggtgtct 1200
   aagagcgtcc gggctcagca gccccaggcc tcagctgctc ctctgcagcc agttctccag 1260
   cctcctccac ccactgccat ctcccagcca gcatcacctt tccaagggaa tgcattcctc 1320
   acctctcagc ctgtgccagt gggtgtggtc ccagccctgc aaccagcctt tgtccctgcc 1380
   cagtcctatc ctgtggccaa tggaatgccc tatccagccc ctaatgtgcc tgtggtgggc 1440
   atcactccct cccagatggt ggccaacgta tttggcactg caggccaccc tcaggctgcc 1500
   catccccatc agtcacccag cctggtcagg cagcagacat tccctcacta cgaggcaagc 1560
   agtgctacca ccagtccctt ctttaagcct cctgctcagc acctcaacgg ttctgcagct 1620
   ttcaatggtg tagatgatgg caggttggcc tcagcagaca ggcatacaga ggttcctaca 1680
   ggcacctgcc cagtggatcc ttttgaagcc cagtgggctg cattagaaaa taagtccaag 1740
   cagcgtacta atccctcccc taccaaccct ttctccagtg acttacagaa gacgtttgaa 1800
   attgaacttt aa                                                     1812
<210> 30
   <211> 1923
   <212> DNA
   <213> Homo sapiens
<400> 30
   atgaacaaat tacggcaaag ttttaggaga aagaaggatg tttatgttcc agaggccagt 60
   cgtccacatc agtggcagac agatgaagaa ggcgttcgca ccggaaaatg tagcttcccg 120
   gttaagtacc ttggccatgt agaagttgat gaatcaagag gaatgcacat ctgtgaagat 180
   gctgtaaaaa gattgaaagc tactggaaag aaagcagtta aagcagttct gtgggtctca 240
   gcagatggac tcagagttgt ggatgaaaaa actaaggacc tcatagttga ccagacgata 300
   gagaaagttt ctttctgtgc cccagacagg aactttgata gagccttttc ttacatatgc 360
   cgtgatggca ccactcgtcg ctggatctgt cactgcttca tggctgtcaa ggacacaggt 420
   gaaaggttga gccatgcagt aggctgtgct tttgcagcct gtttagagcg caagcagaag 480
   cgggagaagg aatgtggagt gactgctact tttgatgcta gtcggaccac ttttacaaga 540
   gaaggatcat tccgtgtcac aacagccact gaacaagcag aaagagagga gatcatgaaa 600
   caaatgcaag atgccaagaa agctgaaaca gataagatag tcgttggttc atcagttgcc 660
   cctggcaaca ctgccccatc cccatcctct cccacctctc ctacttctga tgccacgacc 720
   tctctggaga tgaacaatcc tcatgccatc ccacgccggc atgctccaat tgaacagctt 780
   gctcgccaag gctctttccg aggttttcct gctcttagcc agaagatgtc accctttaaa 840
   cgccaactat ccctacgcat caatgagttg ccttccacta tgcagaggaa gactgatttc 900
   cccattaaaa atgcagtgcc agaagtagaa ggggaggcag agagcatcag ctccctgtgc 960
   tcacagatca ccaatgcctt cagcacacct gaggacccct tctcatctgc tccgatgacc 1020
   aaaccagtga cagtggtggc accacaatct cctaccttcc aagctaatgg cactgactca 1080
   gccttccatg tgcttgctaa gccagcccat actgctctag cacccgtagc aatgcctgtg 1140
   cgtgaaacca acccttgggc ccatgcccct gatgctgcta acaaggaaat tgcagccaca 1200
   tgttcgggga ccgagtgggg tcaatcttct ggtgctgcct ctccaggtct cttccaggcc 1260
   ggtcatagac gtactccctc tgaggccgac cgatggttag aagaggtgtc taagagcgtc 1320
   cgggctcagc agccccaggc ctcagctgct cctctgcagc cagttctcca gcctcctcca 1380
   cccactgcca tctcccagcc agcatcacct ttccaaggga atgcattcct cacctctcag 1440
   cctgtgccag tgggtgtggt cccagccctg caaccagcct ttgtccctgc ccagtcctat 1500
   cctgtggcca atggaatgcc ctatccagcc cctaatgtgc ctgtggtggg catcactccc 1560
   tcccagatgg tggccaacgt atttggcact gcaggccacc ctcaggctgc ccatccccat 1620
   cagtcaccca gcctggtcag gcagcagaca ttccctcact acgaggcaag cagtgctacc 1680
   accagtccct tctttaagcc tcctgctcag cacctcaacg gttctgcagc tttcaatggt 1740
   gtagatgatg gcaggttggc ctcagcagac aggcatacag aggttcctac aggcacctgc 1800
   ccagtggatc cttttgaagc ccagtgggct gcattagaaa ataagtccaa gcagcgtact 1860
   aatccctccc ctaccaaccc tttctccagt gacttacaga agacgtttga aattgaactt 1920
   taa                                                               1923
<210> 31
   <211> 1779
   <212> DNA
   <213> Homo sapiens
<400> 31
   atgaacaaat tacggcaaag ttttaggaga aagaaggatg tttatgttcc agaggccagt 60
   cgtccacatc agtggcagac agatgaagaa ggcgttcgca ccggaaaatg tagcttcccg 120
   gttaagtacc ttggccatgt agaagttgat gaatcaagag gaatgcacat ctgtgaagat 180
   gctgtaaaaa gattgaaagc tactggaaag aaagcagtta aagcagttct gtgggtctca 240
   gcagatggac tcagagttgt ggatgaaaaa actaaggacc tcatagttga ccagacgata 300
   gagaaagttt ctttctgtgc cccagacagg aactttgata gagccttttc ttacatatgc 360
   cgtgatggca ccactcgtcg ctggatctgt cactgcttca tggctgtcaa ggacacaggt 420
   gaaaggttga gccatgcagt aggctgtgct tttgcagcct gtttagagcg caagcagaag 480
   cgggagaagg aatgtggagt gactgctact tttgatgcta gtcggaccac ttttacaaga 540
   gaaggatcat tccgtgtcac aacagccact gaacaagcag aaagagagga gatcatgaaa 600
   caaatgcaag atgccaagaa agctgaaaca gataagatag tcgttggttc atcagttgcc 660
   cctggcaaca ctgccccatc cccatcctct cccacctctc ctacttctga tgccacgacc 720
   tctctggaga tgaacaatcc tcatgccatc ccacgccggc atgctccaat tgaacagctt 780
   gctcgccaag gctctttccg aggttttcct gctcttagcc agaagatgtc accctttaaa 840
   cgccaactat ccctacgcat caatgagttg ccttccacta tgcagaggaa gactgatttc 900
   cccattaaaa atgcagtgcc agaagtagaa ggggaggcag agagcatcag ctccctgtgc 960
   tcacagatca ccaatgcctt cagcacacct gaggacccct tctcatctgc tccgatgacc 1020
   aaaccagtga cagtggtggc accacaatct cctaccttcc aagggaccga gtggggtcaa 1080
   tcttctggtg ctgcctctcc aggtctcttc caggccggtc atagacgtac tccctctgag 1140
   gccgaccgat ggttagaaga ggtgtctaag agcgtccggg ctcagcagcc ccaggcctca 1200
   gctgctcctc tgcagccagt tctccagcct cctccaccca ctgccatctc ccagccagca 1260
   tcacctttcc aagggaatgc attcctcacc tctcagcctg tgccagtggg tgtggtccca 1320
   gccctgcaac cagcctttgt ccctgcccag tcctatcctg tggccaatgg aatgccctat 1380
   ccagccccta atgtgcctgt ggtgggcatc actccctccc agatggtggc caacgtattt 1440
   ggcactgcag gccaccctca ggctgcccat ccccatcagt cacccagcct ggtcaggcag 1500
   cagacattcc ctcactacga ggcaagcagt gctaccacca gtcccttctt taagcctcct 1560
   gctcagcacc tcaacggttc tgcagctttc aatggtgtag atgatggcag gttggcctca 1620
   gcagacaggc atacagaggt tcctacaggc acctgcccag tggatccttt tgaagcccag 1680
   tgggctgcat tagaaaataa gtccaagcag cgtactaatc cctcccctac caaccctttc 1740
   tccagtgact tacagaagac gtttgaaatt gaactttaa                        1779
<210> 32
   <211> 1830
   <212> DNA
   <213> Mus musculus
<400> 32
   atgtcccgca gcgcggcggc cagcggcgga ccccggaggc ctgagcggca cctgccccca 60
   gccccctgtg gggccccggg gcccccagaa acctgcagga cggagccaga cggggcgggc 120
   accatgaaca agttacggca gagcctgcgg cggaggaagc cagcctacgt gcccgaggcg 180
   tcgcgcccgc accagtggca ggcagacgag gacgcggtgc ggaagggcac gtgcagcttc 240
   ccggtcaggt acctgggtca cgtggaggta gaggagtccc ggggaatgca cgtgtgtgaa 300
   gatgcggtga agaagctgaa ggcgatgggc cgaaagtccg tgaagtctgt cctgtgggtg 360
   tcagccgatg ggctccgagt ggtggacgac aaaaccaagg atcttctggt cgaccagacc 420
   atcgaaaagg tctccttttg tgctcctgac cgcaacctgg acaaggcttt ctcctatatc 480
   tgtcgtgacg ggactacccg ccgctggatc tgccactgtt ttctggcact gaaggactcc 540
   ggcgagaggc tgagccacgc tgtgggctgt gcttttgccg cctgcctgga gcgaaaacag 600
   cgacgggaga aggaatgtgg ggtcacggcc gccttcgatg ccagccgcac cagcttcgcc 660
   cgcgagggct ccttccgcct gtctgggggt gggcggcctg ctgagcgaga ggccccggac 720
   aagaagaaag cagaggcagc agctgccccc actgtggctc ctggccctgc ccagcctggg 780
   cacgtgtccc cgacaccagc caccacatcc cctggtgaga agggtgaggc aggcacccct 840
   gtggctgcag gcaccactgc ggccgccatc ccccggcgcc atgcacccct ggagcagctg 900
   gttcgccagg gctccttccg tgggttccca gcactcagcc agaagaactc gcctttcaaa 960
   cggcagctga gcctacggct gaatgagctg ccatccacgc tgcagcgccg cactgacttc 1020
   caggtgaagg gcacagtgcc tgagatggag cctcctggtg ccggcgacag tgacagcatc 1080
   aacgctctgt gcacacagat cagttcatct tttgccagtg ctggagcgcc agcaccaggg 1140
   ccaccacctg ccacaacagg gacttctgcc tggggtgagc cctccgtgcc ccctgcagct 1200
   gccttccagc ctgggcacaa gcggacacct tcagaggctg agcgatggct ggaggaggtg 1260
   tcacaggtgg ccaaggccca gcagcagcag cagcagcaac agcaacagca gcagcagcag 1320
   cagcagcaac agcagcaagc agcctcagtg gccccagtgc ccaccatgcc tcctgccctg 1380
   cagcctttcc ccgcccccgt ggggcccttt gacgctgcac ctgcccaagt ggccgtgttc 1440
   ctgccacccc cacacatgca gccccctttt gtgcccgcct acccgggctt gggctaccca 1500
   ccgatgcccc gggtgcccgt ggtgggcatc acaccctcac agatggtggc aaacgccttc 1560
   tgctcagccg cccagctcca gcctcagcct gccactctgc ttgggaaagc tggggccttc 1620
   ccgccccctg ccatacccag tgcccctggg agccaggccc gccctcgccc caatggggcc 1680
   ccctggcccc ctgagccagc gcctgcccca gctccagagt tggacccctt tgaggcccag 1740
   tgggcggcat tagaaggcaa agccactgta gagaaaccct ccaacccctt ttctggcgac 1800
   ctgcaaaaga cattcgagat tgaactgtag 1830
<210> 33
   <211> 918
   <212> DNA
   <213> Homo sapiens
<400> 33
   atgagtgtgg atccagcttg tccccaaagc ttgccttgct ttgaagcatc cgactgtaaa 60
   gaatcttcac ctatgcctgt gatttgtggg cctgaagaaa actatccatc cttgcaaatg 120
   tcttctgctg agatgcctca cacggagact gtctctcctc ttccttcctc catggatctg 180
   cttattcagg acagccctga ttcttccacc agtcccaaag gcaaacaacc cacttctgca 240
   gagaagagtg tcgcaaaaaa ggaagacaag gtcccggtca agaaacagaa gaccagaact 300
   gtgttctctt ccacccagct gtgtgtactc aatgatagat ttcagagaca gaaatacctc 360
   agcctccagc agatgcaaga actctccaac atcctgaacc tcagctacaa acaggtgaag 420
   acctggttcc agaaccagag aatgaaatct aagaggtggc agaaaaacaa ctggccgaag 480
   aatagcaatg gtgtgacgca gaaggcctca gcacctacct accccagcct ttactcttcc 540
   taccaccagg gatgcctggt gaacccgact gggaaccttc caatgtggag caaccagacc 600
   tggaacaatt caacctggag caaccagacc cagaacatcc agtcctggag caaccactcc 660
   tggaacactc agacctggtg cacccaatcc tggaacaatc aggcctggaa cagtcccttc 720
   tataactgtg gagaggaatc tctgcagtcc tgcatgcagt tccagccaaa ttctcctgcc 780
   agtgacttgg aggctgcctt ggaagctgct ggggaaggcc ttaatgtaat acagcagacc 840
   actaggtatt ttagtactcc acaaaccatg gatttattcc taaactactc catgaacatg 900
   caacctgaag acgtgtga 918
<210> 34
   <211> 759
   <212> DNA
   <213> Homo sapiens
<400> 34
   atgggggtac tgctcacaca gaggacgctg ctcagtctgg tccttgcact cctgtttcca 60
   agcatggcga gcatggcggc tataggcagc tgctcgaaag agtaccgcgt gctccttggc 120
   cagctccaga agcagacaga tctcatgcag gacaccagca gactcctgga cccctatata 180
   cgtatccaag gcctggatgt tcctaaactg agagagcact gcagggagcg ccccggggcc 240
   ttccccagtg aggagaccct gagggggctg ggcaggcggg gcttcctgca gaccctcaat 300
   gccacactgg gctgcgtcct gcacagactg gccgacttag agcagcgcct ccccaaggcc 360
   caggatttgg agaggtctgg gctgaacatc gaggacttgg agaagctgca gatggcgagg 420
   ccgaacatcc tcgggctcag gaacaacatc tactgcatgg cccagctgct ggacaactca 480
   gacacggctg agcccacgaa ggctggccgg ggggcctctc agccgcccac ccccacccct 540
   gcctcggatg cttttcagcg caagctggag ggctgcaggt tcctgcatgg ctaccatcgc 600
   ttcatgcact cagtggggcg ggtcttcagc aagtgggggg agagcccgaa ccggagccgg 660
   agacacagcc cccaccaggc cctgaggaag ggggtgcgca ggaccagacc ctccaggaaa 720
   ggcaagagac tcatgaccag gggacagctg ccccggtag                        759
<210> 35
   <211> 2940
   <212> DNA
   <213> Homo sapiens
<400> 35
   atggctctat ttgcagtctt tcagacaaca ttcttcttaa cattgctgtc cttgaggact 60
   taccagagtg aagtcttggc tgaacgttta ccattgactc ctgtatcact taaagtttcc 120
   accaattcta cgcgtcagag tttgcactta caatggactg tccacaacct tccttatcat 180
   caggaattga aaatggtatt tcagatccag atcagtagga ttgaaacatc caatgtcatc 240
   tgggtgggga attacagcac cactgtgaag tggaaccagg ttctgcattg gagctgggaa 300
   tctgagctcc ctttggaatg tgccacacac tttgtaagaa taaagagttt ggtggacgat 360
   gccaagttcc ctgagccaaa tttctggagc aactggagtt cctgggagga agtcagtgta 420
   caagattcta ctggacagga tatattgttc gttttcccta aagataagct ggtggaagaa 480
   ggcaccaatg ttaccatttg ttacgtttct aggaacattc aaaataatgt atcctgttat 540
   ttggaaggga aacagattca tggagaacaa cttgatccac atgtaactgc attcaacttg 600
   aatagtgtgc ctttcattag gaataaaggg acaaatatct attgtgaggc aagtcaagga 660
   aatgtcagtg aaggcatgaa aggcatcgtt ctttttgtct caaaagtact tgaggagccc 720
   aaggactttt cttgtgaaac cgaggacttc aagactttgc actgtacttg ggatcctggg 780
   acggacactg ccttggggtg gtctaaacaa ccttcccaaa gctacacttt atttgaatca 840
   ttttctgggg aaaagaaact ttgtacacac aaaaactggt gtaattggca aataactcaa 900
   gactcacaag aaacctataa cttcacactc atagctgaaa attacttaag gaagagaagt 960
   gtcaatatcc tttttaacct gactcatcga gtttatttaa tgaatccttt tagtgtcaac 1020
   tttgaaaatg taaatgccac aaatgccatc atgacctgga aggtgcactc cataaggaat 1080
   aatttcacat atttgtgtca gattgaactc catggtgaag gaaaaatgat gcaatacaat 1140
   gtttccatca aggtgaacgg tgagtacttc ttaagtgaac tggaacctgc cacagagtac 1200
   atggcgcgag tacggtgtgc tgatgccagc cacttctgga aatggagtga atggagtggt 1260
   cagaacttca ccacacttga agctgctccc tcagaggccc ctgatgtctg gagaattgtg 1320
   agcttggagc caggaaatca tactgtgacc ttattctgga agccattatc aaaactgcat 1380
   gccaatggaa agatcctgtt ctataatgta gttgtagaaa acctagacaa accatccagt 1440
   tcagagctcc attccattcc agcaccagcc aacagcacaa aactaatcct tgacaggtgt 1500
   tcctaccaaa tctgcgtcat agccaacaac agtgtgggtg cttctcctgc ttctgtaata 1560
   gtcatctctg cagaccccga aaacaaagag gttgaggaag aaagaattgc aggcacagag 1620
   ggtggattct ctctgtcttg gaaaccccaa cctggagatg ttataggcta tgttgtggac 1680
   tggtgtgacc atacccagga tgtgctcggt gatttccagt ggaagaatgt aggtcccaat 1740
   accacaagca cagtcattag cacagatgct tttaggccag gagttcgata tgacttcaga 1800
   atttatgggt tatctacaaa aaggattgct tgtttattag agaaaaaaac aggatactct 1860
   caggaacttg ctccttcaga caaccctcac gtgctggtgg atacattgac atcccactcc 1920
   ttcactctga gttggaaaga ttactctact gaatctcaac ctggttttat acaagggtac 1980
   catgtctatc tgaaatccaa ggcgaggcag tgccacccac gatttgaaaa ggcagttctt 2040
   tcagatggtt cagaatgttg caaatacaaa attgacaacc cggaagaaaa ggcattgatt 2100
   gtggacaacc taaagccaga atccttctat gagtttttca tcactccatt cactagtgct 2160
   ggtgaaggcc ccagtgctac gttcacgaag gtcacgactc cggatgaaca ctcctcgatg 2220
   ctgattcata tcctactgcc catggttttc tgcgtcttgc tcatcatggt catgtgctac 2280
   ttgaaaagtc agtggatcaa ggagacctgt tatcctgaca tccctgaccc ttacaagagc 2340
   agcatcctgt cattaataaa attcaaggag aaccctcacc taataataat gaatgtcagt 2400
   gactgtatcc cagatgctat tgaagttgta agcaagccag aagggacaaa gatacagttc 2460
   ctaggcacta ggaagtcact cacagaaacc gagttgacta agcctaacta cctttatctc 2520
   cttccaacag aaaagaatca ctctggccct ggcccctgca tctgttttga gaacttgacc 2580
   tataaccagg cagcttctga ctctggctct tgtggccatg ttccagtatc cccaaaagcc 2640
   ccaagtatgc tgggactaat gacctcacct gaaaatgtac taaaggcact agaaaaaaac 2700
   tacatgaact ccctgggaga aatcccagct ggagaaacaa gtttgaatta tgtgtcccag 2760
   ttggcttcac ccatgtttgg agacaaggac agtctcccaa caaacccagt agaggcacca 2820
   cactgttcag agtataaaat gcaaatggca gtctccctgc gtcttgcctt gcctcccccg 2880
   accgagaata gcagcctctc ctcaattacc cttttagatc caggtgaaca ctactgctaa 2940
<210> 36
   <211> 798
   <212> DNA
   <213> Homo sapiens
<400> 36
   atgcacttct acagactatt ccttggggcc acacgtaggt tcttgaatcc cgaatggaaa 60
   ggggagattg ataactggtg tgtttatgtt cttacaagtc ttctgccttt taaaatccag 120
   tcccaggaca tcaaagctct gcagaaagaa ctcgagcaat ttgccaagct cctgaagcag 180
   aagaggatca ccctgggata tacacaggcc gatgtggggc tcaccctggg ggttctattt 240
   gggaaggtat tcagccaaac gaccatctgc cgctttgagg ctctgcagct tagcttcaag 300
   aacatgtgta agctgcggcc cttgctgcag aagtgggtgg aggaagctga caacaatgaa 360
   aatcttcagg agatatgcaa agcagaaacc ctcgtgcagg cccgaaagag aaagcgaacc 420
   agtatcgaga accgagtgag aggcaacctg gagaatttgt tcctgcagtg cccgaaaccc 480
   acactgcagc agatcagcca catcgcccag cagcttgggc tcgagaagga tgtggtccga 540
   gtgtggttct gtaaccggcg ccagaagggc aagcgatcaa gcagcgacta tgcacaacga 600
   gaggattttg aggctgctgg gtctcctttc tcagggggac cagtgtcctt tcctctggcc 660
   ccagggcccc attttggtac cccaggctat gggagccctc acttcactgc actgtactcc 720
   tcggtccctt tccctgaggg ggaagccttt ccccctgtct ccgtcaccac tctgggctct 780
   cccatgcatt caaactga                                               798
<210> 37
   <211> 1083
   <212> DNA
   <213> Homo sapiens
<400> 37
   atggcgggac acctggcttc ggatttcgcc ttctcgcccc ctccaggtgg tggaggtgat 60
   gggccagggg ggccggagcc gggctgggtt gatcctcgga cctggctaag cttccaaggc 120
   cctcctggag ggccaggaat cgggccgggg gttgggccag gctctgaggt gtgggggatt 180
   cccccatgcc ccccgccgta tgagttctgt ggggggatgg cgtactgtgg gccccaggtt 240
   ggagtggggc tagtgcccca aggcggcttg gagacctctc agcctgaggg cgaagcagga 300
   gtcggggtgg agagcaactc cgatggggcc tccccggagc cctgcaccgt cacccctggt 360
   gccgtgaagc tggagaagga gaagctggag caaaacccgg aggagtccca ggacatcaaa 420
   gctctgcaga aagaactcga gcaatttgcc aagctcctga agcagaagag gatcaccctg 480
   ggatatacac aggccgatgt ggggctcacc ctgggggttc tatttgggaa ggtattcagc 540
   caaacgacca tctgccgctt tgaggctctg cagcttagct tcaagaacat gtgtaagctg 600
   cggcccttgc tgcagaagtg ggtggaggaa gctgacaaca atgaaaatct tcaggagata 660
   tgcaaagcag aaaccctcgt gcaggcccga aagagaaagc gaaccagtat cgagaaccga 720
   gtgagaggca acctggagaa tttgttcctg cagtgcccga aacccacact gcagcagatc 780
   agccacatcg cccagcagct tgggctcgag aaggatgtgg tccgagtgtg gttctgtaac 840
   cggcgccaga agggcaagcg atcaagcagc gactatgcac aacgagagga ttttgaggct 900
   gctgggtctc ctttctcagg gggaccagtg tcctttcctc tggccccagg gccccatttt 960
   ggtaccccag gctatgggag ccctcacttc actgcactgt actcctcggt ccctttccct 1020
   gagggggaag cctttccccc tgtctccgtc accactctgg gctctcccat gcattcaaac 1080
   tga                                                               1083
<210> 38
   <211> 953
   <212> DNA
   <213> Homo sapiens
<400> 38
   tgtacaacat gatggagacg gagctgaagc cgccgggccc gcagcaaact tcggggggcg 60
   gcggcggcaa ctccaccgcg gcggcggccg gcggcaacca gaaaaacagc ccggaccgcg 120
   tcaagcggcc catgaatgcc ttcatggtgt ggtcccgcgg gcagcggcgc aagatggccc 180
   aggagaaccc caagatgcac aactcggaga tcagcaagcg cctgggcgcc gagtggaaac 240
   ttttgtcgga gacggagaag cggccgttca tcgacgaggc taagcggctg cgagcgctgc 300
   acatgaagga gcacccggat tataaatacc ggccccggcg gaaaaccaag acgctcatga 360
   agaaggataa gtacacgctg cccggcgggc tgctggcccc cggcggcaat agcatggcga 420
   gcggggtcgg ggtgggcgcc ggcctgggcg cgggcgtgaa ccagcgcatg gacagttacg 480
   cgcacatgaa cggctggagc aacggcagct acagcatgat gcaggaccag ctgggctacc 540
   cgcagcaccc gggcctcaat gcgcacggcg cagcgcagat gcagcccatg caccgctacg 600
   acgtgagcgc cctgcagtac aactccatga ccagctcgca gacctacatg aacggctcgc 660
   ccacctacag catgtcctac tcgcagcagg gcacccctgg catggctctt ggctccatgg 720
   gttcggtggt caagtccgag gccagctcca gcccccctgt ggttacctct tcctcccact 780
   ccagggcgcc ctgccaggcc ggggacctcc gggacatgat cagcatgtat ctccccggcg 840
   ccgaggtgcc ggaacccgcc gcccccagca gacttcacat gtcccagcac taccagagcg 900
   gcccggtgcc cggcacggcc attaacggca cactgcccct ctcacacatg tga       953
<210> 39
   <211> 621
   <212> DNA
   <213> Homo sapiens
<400> 39
   atgtcggggc ccgggacggc cgcggtagcg ctgctcccgg cggtcctgct ggccttgctg 60
   gcgccctggg cgggccgagg gggcgccgcc gcacccactg cacccaacgg cacgctggag 120
   gccgagctgg agcgccgctg ggagagcctg gtggcgctct cgttggcgcg cctgccggtg 180
   gcagcgcagc ccaaggaggc ggccgtccag agcggcgccg gcgactacct gctgggcatc 240
   aagcggctgc ggcggctcta ctgcaacgtg ggcatcggct tccacctcca ggcgctcccc 300
   gacggccgca tcggcggcgc gcacgcggac acccgcgaca gcctgctgga gctctcgccc 360
   gtggagcggg gcgtggtgag catcttcggc gtggccagcc ggttcttcgt ggccatgagc 420
   agcaagggca agctctatgg ctcgcccttc ttcaccgatg agtgcacgtt caaggagatt 480
   ctccttccca acaactacaa cgcctacgag tcctacaagt accccggcat gttcatcgcc 540
   ctgagcaaga atgggaagac caagaagggg aaccgagtgt cgcccaccat gaaggtcacc 600
   cacttcctcc ccaggctgtg a                                          621
<210> 40
   <211> 1443
   <212> DNA
   <213> Homo sapiens
<400> 40
   atggaggtgg cgccggagca gccgcgctgg atggcgcacc cggccgtgct gaatgcgcag 60
   caccccgact cacaccaccc gggcctggcg cacaactaca tggaacccgc gcagctgctg 120
   cctccagacg aggtggacgt cttcttcaat cacctcgact cgcagggcaa cccctactat 180
   gccaaccccg ctcacgcgcg ggcgcgcgtc tcctacagcc ccgcgcacgc ccgcctgacc 240
   ggaggccaga tgtgccgccc acacttgttg cacagcccgg gtttgccctg gctggacggg 300
   ggcaaagcag ccctctctgc cgctgcggcc caccaccaca acccctggac cgtgagcccc 360
   ttctccaaga cgccactgca cccctcagct gctggaggcc ctggaggccc actctctgtg 420
   tacccagggg ctgggggtgg gagcggggga ggcagcggga gctcagtggc ctccctcacc 480
   cctacagcag cccactctgg ctcccacctt ttcggcttcc cacccacgcc acccaaagaa 540
   gtgtctcctg accctagcac cacgggggct gcgtctccag cctcatcttc cgcggggggt 600
   agtgcagccc gaggagagga caaggacggc gtcaagtacc aggtgtcact gacggagagc 660
   atgaagatgg aaagtggcag tcccctgcgc ccaggcctag ctactatggg cacccagcct 720
   gctacacacc accccatccc cacctacccc tcctatgtgc cggcggctgc ccacgactac 780
   agcagcggac tcttccaccc cggaggcttc ctggggggac cggcctccag cttcacccct 840
   aagcagcgca gcaaggctcg ttcctgttca gaaggccggg agtgtgtcaa ctgtggggcc 900
   acagccaccc ctctctggcg gcgggacggc accggccact acctgtgcaa tgcctgtggc 960
   ctctaccaca agatgaatgg gcagaaccga ccactcatca agcccaagcg aagactgtcg 1020
   gccgccagaa gagccggcac ctgttgtgca aattgtcaga cgacaaccac caccttatgg 1080
   cgccgaaacg ccaacgggga ccctgtctgc aacgcctgtg gcctctacta caagctgcac 1140
   aatgttaaca ggccactgac catgaagaag gaagggatcc agactcggaa ccggaagatg 1200
   tccaacaagt ccaagaagag caagaaaggg gcggagtgct tcgaggagct gtcaaagtgc 1260
   atgcaggaga agtcatcccc cttcagtgca gctgccctgg ctggacacat ggcacctgtg 1320
   ggccacctcc cgcccttcag ccactccgga cacatcctgc ccactccgac gcccatccac 1380
   ccctcctcca gcctctcctt cggccacccc cacccgtcca gcatggtgac cgccatgggc 1440
   tag 1443
<210> 41
   <211> 1335
   <212> DNA
   <213> Homo sapiens
<400> 41
   atggaggtga cggcggacca gccgcgctgg gtgagccacc accaccccgc cgtgctcaac 60
   gggcagcacc cggacacgca ccacccgggc ctcagccact cctacatgga cgcggcgcag 120
   tacccgctgc cggaggaggt ggatgtgctt tttaacatcg acggtcaagg caaccacgtc 180
   ccgccctact acggaaactc ggtcagggcc acggtgcaga ggtaccctcc gacccaccac 240
   gggagccagg tgtgccgccc gcctctgctt catggatccc taccctggct ggacggcggc 300
   aaagccctgg gcagccacca caccgcctcc ccctggaatc tcagcccctt ctccaagacg 360
   tccatccacc acggctcccc ggggcccctc tccgtctacc ccccggcctc gtcctcctcc 420
   ttgtcggggg gccacgccag cccgcacctc ttcaccttcc cgcccacccc gccgaaggac 480
   gtctccccgg acccatcgct gtccacccca ggctcggccg gctcggcccg gcaggacgag 540
   aaagagtgcc tcaagtacca ggtgcccctg cccgacagca tgaagctgga gtcgtcccac 600
   tcccgtggca gcatgaccgc cctgggtgga gcctcctcgt cgacccacca ccccatcacc 660
   acctacccgc cctacgtgcc cgagtacagc tccggactct tcccccccag cagcctgctg 720
   ggcggctccc ccaccggctt cggatgcaag tccaggccca aggcccggtc cagcacagaa 780
   ggcagggagt gtgtgaactg tggggcaacc tcgaccccac tgtggcggcg agatggcacg 840
   ggacactacc tgtgcaacgc ctgcgggctc tatcacaaaa tgaacggaca gaaccggccc 900
   ctcattaagc ccaagcgaag gctgtctgca gccaggagag cagggacgtc ctgtgcgaac 960
   tgtcagacca ccacaaccac actctggagg aggaatgcca atggggaccc tgtctgcaat 1020
   gcctgtgggc tctactacaa gcttcacaat attaacagac ccctgactat gaagaaggaa 1080
   ggcatccaga ccagaaaccg aaaaatgtct agcaaatcca aaaagtgcaa aaaagtgcat 1140
   gactcactgg aggacttccc caagaacagc tcgtttaacc cggccgccct ctccagacac 1200
   atgtcctccc tgagccacat ctcgcccttc agccactcca gccacatgct gaccacgccc 1260
   acgccgatgc acccgccatc cagcctgtcc tttggaccac accacccctc cagcatggtc 1320
   accgccatgg gttag 1335
<210> 42
   <211> 1329
   <212> DNA
   <213> Homo sapiens
<400> 42
   atgtatcaga gcttggccat ggccgccaac cacgggccgc cccccggtgc ctacgaggcg 60
   ggcggccccg gcgccttcat gcacggcgcg ggcgccgcgt cctcgccagt ctacgtgccc 120
   acaccgcggg tgccctcctc cgtgctgggc ctgtcctacc tccagggcgg aggcgcgggc 180
   tctgcgtccg gaggcgcctc gggcggcagc tccggtgggg ccgcgtctgg tgcggggccc 240
   gggacccagc agggcagccc gggatggagc caggcgggag ccgacggagc cgcttacacc 300
   ccgccgccgg tgtcgccgcg cttctccttc ccggggacca ccgggtccct ggcggccgcc 360
   gccgccgctg ccgcggcccg ggaagctgcg gcctacagca gtggcggcgg agcggcgggt 420
   gcgggcctgg cgggccgcga gcagtacggg cgcgccggct tcgcgggctc ctactccagc 480
   ccctacccgg cttacatggc cgacgtgggc gcgtcctggg ccgcagccgc cgccgcctcc 540
   gccggcccct tcgacagccc ggtcctgcac agcctgcccg gccgggccaa cccggccgcc 600
   cgacacccca atctcgatat gtttgacgac ttctcagaag gcagagagtg tgtcaactgt 660
   ggggctatgt ccaccccgct ctggaggcga gatgggacgg gtcactatct gtgcaacgcc 720
   tgcggcctct accacaagat gaacggcatc aaccggccgc tcatcaagcc tcagcgccgg 780
   ctgtccgcct cccgccgagt gggcctctcc tgtgccaact gccagaccac caccaccacg 840
   ctgtggcgcc gcaatgcgga gggcgagcct gtgtgcaatg cctgcggcct ctacatgaag 900
   ctccacgggg tccccaggcc tcttgcaatg cggaaagagg ggatccaaac cagaaaacgg 960
   aagcccaaga acctgaataa atctaagaca ccagcagctc cttcaggcag tgagagcctt 1020
   cctcccgcca gcggtgcttc cagcaactcc agcaacgcca ccaccagcag cagcgaggag 1080
   atgcgtccca tcaagacgga gcctggcctg tcatctcact acgggcacag cagctccgtg 1140
   tcccagacgt tctcagtcag tgcgatgtct ggccatgggc cctccatcca ccctgtcctc 1200
   tcggccctga agctctcccc acaaggctat gcgtctcccg tcagccagtc tccacagacc 1260
   agctccaagc aggactcttg gaacagcctg gtcttggccg acagtcacgg ggacataatc 1320
   actgcgtaa 1329
<210> 43
   <211> 1194
   <212> DNA
   <213> Homo sapiens
<400> 43
   atgtaccaga gcctggcgct ggccgcgagc ccccgccagg ccgcctacgc cgactcgggc 60
   tccttcctgc acgctccggg cgccggctct ccgatgtttg tgccgccggc gcgcgtcccc 120
   tcgatgctgt cctacctgtc cgggtgtgag ccgagcccgc agccccccga gctcgctgcg 180
   cgccccggct gggcgcagac agccaccgcg gattcgtcgg ccttcggccc gggcagtccg 240
   caccccccag ccgcgcaccc gcccggggcc accgccttcc ctttcgcgca cagcccctcg 300
   gggcccggca gcggcggcag cgcggggggc cgagacggca gtgcctacca gggcgcgctg 360
   ttgcctcgag aacagttcgc ggccccgctt gggcggccgg tggggacctc gtactccgcc 420
   acctacccgg cctacgtgag ccccgacgtg gcccagtcct ggactgccgg gcccttcgat 480
   ggcagcgtcc tgcacggcct cccaggccgc aggcccacct tcgtgtccga cttcttggag 540
   gagttcccgg gtgagggtcg tgagtgtgtc aactgcgggg ccctgtccac accgctgtgg 600
   cgccgagatg gcaccggcca ctacctgtgc aatgcctgcg gcctctacca caagatgaat 660
   ggcgtcaacc ggccgctcgt tcggcctcag aagcgcctgt cctcgtcccg ccgcgccggc 720
   ctctgctgca ccaactgcca cacgaccaac accacgctgt ggcggcggaa ctcggagggg 780
   gagcccgtgt gcaatgcctg cggcctctac atgaagctgc acggggtgcc gcggcctctg 840
   gctatgaaga aagaaagcat ccagacacgg aagcggaagc caaagaccat cgccaaggcc 900
   aggggctcct caggatccac aaggaatgcc tcggcctccc catctgctgt cgccagcact 960
   gacagctcag cagccacttc caaagccaag cccagcctgg cgtccccagt gtgccctggg 1020
   cccagcatgg ccccccaggc ctctggccag gaggatgact ctcttgcccc cggccacttg 1080
   gagttcaagt tcgagcctga ggactttgcc ttcccctcca cggccccgag cccccaggct 1140
   ggcctcaggg gggctctgcg ccaagaggcc tggtgtgcgc tggccttggc ctag 1194
<210> 44
   <211> 1788
   <212> DNA
   <213> Homo sapiens
<400> 44
   atggccttga ctgacggcgg ctggtgcttg ccgaagcgct tcggggccgc gggtgcggac 60
   gccagcgact ccagagcctt tccagcgcgg gagccctcca cgccgccttc ccccatctct 120
   tcctcgtcct cctcctgctc ccggggcgga gagcggggcc ccggcggcgc cagcaactgc 180
   gggacgcctc agctcgacac ggaggcggcg gccggacccc cggcccgctc gctgctgctc 240
   agttcctacg cttcgcatcc cttcggggct ccccacggac cttcggcgcc tggggtcgcg 300
   ggccccgggg gcaacctgtc gagctgggag gacttgctgc tgttcactga cctcgaccaa 360
   gccgcgaccg ccagcaagct gctgtggtcc agccgcggcg ccaagctgag ccccttcgca 420
   cccgagcagc cggaggagat gtaccagacc ctcgccgctc tctccagcca gggtccggcc 480
   gcctacgacg gcgcgcccgg cggcttcgtg cactctgcgg ccgcggcggc agcagccgcg 540
   gcggcggcca gctccccggt ctacgtgccc accacccgcg tgggttccat gctgcccggc 600
   ctaccgtacc acctgcaggg gtcgggcagt gggccagcca accacgcggg cggcgcgggc 660
   gcgcaccccg gctggcctca ggcctcggcc gacagccctc catacggcag cggaggcggc 720
   gcggctggcg gcggggccgc ggggcctggc ggcgctggct cagccgcggc gcacgtctcg 780
   gcgcgcttcc cctactctcc cagcccgccc atggccaacg gcgccgcgcg ggagccggga 840
   ggctacgcgg cggcgggcag tgggggcgcg ggaggcgtga gcggcggcgg cagtagcctg 900
   gcggccatgg gcggccgcga gccccagtac agctcgctgt cggccgcgcg gccgctgaac 960
   gggacgtacc accaccacca ccaccaccac caccaccatc cgagccccta ctcgccctac 1020
   gtgggggcgc cactgacgcc tgcctggccc gccggaccct tcgagacccc ggtgctgcac 1080
   agcctgcaga gccgcgccgg agccccgctc ccggtgcccc ggggtcccag tgcagacctg 1140
   ctggaggacc tgtccgagag ccgcgagtgc gtgaactgcg gctccatcca gacgccgctg 1200
   tggcggcggg acggcaccgg ccactacctg tgcaacgcct gcgggctcta cagcaagatg 1260
   aacggcctca gccggcccct catcaagccg cagaagcgcg tgccttcatc acggcggctt 1320
   ggattgtcct gtgccaactg tcacaccaca actaccacct tatggcgcag aaacgccgag 1380
   ggtgaacccg tgtgcaatgc ttgtggactc tacatgaaac tccatggggt gcccagacca 1440
   cttgctatga aaaaagaggg aattcaaacc aggaaacgaa aacctaagaa cataaataaa 1500
   tcaaagactt gctctggtaa tagcaataat tccattccca tgactccaac ttccacctct 1560
   tctaactcag atgattgcag caaaaatact tcccccacaa cacaacctac agcctcaggg 1620
   gcgggtgccc cggtgatgac tggtgcggga gagagcacca atcccgagaa cagcgagctc 1680
   aagtattcgg gtcaagatgg gctctacata ggcgtcagtc tcgcctcgcc ggccgaagtc 1740
   acgtcctccg tgcgaccgga ttcctggtgc gccctggccc tggcctga              1788
<210> 45
   <211> 1674
   <212> DNA
   <213> Homo sapiens
<400> 45
   atggtgtcca agctcacgtc gctccagcaa gaactcctga gcgccctgct gagctccggg 60
   gtcaccaagg aggtgctggt tcaggccttg gaggagttgc tgccatcccc gaacttcggg 120
   gtgaagctgg agacgctgcc cctgtcccct ggcagcgggg ccgagcccga caccaagccg 180
   gtcttccata ctctcaccaa cggccacgcc aagggccgct tgtccggcga cgagggctcc 240
   gaggacggcg acgactatga cacacctccc atcctcaagg agctgcaggc gctcaacacc 300
   gaggaggcgg cggagcagcg ggcggaggtg gaccggatgc tcagtgagga cccttggagg 360
   gctgctaaaa tgatcaaggg ttacatgcag caacacaaca tcccccagag ggaggtggtc 420
   gatgtcaccg gcctgaacca gtcgcacctc tcccagcatc tcaacaaggg cacccctatg 480
   aagacccaga agcgtgccgc tctgtacacc tggtacgtca gaaagcaacg agagatcctc 540
   cgacaattca accagacagt ccagagttct ggaaatatga cagacaaaag cagtcaggat 600
   cagctgctgt ttctctttcc agagttcagt caacagagcc atgggcctgg gcagtccgat 660
   gatgcctgct ctgagcccac caacaagaag atgcgccgca accggttcaa atgggggccc 720
   gcgtcccagc aaatcttgta ccaggcctac gatcggcaaa agaaccccag caaggaagag 780
   agagaggcct tagtggagga atgcaacagg gcagaatgtt tgcagcgagg ggtgtccccc 840
   tccaaagccc acggcctggg ctccaacttg gtcactgagg tccgtgtcta caactggttt 900
   gcaaaccgca ggaaggagga ggcattccgg caaaagctgg ccatggacgc ctatagctcc 960
   aaccagactc acagcctgaa ccctctgctc tcccacggct ccccccacca ccagcccagc 1020
   tcctctcctc caaacaagct gtcaggagtg cgctacagcc agcagggaaa caatgagatc 1080
   acttcctcct caacaatcag tcaccatggc aacagcgcca tggtgaccag ccagtcggtt 1140
   ttacagcaag tctccccagc cagcctggac ccaggccaca atctcctctc acctgatggt 1200
   aaaatgatct cagtctcagg aggaggtttg cccccagtca gcaccttgac gaatatccac 1260
   agcctctccc accataatcc ccagcaatct caaaacctca tcatgacacc cctctctgga 1320
   gtcatggcaa ttgcacaaag cctcaacacc tcccaagcac agagtgtccc tgtcatcaac 1380
   agtgtggccg gcagcctggc agccctgcag cccgtccagt tctcccagca gctgcacagc 1440
   cctcaccagc agcccctcat gcagcagagc ccaggcagcc acatggccca gcagcccttc 1500
   atggcagctg tgactcagct gcagaactca cacatgtacg cacacaagca ggaacccccc 1560
   cagtattccc acacctcccg gtttccatct gcaatggtgg tcacagatac cagcagcatc 1620
   agtacactca ccaacatgtc ttcaagtaaa cagtgtcctc tacaagcctg gtga 1674
<210> 46
   <211> 1887
   <212> DNA
   <213> Rattus norvegicus
<400> 46
   atggtttcta agttgagcca gctgcagacg gagctcctgg ctgctctgct cgagtcgggc 60
   ctgagcaaag aggctctgat ccaggctctg ggggagcccg ggccctacct gatggttgga 120
   gatggtcccc tggacaaggg ggagtcctgc ggtgggactc gaggggacct gaccgagctg 180
   cccaatggcc tgggggagac gcgtggctcg gaagatgaca cggatgacga tggggaagac 240
   ttcgcgccac ccattctgaa agagctggag aacctcagcc cagaggaggc agcccaccag 300
   aaagccgtgg tggagtcact tcttcaggag gacccatggc gcgtggcaaa gatggtcaag 360
   tcgtacctgc agcaacacaa catcccccag cgggaggtgg tggacactac gggtctcaac 420
   cagtcccacc tgtcccagca cctcaacaag ggcaccccca tgaagacgca gaagcgggcc 480
   gcgctgtaca cctggtacgt ccgcaagcag cgagaggtgg ctcagcaatt cacccacgcg 540
   gggcagggcg gactgattga agagcccaca ggtgatgagc tgccaaccaa aaaggggcgg 600
   aggaaccggt tcaagtgggg ccccgcatcc cagcagatcc tgttccaggc ttacgagagg 660
   cagaagaacc ccagcaagga agagcgagag accttggtgg aggagtgcaa tagggcggag 720
   tgcatccaga gaggggtgtc accatcgcag gcccaggggc taggctccaa ccttgtcacc 780
   gaggtgcgtg tctacaactg gtttgccaac cggcgcaagg aagaagcctt tcggcataag 840
   ctggccatgg acacgtataa cgggcctcca cccgggccag gccccggccc tgcgctacct 900
   gcccacagtt ccccgggcct gcccacaacc accctctctc ccagtaaggt ccacggtgtg 960
   cggtatggac agtctgcaac cagcgaggca gctgaggtgc cctccagcag cggaggtccc 1020
   ttagtcacag tgtctgcggc cttacaccaa gtgtccccca caggcttgga gcccagcagc 1080
   ctgctgagca ccgaggccaa gctggtctca gccacggggg gtcccctgcc tcccgtcagc 1140
   accctgacag cactgcacag cttggagcag acgtctccag gtctcaacca gcagccgcag 1200
   aaccttatca tggcctcgct gcctggggtc atgaccatcg gcccagggga gcccgcctcc 1260
   ctgggtccca cgttcactaa cacgggtgcc tctaccctgg tcattggtct ggcctccaca 1320
   caggcacaga gcgtgccagt catcaacagc atggggagca gcctgaccac cctgcagccg 1380
   gtccagtttt cccagccact gcacccttcc tatcagcagc ctctcatgcc ccctgtacag 1440
   agccacgtgg cccagagtcc cttcatggca accatggccc agctgcagag cccccacgcc 1500
   ctgtacagcc acaagcctga ggtggcccag tacacgcata caagcctgct tccgcagacc 1560
   atgctgatca cagacaccaa cctcagcacc cttgccagcc tcacgcccac caagcaggtc 1620
   ttcacctcag acacagaggc ctccagtgag cctgggcttc atgagccgtc gtctccagcc 1680
   acaaccattc acatccccag ccaggacccg tcaaacatcc agcacctgca gcctgctcac 1740
   cggctcagca ccagtcccac agtgtcctcc agcagcctgg tgttgtacca gagttctgac 1800
   tccaacgggc acagccacct gctgccatcc aaccacggtg tcatcgagac ttttatctcc 1860
   acccagatgg cctcctcctc ccagtaa                                     1887
<210> 47
   <211> 1407
   <212> DNA
   <213> Mus musculus
<400> 47
   atgttaggga ctgtgaagat ggaagggcat gagagcaacg actggaacag ctactacgcg 60
   gacacgcagg aggcctactc ctctgtccct gtcagcaaca tgaactccgg cctgggctct 120
   atgaactcca tgaacaccta catgaccatg aacaccatga ccacgagcgg caacatgacc 180
   ccggcttcct tcaacatgtc ctacgccaac acgggcttag gggccggcct gagtcccggt 240
   gctgtggctg gcatgccagg ggcctctgca ggcgccatga acagcatgac tgcggcgggc 300
   gtcacggcca tgggtacggc gctgagcccg ggaggcatgg gctccatggg cgcgcagccc 360
   gtcacctcca tgaacggcct gggtccctac gccgccgcca tgaacccgtg catgagtccc 420
   atggcgtacg cgccgtccaa cctgggccgc agccgcgcgg ggggcggcgg cgacgccaag 480
   acattcaagc gcagctaccc tcacgccaag ccgccttact cctacatctc gctcatcacg 540
   atggccatcc agcaggcgcc cagcaagatg ctcacgctga gcgagatcta ccagtggatc 600
   atggacctct tcccctatta ccgccagaac cagcagcgct ggcagaactc catccgccac 660
   tcgctgtcct tcaacgattg tttcgtcaag gtggcacgat ccccagacaa gccaggcaag 720
   ggctcctact ggacgctgca cccggactcc ggcaacatgt tcgagaacgg ctgctacttg 780
   cgccgccaaa agcgcttcaa gtgtgagaag cagccggggg ccggaggtgg gagtgggggc 840
   ggcggctcca aagggggccc agaaagtcgc aaggacccct caggcccggg gaaccccagc 900
   gccgagtcac cccttcattg gggtgtgcac ggaaaggcta gccagctaga gggcgcgccg 960
   gcccccgggc ccgccgccag cccccagact ctggaccaca gcggggccac ggcgacaggg 1020
   ggcgcttcgg agttgaagtc tccagcgtct tcatctgcgc cccccataag ctccgggcca 1080
   ggggcgctgg catctgtacc cccctctcac ccggctcacg gcctggcacc ccacgaatct 1140
   cagctgcatc tgaaagggga tccccactac tcctttaatc accccttctc catcaacaac 1200
   ctcatgtcct cctccgagca acagcacaag ctggacttca aggcatacga gcaggcgctg 1260
   cagtactctc cttatggcgc taccttgccc gccagtctgc cccttggcag cgcctcagtg 1320
   gccacgagga gccccatcga gccctcagcc ctggagccag cctactacca aggtgtgtat 1380
   tccagacccg tgctaaatac ttcctag                                      1407
<210> 48
   <211> 1062
   <212> DNA
   <213> Mus musculus
<400> 48
   atgctgggct cagtgaagat ggaggctcat gacctggccg agtggagcta ctacccggag 60
   gcgggcgagg tgtattctcc agtgaatcct gtgcccacca tggcccctct caactcctac 120
   atgaccttga acccactcag ctctccctac cctcccggag ggcttcaggc ctccccactg 180
   cctacaggac ccctggcacc cccagccccc actgcgccct tggggcccac cttcccaagc 240
   ttgggcactg gtggcagcac cggaggcagt gcttccgggt atgtagcccc agggcccggg 300
   cttgtacatg gaaaagagat ggcaaagggg taccggcggc cactggccca cgccaaacca 360
   ccatattcct acatctctct cataaccatg gctattcagc aggctccagg caagatgctg 420
   accctgagtg aaatctacca atggatcatg gacctcttcc cgtactaccg ggagaaccag 480
   caacgttggc agaactccat ccggcattcg ctgtccttca atgactgctt cgtcaaggtg 540
   gcacgctccc cagacaagcc aggcaaaggc tcctactggg ccttgcatcc cagctctggg 600
   aacatgtttg agaacggatg ctatctccgc cggcagaagc gcttcaagct ggaggagaag 660
   gcaaagaaag gaaacagcgc cacatcggcc agcaggaatg gtactgcggg gtcagccacc 720
   tctgccacca ctacagctgc cactgcagtc acctccccgg ctcagcccca gcctacgcca 780
   tctgagcccg aggcccagag tggggatgat gtggggggtc tggactgcgc ctcacctcct 840
   tcgtccacac cttatttcag cggcctggag ctcccggggg aactaaagtt ggatgcgccc 900
   tataacttca accacccttt ctctatcaac aacctgatgt cagaacagac atcgacacct 960
   tccaaactgg atgtggggtt tgggggctac ggggctgaga gtggggagcc tggagtctac 1020
   taccagagcc tctattcccg ctctctgctt aatgcatcct ag                     1062
<210> 49
   <211> 1380
   <212> DNA
   <213> Mus musculus
<400> 49
   atgctgggag ccgtgaagat ggaagggctc gagccatccg actggagcag ctactacgcg 60
   gagcccgagg gctactcttc cgtgagcaac atgaacgccg gcctggggat gaatggcatg 120
   aacacataca tgagcatgtc cgcggctgcc atgggcggcg gttccggcaa catgagcgcg 180
   ggctccatga acatgtcatc ctatgtgggc gctggaatga gcccgtcgct agctggcatg 240
   tccccgggcg ccggcgccat ggcgggcatg agcggctcag ccggggcggc cggcgtggcg 300
   ggcatgggac ctcacctgag tccgagtctg agcccgctcg ggggacaggc ggccggggcc 360
   atgggtggcc ttgcccccta cgccaacatg aactcgatga gccccatgta cgggcaggcc 420
   ggcctgagcc gcgctcggga ccccaagaca taccgacgca gctacacaca cgccaaacct 480
   ccctactcgt acatctcgct catcaccatg gccatccagc agagccccaa caagatgctg 540
   acgctgagcg agatctatca gtggatcatg gacctcttcc ctttctaccg gcagaaccag 600
   cagcgctggc agaactccat ccgccactct ctctccttca acgactgctt tctcaaggtg 660
   ccccgctcgc cagacaagcc tggcaagggc tccttctgga ccctgcaccc agactcgggc 720
   aacatgttcg agaacggctg ctacctgcgc cgccagaagc gcttcaagtg tgagaagcaa 780
   ctggcactga aggaagccgc gggtgcggcc agtagcggag gcaagaagac cgctcctggg 840
   tcccaggcct ctcaggctca gctcggggag gccgcgggct cggcctccga gactccggcg 900
   ggcaccgagt ccccccattc cagcgcttct ccgtgtcagg agcacaagcg aggtggccta 960
   agcgagctaa agggagcacc tgcctctgcg ctgagtcctc ccgagccggc gccctcgcct 1020
   gggcagcagc agcaggctgc agcccacctg ctgggcccac ctcaccaccc aggcctgcca 1080
   ccagaggccc acctgaagcc cgagcaccat tacgccttca accacccctt ctctatcaac 1140
   aacctcatgt cgtccgagca gcaacatcac cacagccacc accaccatca gccccacaaa 1200
   atggacctca aggcctacga acaggtcatg cactacccag ggggctatgg ttcccccatg 1260
   ccaggcagct tggccatggg cccagtcacg aacaaagcgg gcctggatgc ctcgcccctg 1320
   gctgcagaca cttcctacta ccaaggagtg tactccaggc ctattatgaa ctcatcctaa 1380
<210> 50
   <211> 1053
   <212> DNA
   <213> Homo sapiens
<400> 50
   atgctgggct cagtgaagat ggaggcccat gacctggccg agtggagcta ctacccggag 60
   gcgggcgagg tctactcgcc ggtgacccca gtgcccacca tggcccccct caactcctac 120
   atgaccctga atcctctaag ctctccctat ccccctgggg ggctccctgc ctccccactg 180
   ccctcaggac ccctggcacc cccagcacct gcagcccccc tggggcccac tttcccaggc 240
   ctgggtgtca gcggtggcag cagcagctcc gggtacgggg ccccgggtcc tgggctggtg 300
   cacgggaagg agatgccgaa ggggtatcgg cggcccctgg cacacgccaa gccaccgtat 360
   tcctatatct cactcatcac catggccatc cagcaggcgc cgggcaagat gctgaccttg 420
   agtgaaatct accagtggat catggacctc ttcccttact accgggagaa tcagcagcgc 480
   tggcagaact ccattcgcca ctcgctgtct ttcaacgact gcttcgtcaa ggtggcgcgt 540
   tccccagaca agcctggcaa gggctcctac tgggccctac accccagctc agggaacatg 600
   tttgagaatg gctgctacct gcgccgccag aaacgcttca agctggagga gaaggtgaaa 660
   aaagggggca gcggggctgc caccaccacc aggaacggga cagggtctgc tgcctcgacc 720
   accacccccg cggccacagt cacctccccg ccccagcccc cgcctccagc ccctgagcct 780
   gaggcccagg gcggggaaga tgtgggggct ctggactgtg gctcacccgc ttcctccaca 840
   ccctatttca ctggcctgga gctcccaggg gagctgaagc tggacgcgcc ctacaacttc 900
   aaccaccctt tctccatcaa caacctaatg tcagaacaga caccagcacc tcccaaactg 960
   gacgtggggt ttgggggcta cggggctgaa ggtggggagc ctggagtcta ctaccagggc 1020
   ctctattccc gctctttgct taatgcatcc tag 1053
<210> 51
   <211> 1422
   <212> DNA
   <213> Homo sapiens
<400> 51
   atgttaggaa ctgtgaagat ggaagggcat gaaaccagcg actggaacag ctactacgca 60
   gacacgcagg aggcctactc ctcggtcccg gtcagcaaca tgaactcagg cctgggctcc 120
   atgaactcca tgaacaccta catgaccatg aacaccatga ctacgagcgg caacatgacc 180
   ccggcgtcct tcaacatgtc ctatgccaac ccggccttag gggccggcct gagtcccggc 240
   gcagtagccg gcatgccggg gggctcggcg ggcgccatga acagcatgac tgcggccggc 300
   gtgacggcca tgggtacggc gctgagcccg agcggcatgg gcgccatggg tgcgcagcag 360
   gcggcctcca tgatgaatgg cctgggcccc tacgcggccg ccatgaaccc gtgcatgagc 420
   cccatggcgt acgcgccgtc caacctgggc cgcagccgcg cgggcggcgg cggcgacgcc 480
   aagacgttca agcgcagtta cccgcacgcc aagccgccct actcgtacat ctcgctcatc 540
   accatggcca tccagcgggc gcccagcaag atgctcacgc tgagcgagat ctaccagtgg 600
   atcatggacc tcttccccta ttaccggcag aaccagcagc gctggcagaa ctccatccgc 660
   cactcgctgt ccttcaatga ctgcttcgtc aaggtggcac gctccccgga caagccgggc 720
   aagggctcct actggacgct gcacccggac tccggcaaca tgttcgagaa cggctgctac 780
   ttgcgccgcc agaagcgctt caagtgcgag aagcagccgg gggccggcgg cgggggcggg 840
   agcggaagcg gggcagcggc cgccaagggc ggccctgaga gccgcaagga cccctctggc 900
   gcctctaacc ccagcgccga ctcgcccctc catcggggtg tgcacgggaa gaccggccag 960
   ctagagggcg cgccggcccc gggcccggcc gccagccccc agactctgga ccacagtggg 1020
   gcgacggcga cagggggcgc ctcggagttg aagactccag cctcctcaac tgcgcccccc 1080
   ataagctccg ggcccggggc gctggcctct gtgcccgcct ctcacccggc acacggcttg 1140
   gcaccccacg agtcccagct gcacctgaaa ggggaccccc actactcctt caaccacccg 1200
   ttctccatca acaacctcat gtcctcctcg gagcagcagc ataagctgga cttcaaggca 1260
   tacgaacagg cactgcaata ctcgccttac ggctctacgt tgcccgccag cctgcctcta 1320
   ggcagcgcct cggtgaccac caggagcccc atcgagccct cagccctgga gccggcgtac 1380
   taccaaggtg tgtattccag acccgtccta aacacttcct ag                    1422
<210> 52
   <211> 1425
   <212> DNA
   <213> Mus musculus
<400> 52
   atgcgactct ctaaaaccct tgccggcatg gatatggccg actacagcgc tgccctggac 60
   ccagcctaca ccaccctgga gtttgaaaat gtgcaggtgt tgaccatggg caatgacacg 120
   tccccatctg aaggtgccaa cctcaattca tccaacagcc tgggcgtcag tgccctgtgc 180
   gccatctgtg gcgaccgggc caccggcaaa cactacggag cctcgagctg tgacggctgc 240
   aaggggttct tcaggaggag cgtgaggaag aaccacatgt actcctgcag gtttagccga 300
   caatgtgtgg tagacaaaga taagaggaac cagtgtcgtt actgcaggct taagaagtgc 360
   ttccgggctg gcatgaagaa ggaagctgtc caaaatgagc gggaccggat cagcacgcgg 420
   aggtcaagct acgaggacag cagcctgccc tccatcaacg cgctcctgca ggcagaggtt 480
   ctgtcccagc agatcacctc tcccatctct gggatcaatg gcgacattcg ggcaaagaag 540
   attgccaaca tcacagacgt gtgtgagtct atgaaggagc agctgctggt cctggtcgag 600
   tgggccaagt acatcccggc cttctgcgaa ctccttctgg atgaccaggt ggcgctgctc 660
   agggcccacg ccggtgagca tctgctgctt ggagccacca agaggtccat ggtgtttaag 720
   gacgtgctgc tcctaggcaa tgactacatc gtccctcggc actgtccaga gctagcggag 780
   atgagccgtg tgtccatccg catcctcgat gagctggtcc tgcccttcca agagctgcag 840
   attgatgaca atgaatatgc ctgcctcaaa gccatcatct tctttgatcc agatgccaag 900
   gggctgagtg acccgggcaa gatcaagcgg ctgcggtcac aggtgcaagt gagcctggag 960
   gattacatca acgaccggca gtacgactct cggggccgct ttggagagct gctgctgctg 1020
   ttgcccacgc tgcagagcat cacctggcag atgatcgaac agatccagtt catcaagctc 1080
   ttcggcatgg ccaagattga caacctgctg caggagatgc ttctcggagg gtctgccagt 1140
   gatgcacccc acacccacca ccccctgcac cctcacctga tgcaagaaca catgggcacc 1200
   aatgtcattg ttgctaacac gatgccctct cacctcagca atggacagat gtgtgagtgg 1260
   ccccgaccca gggggcaggc agccactccc gagactccac agccatcacc accaagtggc 1320
   tcgggatctg aatcctacaa gctcctgcca ggagccatca ccaccatcgt caagcctccc 1380
   tctgccattc cccagccaac gatcaccaag caagaagcca tctag                 1425
<210> 53
   <211> 1398
   <212> DNA
   <213> Rattus norvegicus
<400> 53
   atggacatgg ctgactacag tgctgccttg gacccagcct acaccaccct ggagtttgaa 60
   aatgtgcagg tgttgaccat gggcaatgac acatccccat ctgaaggtgc caacctcaac 120
   tcatccaaca gcctgggtgt cagtgccctg tgtgccatct gtggcgatcg ggccactggc 180
   aaacactacg gagcctcaag ctgtgacggc tgcaagggat tcttcaggag gagcgtgagg 240
   aagaaccaca tgtactcctg caggtttagc aggcagtgcg tggtagacaa agataagagg 300
   aaccagtgtc gttactgcag gctcaagaag tgcttccggg ctggcatgaa gaaagaagcc 360
   gtccaaaatg agcgggatcg gatcagcacg cggaggtcaa gctacgagga cagcagccta 420
   ccctccatta atgcgctcct gcaggcagag gtcctgtctc agcagatcac ctcccccatc 480
   tctgggatca atggcgacat tcgggccaag aagattgcca acatcacgga tgtgtgtgag 540
   tctatgaagg agcagctgct ggttctggtc gaatgggcca agtacatccc ggccttctgt 600
   gaacttcttc tggatgacca ggtggcgctg ctcagagccc acgctggtga gcacctgctg 660
   cttggagcca ccaagaggtc catggtgttc aaggatgtgc tgctcctagg caatgactac 720
   atcgtccctc ggcactgtcc agagctagca gagatgagcc gtgtgtccat tcgcatcctc 780
   gatgagctgg tcttgccctt ccaagagctg cagatcgatg ataatgaata cgcctgcctc 840
   aaagccatca tcttctttga cccagatgcc aaggggctga gtgacccagg caagatcaag 900
   cggctgcggt cacaggtgca ggtgagcctg gaggattaca tcaacgaccg gcagtatgac 960
   tctcggggtc gttttggaga gctgctgctg ctcctgccca ctctgcagag cattacctgg 1020
   cagatgatcg agcagatcca gttcatcaag ctctttggca tggccaagat tgacaacctg 1080
   ctgcaggaga tgctgcttgg agggtctgcc agtgacgcgc cccacgccca ccaccccctg 1140
   caccctcacc tgatgcaaga acacatgggc accaatgtca tagttgccaa cacgatgccc 1200
   tctcacctca gcaatggaca gatgtgtgag tggccccggc ccagggggca ggcagccacc 1260
   cctgagactc cacagccatc accaccaagt ggctctggat ctgaatccta caagctcctg 1320
   ccaggagcca tcaccaccat cgtcaagcct ccctctgcca tcccccagcc aacgatcacc 1380
   aagcaggaag ccatctag                                               1398
<210> 54
   <211> 1398
   <212> DNA
   <213> Mus musculus
<400> 54
   atgaacgcac agctgaccat ggaggcgatc ggcgagctgc acggggtgag ccatgagccg 60
   gtgcccgccc ctgctgacct gctgggcggc agccctcacg cgcgcagctc cgtgggacac 120
   cgcggcagcc acctgcctcc cgcgcacccg cgttccatgg gcatggcgtc cctgctggac 180
   ggcggcagcg gaggcagcga ttaccaccac caccaccgcg cccctgagca cagcttggct 240
   ggccccctgc accccaccat gaccatggcc tgtgaaactc ccccaggtat gagcatgccc 300
   accacctaca ctaccttaac ccctctgcag ccgctgccgc ccatctccac cgtgtccgac 360
   aagttccctc accatcatca ccaccaccat caccaccacc acccacacca ccaccagcgc 420
   ctggcgggca acgtgagcgg tagtttcaca cttatgcggg atgagcgcgg gctggcctct 480
   atgaataacc tctatacccc ctaccacaag gacgtggctg gcatgggcca gagcctctcg 540
   cccctctctg gctccggtct gggcagcatt cacaactccc agcaaggact tccccactat 600
   gctcatcccg gcgcggctat gcccaccgac aagatgctca ccccaaatgg ctttgaagcc 660
   caccaccctg ccatgctcgg tcgccacggg gagcagcacc tcacgcccac ctcggccggc 720
   atggtaccca tcaacggcct tcctccgcac catcctcatg cccacctgaa tgcccagggc 780
   cacggacagc tcctgggcac agcccgagag cccaaccctt cggtgaccgg cgcgcaggtc 840
   agcaatggaa gtaattcagg gcagatggaa gagatcaata ccaaagaggt ggcgcagcgt 900
   atcaccaccg agctcaaacg ttacagcatc ccacaggcca tcttcgcgca gagggtgctc 960
   tgccgttccc aggggaccct ttcggacctg ctgcgaaacc ccaagccctg gagcaaactc 1020
   aagtcgggtc gggagacctt ccggaggatg tggaagtggc tgcaggagcc ggagttccag 1080
   cgcatgtcgg cgctccgctt agcagcctgc aaacggaaag agcaagaaca tgggaaggac 1140
   agaggcaaca cccccaaaaa gcccaggctg gtcttcacag acgtccaacg tcgaactcta 1200
   catgcaatat tcaaggaaaa taagcgtccg tccaaagaat tacaaatcac catctcccag 1260
   cagctggggt tggagctgag cactgtcagc aacttcttca tgaatgccag aaggaggagt 1320
   ctggacaagt ggcaggacga gggcggctcc aactcaggca gttcatcgtc ctcatcgagc 1380
   acttgtacca aagcatga 1398
<210> 55
   <211> 1002
   <212> DNA
   <213> Xenopus laevis
<400> 55
   atggagaagt ccaagaattt caggattgac gctctcctgg cgatagatcc ccccaaggct 60
   cagacctccc cattggctct ggtcacctcg ctgtcctcct cgtctctctc cgggagcccc 120
   ccgtccgagc acactgacag cctcaggact gactccccct cccctccaag gacttgtgga 180
   ctggtcccta aaccaggttt cctgagcagc caccagcacc ccccaaacat gatgtcattg 240
   cacccccagg ctgctccagg gatcccccct caggccctgt atggacaccc gatgtacagc 300
   tacttggcag cggggcagca cccagctctg tcctacccct actcccagat gcagagcagc 360
   caccaccccc accccatgga ccccatcaag atcagcgctg gcaccttcca actggaccag 420
   tggctcagag cctccactgc cggcatgatg ctgcccaaaa tggcagactt taactcccag 480
   gcccaatcca acctgctggg aaagtgcaga agaccaagga cagcgtttac cagtcagcag 540
   ctgttggaac tggagcacca attcaagctg aacaagtacc tctccaggcc gaaacgcttt 600
   gaagtggcca cttccctgat gctcactgag acgcaggtga agatctggtt ccagaacagg 660
   cgcatgaaat ggaagaggag taagaaagcc aaggagcagg cggcgcagga ctcagcagag 720
   aaacagcaga gggcaggcaa gggcagcagc gaggagaagt gctcggatga gctgcaggaa 780
   gagaagaaat cctaccatct ccatcccagg ggggagccca tcaaagggaa cggccgcctg 840
   cagcccagag actatacaga cagcgaagag gacgaggagg aggacaggga agaggaggaa 900
   gaggaagatc acagagggga ggggaagcgg ttttaccatc attcttctga ctgcacatcc 960
   gaggaagagg agaacagcca caataagcag agcggccact ga 1002
<210> 56
   <211> 1215
   <212> DNA
   <213> Mus musculus
<400> 56
   atggaaaaat ccaaaaattt ccgcatcgac gccctgctgg ccgtggatcc cccgcgagcc 60
   gcctccacgc agagcgcgcc tctggccttg gtcacttccc tcgcgactac agtatctggt 120
   cccggccgcg gcggcagcgg cggcgggggg accagtagcg gggcgagccg tagctgcagt 180
   cccgcatcct cggaggccac tgcagcgccc ggtgaccggc tgagagctga gagcccgtcg 240
   cccccacgct tgctggctgc acactgcgcg ctgctgccca agcccggatt cctgggcgcc 300
   ggaggaggcg gcggcgcggc gggtgggccg ggcactcccc accaccacgc gcaccctggt 360
   gcagcagccg ccgcggctgc cgctgccgct gccgcggctg ccggtggcct ggcactgggg 420
   ctgcacccgg ggggcgcaca gggcggcgcg ggcctccctg cacaggcggc tctctatgga 480
   cacccggtct acagttattc ggcagcagct gcagcggccg cgctagctgg ccagcacccg 540
   gcgctttcct actcataccc tcaggtgcag ggcgcgcacc ctgcgcaccc tgccgacccc 600
   atcaagctgg gtgccagcac cttccaactg gaccagtggc tgcgcgcgtc tactgcgggc 660
   atgatcctgc ccaagatgcc ggacttcagc tgtcaggcgc agtcgaacct cttggggaag 720
   tgccgaaggc ctcgcacggc cttcaccagc cagcagctgt tggagctgga acaccagttc 780
   aagctcaaca agtacctgtc tcgacccaag cgttttgagg tggctacctc gctcatgctc 840
   accgagactc aggtgaagat ttggttccag aaccgccgaa tgaaatggaa acgcagcaaa 900
   aaggccaaag agcaggctgc gcaggaggcg gagaagcaga agggcggcgg cgggggcacc 960
   ggcaaaggcg gcagtgagga gaagacggaa gaggagctga tggggcctcc ggtttcgggg 1020
   gacaaggcaa gcggccgtcg cctgcgggac ttgcgggaca gtgaccctga tgaggacgag 1080
   gatgatgaag aagaggacaa cttcccgtac agcaatggtg ccggtgccca tgctgcctca 1140
   tccgactgct catctgagga cgactcgcct cctccaagac taggcgggcc tggacaccaa 1200
   cctctgcccc agtag                                                  1215
<210> 57
   <211> 1215
   <212> DNA
   <213> Homo sapiens
<400> 57
   atggaaaaat ccaaaaattt ccgcatcgac gccctgctgg ccgtggatcc cccgcgagcc 60
   gcctccacgc agagcgcgcc tctggccttg gtcacttccc tcgcgactac agtatctggt 120
   cccggccgcg gcggcagcgg cggcgggggg accagtagcg gggcgagccg tagctgcagt 180
   cccgcatcct cggaggccac tgcagcgccc ggtgaccggc tgagagctga gagcccgtcg 240
   cccccacgct tgctggctgc acactgcgcg ctgctgccca agcccggatt cctgggcgcc 300
   ggaggaggcg gcggcgcggc gggtgggccg ggcactcccc accaccacgc gcaccctggt 360
   gcagcagccg ccgcggctgc cgctgccgct gccgcggctg ccggtggcct ggcactgggg 420
   ctgcacccgg ggggcgcaca gggcggcgcg ggcctccctg cacaggcggc tctctatgga 480
   cacccggtct acagttattc ggcagcagct gcagcggccg cgctagctgg ccagcacccg 540
   gcgctttcct actcataccc tcaggtgcag ggcgcgcacc ctgcgcaccc tgccgacccc 600
   atcaagctgg gtgccagcac cttccaactg gaccagtggc tgcgcgcgtc tactgcgggc 660
   atgatcctgc ccaagatgcc ggacttcagc tgtcaggcgc agtcgaacct cttggggaag 720
   tgccgaaggc ctcgcacggc cttcaccagc cagcagctgt tggagctgga acaccagttc 780
   aagctcaaca agtacctgtc tcgacccaag cgttttgagg tggctacctc gctcatgctc 840
   accgagactc aggtgaagat ttggttccag aaccgccgaa tgaaatggaa acgcagcaaa 900
   aaggccaaag agcaggctgc gcaggaggcg gagaagcaga agggcggcgg cgggggcacc 960
   ggcaaaggcg gcagtgagga gaagacggaa gaggagctga tggggcctcc ggtttcgggg 1020
   gacaaggcaa gcggccgtcg cctgcgggac ttgcgggaca gtgaccctga tgaggacgag 1080
   gatgatgaag aagaggacaa cttcccgtac agcaatggtg ccggtgccca tgctgcctca 1140
   tccgactgct catctgagga cgactcgcct cctccaagac taggcgggcc tggacaccaa 1200
   cctctgcccc agtag                                                  1215
<210> 58
   <211> 846
   <212> DNA
   <213> Homo sapiens
<400> 58
   atgacttcca aggaggacgg caaggcggcg ccgggggagg agcggcggcg cagcccgctg 60
   gaccacctgc ctccgcctgc caactccaac aagccactga cgccgttcag catcgaggac 120
   atcctcaaca agccgtctgt gcggagaagt tactcgctgt gcggggcggc gcacctgctg 180
   gccgccgcgg acaagcacgc gcagggcggc ttgcccctgg cgggccgcgc gctgctctcg 240
   cagacctcgc cgctgtgcgc gctggaggag ctcgccagca agacgtttaa ggggctggag 300
   gtcagcgttc tgcaggcagc cgaaggccgc gacggtatga ccatctttgg gcagcggcag 360
   acccctaaga agcggcgaaa gtcgcgcacg gccttcacca accaccagat ctatgaattg 420
   gaaaagcgct ttctatacca gaagtacctg tcccccgccg atcgcgacca aatcgcgcag 480
   cagctgggcc tcaccaacgc gcaagtcatc acctggttcc agaatcggcg cgctaagctc 540
   aagcgggacc tggaggagat gaaggccgac gtagagtccg ccaagaaact gggccccagc 600
   gggcagatgg acatcgtggc gctggccgaa ctcgagcaga actcggaggc cacagccggc 660
   ggtggcggcg gctgcggcag ggccaagtcg aggcccggct ctccggtcct ccccccaggc 720
   gccccgaagg ccccgggcgc tggcgccctg cagctctcgc ctgcctctcc gctcacggac 780
   cagccggcca gcagccagga ctgctcggag gacgaggaag acgaagagat cgacgtggac 840
   gattga                                                            846
<210> 59
   <211> 1119
   <212> DNA
   <213> Mus musculus
<400> 59
   atgttggacg gcatcaagat ggaggagcac gccctgcgcc ccgggcccgc cactctgggg 60
   gtgctgctgg gctccgactg cccgcatccc gccgtctgcg agggctgcca gcggcccatc 120
   tccgaccgct tcctgatgcg agtcaacgag tcgtcctggc acgaggagtg tttgcagtgc 180
   gcggcgtgtc agcaagccct caccaccagc tgctacttcc gggatcggaa actgtactgc 240
   aaacaagact accaacagct cttcgcggcc aagtgcagcg gctgcatgga gaagatcgcc 300
   cccaccgagt tcgtgatgcg ggcgctggag tgcgtgtacc acctgggctg cttctgctgc 360
   tgcgtgtgtg aacggcagct acgcaagggc gacgaattcg tgctcaagga gggccagctg 420
   ctgtgcaagg gtgactacga gaaggagaag gacctgctca gctccgtgag ccccgacgag 480
   tccgactccg tgaagagcga ggatgaagat ggggacatga agccggccaa ggggcagggc 540
   agtcagagca agggcagcgg ggatgacggg aaggacccgc ggaggcccaa gcgaccccgg 600
   accatcctca ccacgcagca gcgaagagcc ttcaaggcct ccttcgaggt ctcgtcgaag 660
   ccttgccgaa aggtccgaga gacactggca gctgagacgg gcctcagtgt gcgcgtggtc 720
   caggtctggt ttcagaacca aagagcaaag atgaagaagc tggcgcggcg gcaccagcag 780
   cagcaggagc agcagaactc ccagcggctg ggccaggagg tcctgtccag ccgcatggag 840
   ggcatgatgg cttcctacac gccgctggcc ccaccacagc agcagatcgt ggccatggaa 900
   cagagcccct acggcagcag cgaccccttc cagcagggcc tcacgccgcc ccaaatgcca 960
   gggaacgact ccatcttcca tgacatcgac agcgatacct ccttaaccag cctcagcgac 1020
   tgcttcctcg gctcctcaga cgtgggctcc ctgcaggccc gcgtggggaa ccccatcgac 1080
   cggctctact ccatgcagag ttcctacttc gcctcctga                        1119
<210> 60
   <211> 714
   <212> DNA
   <213> Homo sapiens
<400> 60
   atgccagccc gccttgagac ctgcatctcc gacctcgact gcgccagcag cagcggcagt 60
   gacctatccg gcttcctcac cgacgaggaa gactgtgcca gactccaaca ggcagcctcc 120
   gcttcggggc cgcccgcgcc ggcccgcagg ggcgcgccca atatctcccg ggcgtctgag 180
   gttccagggg cacaggacga cgagcaggag aggcggcggc gccgcggccg gacgcgggtc 240
   cgctccgagg cgctgctgca ctcgctgcgc aggagccggc gcgtcaaggc caacgatcgc 300
   gagcgcaacc gcatgcacaa cttgaacgcg gccctggacg cactgcgcag cgtgctgccc 360
   tcgttccccg acgacaccaa gctcaccaaa atcgagacgc tgcgcttcgc ctacaactac 420
   atctgggctc tggccgagac actgcgcctg gcggatcaag ggctgcccgg aggcggtgcc 480
   cgggagcgcc tcctgccgcc gcagtgcgtc ccctgcctgc ccggtccccc aagccccgcc 540
   agcgacgcgg agtcctgggg ctcaggtgcc gccgccgcct ccccgctctc tgaccccagt 600
   agcccagccg cctccgaaga cttcacctac cgccccggcg accctgtttt ctccttccca 660
   agcctgccca aagacttgct ccacacaacg ccctgtttca ttccttacca ctag       714
<210> 61
   <211> 819
   <212> DNA
   <213> Homo sapiens
<400> 61
   atgttcgtca aatccgagac cttggagttg aaggaggaag aggacgtgtt agtgctgctc 60
   ggatcggcct cccccgcctt ggcggccctg accccgctgt catccagcgc cgacgaagaa 120
   gaggaggagg agccgggcgc gtcaggcggg gcgcgtcggc agcgcggggc tgaggccggg 180
   cagggggcgc ggggcggcgt ggctgcgggt gcggagggct gccggcccgc acggctgctg 240
   ggtctggtac acgattgcaa acggcgccct tcccgggcgc gggccgtctc ccgaggcgcc 300
   aagacggccg agacggtgca gcgcatcaag aagacccgta gactgaaggc caacaaccgc 360
   gagcgaaacc gcatgcacaa cctcaacgcg gcactggacg cgctgcgcga ggtgctcccc 420
   acgttccccg aggacgccaa gctcaccaag atcgagaccc tgcgcttcgc ccacaactac 480
   atctgggcac tcaccgagac cctgcgcctg gcggatcact gcgggggcgg cggcgggggc 540
   ctgccggggg cgctcttctc cgaggcagtg ttgctgagcc cgggaggcgc cagcgccgcc 600
   ctgagcagca gcggagacag cccctcgccc gcctccacgt ggagttgcac caacagcccc 660
   gcgccgtcct cctccgtgtc ctccaattcc acctccccct acagctgcac tttatcgccc 720
   gccagcccgg ccgggtcaga catggactat tggcagcccc cacctcccga caagcaccgc 780
   tatgcacctc acctccccat agccagggat tgtatctag                       819
<210> 62
   <211> 645
   <212> DNA
   <213> Mus musculus
<400> 62
   atgacgcctc aaccctcggg tgcgcccact gtccaagtga cccgtgagac ggagcggtcc 60
   ttccccagag cctcggaaga cgaagtgacc tgccccacgt ccgccccgcc cagccccact 120
   cgcacacggg ggaactgcgc agaggcggaa gagggaggct gccgaggggc cccgaggaag 180
   ctccgggcac ggcgcggggg acgcagccgg cctaagagcg agttggcact gagcaagcag 240
   cgacggagtc ggcgaaagaa ggccaacgac cgcgagcgca atcgaatgca caacctcaac 300
   tcggcactgg acgccctgcg cggtgtcctg cccaccttcc cagacgacgc gaagctcacc 360
   aagatcgaga cgctgcgctt cgcccacaac tacatctggg cgctgactca aacgctgcgc 420
   atagcggacc acagcttgta cgcgctggag ccgccggcgc cgcactgcgg ggagctgggc 480
   agcccaggcg gttcccccgg ggactggggg tccctctact ccccagtctc ccaggctggc 540
   agcctgagtc ccgccgcgtc gctggaggag cgacccgggc tgctgggggc caccttttcc 600
   gcctgcttga gcccaggcag tctggctttc tcagattttc tgtga                 645
<210> 63
   <211> 711
   <212> DNA
   <213> Homo sapiens
<400> 63
   atggaaagct ctgccaagat ggagagcggc ggcgccggcc agcagcccca gccgcagccc 60
   cagcagccct tcctgccgcc cgcagcctgt ttctttgcca cggccgcagc cgcggcggcc 120
   gcagccgccg cagcggcagc gcagagcgcg cagcagcagc agcagcagca gcagcagcag 180
   cagcaggcgc cgcagctgag accggcggcc gacggccagc cctcaggggg cggtcacaag 240
   tcagcgccca agcaagtcaa gcgacagcgc tcgtcttcgc ccgaactgat gcgctgcaaa 300
   cgccggctca acttcagcgg ctttggctac agcctgccgc agcagcagcc ggccgccgtg 360
   gcgcgccgca acgagcgcga gcgcaaccgc gtcaagttgg tcaacctggg ctttgccacc 420
   cttcgggagc acgtccccaa cggcgcggcc aacaagaaga tgagtaaggt ggagacactg 480
   cgctcggcgg tcgagtacat ccgcgcgctg cagcagctgc tggacgagca tgacgcggtg 540
   agcgccgcct tccaggcagg cgtcctgtcg cccaccatct cccccaacta ctccaacgac 600
   ttgaactcca tggccggctc gccggtctca tcctactcgt cggacgaggg ctcttacgac 660
   ccgctcagcc ccgaggagca ggagcttctc gacttcacca actggttctg a          711
<210> 64
   <211> 957
   <212> DNA
   <213> Mus musculus
<400> 64
   atggagcttc tatcgccgcc actccgggac atagacttga caggccccga cggctctctc 60
   tgctcctttg agacagcaga cgacttctat gatgacccgt gtttcgactc accagacctg 120
   cgcttttttg aggacctgga cccgcgcctg gtgcacatgg gagccctcct gaaaccggag 180
   gagcacgcac acttccctac tgcggtgcac ccaggcccag gcgctcgtga ggatgagcat 240
   gtgcgcgcgc ccagcgggca ccaccaggcg ggtcgctgct tgctgtgggc ctgcaaggcg 300
   tgcaagcgca agaccaccaa cgctgatcgc cgcaaggccg ccaccatgcg cgagcgccgc 360
   cgcctgagca aagtgaatga ggccttcgag acgctcaagc gctgcacgtc cagcaacccg 420
   aaccagcggc tacccaaggt ggagatcctg cgcaacgcca tccgctacat cgaaggtctg 480
   caggctctgc tgcgcgacca ggacgccgcg ccccctggcg ccgctgcctt ctacgcacct 540
   ggaccgctgc ccccaggccg tggcagcgag cactacagtg gcgactcaga tgcatccagc 600
   ccgcgctcca actgctctga tggcatgatg gattacagcg gccccccaag cggcccccgg 660
   cggcagaatg gctacgacac cgcctactac agtgaggcgg cgcgcgagtc caggccaggg 720
   aagagtgcgg ctgtgtcgag cctcgactgc ctgtccagca tagtggagcg catctccaca 780
   gacagccccg ctgcgcctgc gctgcttttg gcagatgcac caccagagtc gcctccgggt 840
   ccgccagagg gggcatccct aagcgacaca gaacagggaa cccagacccc gtctcccgac 900
   gccgcccctc agtgtcctgc aggctcaaac cccaatgcga tttatcaggt gctttga    957
<210> 65
   <211> 963
   <212> DNA
   <213> Homo sapiens
<400> 65
   atggagctac tgtcgccacc gctccgcgac gtagacctga cggcccccga cggctctctc 60
   tgctcctttg ccacaacgga cgacttctat gacgacccgt gtttcgactc cccggacctg 120
   cgcttcttcg aagacctgga cccgcgcctg atgcacgtgg gcgcgctcct gaaacccgaa 180
   gagcactcgc acttccccgc ggcggtgcac ccggccccgg gcgcacgtga ggacgagcat 240
   gtgcgcgcgc ccagcgggca ccaccaggcg ggccgctgcc tactgtgggc ctgcaaggcg 300
   tgcaagcgca agaccaccaa cgccgaccgc cgcaaggccg ccaccatgcg cgagcggcgc 360
   cgcctgagca aagtaaatga ggcctttgag acactcaagc gctgcacgtc gagcaatcca 420
   aaccagcggt tgcccaaggt ggagatcctg cgcaacgcca tccgctatat cgagggcctg 480
   caggctctgc tgcgcgacca ggacgccgcg ccccctggcg ccgcagccgc cttctatgcg 540
   ccgggcccgc tgcccccggg ccgcggcggc gagcactaca gcggcgactc cgacgcgtcc 600
   agcccgcgct ccaactgctc cgacggcatg atggactaca gcggcccccc gagcggcgcc 660
   cggcggcgga actgctacga aggcgcctac tacaacgagg cgcccagcga acccaggccc 720
   gggaagagtg cggcggtgtc gagcctagac tgcctgtcca gcatcgtgga gcgcatctcc 780
   accgagagcc ctgcggcgcc cgccctcctg ctggcggacg tgccttctga gtcgcctccg 840
   cgcaggcaag aggctgccgc ccccagcgag ggagagagca gcggcgaccc cacccagtca 900
   ccggacgccg ccccgcagtg ccctgcgggt gcgaacccca acccgatata ccaggtgctc 960
   tga 963
<210> 66
   <211> 768
   <212> DNA
   <213> Homo sapiens
<400> 66
   atggacgtga tggatggctg ccagttctca ccttctgagt acttctacga cggctcctgc 60
   ataccgtccc ccgagggtga atttggggac gagtttgtgc cgcgagtggc tgccttcgga 120
   gcgcacaaag cagagctgca gggctcagat gaggacgagc acgtgcgagc gcctaccggc 180
   caccaccagg ctggtcactg cctcatgtgg gcctgcaaag cctgcaagag gaagtccacc 240
   accatggatc ggcggaaggc agccactatg cgcgagcgga ggcgcctgaa gaaggtcaac 300
   caggctttcg aaaccctcaa gaggtgtacc acgaccaacc ccaaccagag gctgcccaag 360
   gtggagatcc tcaggaatgc catccgctac atcgagagcc tgcaggagtt gctgagagag 420
   caggtggaga actactatag cctgccggga cagagctgct cggagcccac cagccccacc 480
   tccaactgct ctgatggcat gcccgaatgt aacagtcctg tctggtccag aaagagcagt 540
   acttttgaca gcatctactg tcctgatgta tcaaatgtat atgccacaga taaaaactcc 600
   ttatccagct tggattgctt atccaacata gtggaccgga tcacctcctc agagcaacct 660
   gggttgcctc tccaggatct ggcttctctc tctccagttg ccagcaccga ttcacagcct 720
   gcaactccag gggcttctag ttccaggctt atctatcatg tgctatga 768
<210> 67
   <211> 729
   <212> DNA
   <213> Homo sapiens
<400> 67
   atgatgatgg acctttttga aactggctcc tatttcttct acttggatgg ggaaaatgtt 60
   actctgcagc cattagaagt ggcagaaggc tctcctttgt atccagggag tgatggtacc 120
   ttgtccccct gccaggacca aatgcccccg gaagcgggga gcgacagcag cggagaggaa 180
   catgtcctgg cgcccccggg cctgcagcct ccacactgcc ccggccagtg tctgatctgg 240
   gcttgcaaga cctgcaagag aaaatctgcc cccactgacc ggcgaaaagc cgccaccctg 300
   cgcgaaagga ggaggctaaa gaaaatcaac gaggccttcg aggcactgaa gcggcgaact 360
   gtggccaacc ccaaccagag gctgcccaag gtggagattc tgcggagcgc catcagctat 420
   attgagcggc tgcaggacct gctgcaccgg ctggatcagc aggagaagat gcaggagctg 480
   ggggtggacc ccttcagcta cagacccaaa caagaaaatc ttgagggtgc ggatttcctg 540
   cgcacctgca gctcccagtg gccaagtgtt tccgatcatt ccagggggct cgtgataacg 600
   gctaaggaag gaggagcaag tattgattcg tcagcctcga gtagccttcg atgcctttct 660
   tccatcgtgg acagtatttc ctcggaggaa cgcaaactcc cctgcgtgga ggaagtggtg 720
   gagaagtaa 729
<210> 68
   <211> 1356
   <212> DNA
   <213> Homo sapiens
<400> 68
   atgccgaaga acaagaagcg gaacactccc caccgcggta gcagtgctgg cggcggcggg 60
   tcaggagcag ccgcagcgac ggcggcgaca gcaggtggcc agcatcgaaa tgttcagcct 120
   tttagtgatg aagatgcatc aattgaaaca atgagccatt gcagtggtta tagcgatcct 180
   tccagttttg ctgaagatgg accagaagtc cttgatgagg aaggaactca agaagaccta 240
   gagtacaagt tgaagggatt aattgaccta accctggata agagtgcgaa gacaaggcaa 300
   gcagctcttg aaggtattaa aaatgcactg gcttcaaaaa tgctgtatga atttattctg 360
   gaaaggagaa tgactttaac tgatagcatt gaacgctgcc tgaaaaaagg taagagtgat 420
   gagcaacgtg cagctgcagc gttagcatct gttctttgta ttcagctggg ccctggaatt 480
   gaaagtgaag agattttgaa aactcttgga ccaatcctaa agaaaatcat ttgtgatggg 540
   tcagctagta tgcaggctag gcaaacttgt gcaacttgct ttggtgtttg ctgttttatt 600
   gccacagatg acattactga actatactca actctggaat gtttggaaaa tatcttcact 660
   aaatcctatc tcaaagagaa agacactact gttatttgca gcactcctaa tacagtgctt 720
   catatcagct ctcttcttgc atggacacta ctgctgacca tatgcccaat caatgaagtg 780
   aagaaaaagc ttgagatgca tttccataag cttccaagcc tcctctcttg tgatgatgta 840
   aacatgagaa tagctgctgg tgaatctttg gcacttctct ttgaattggc cagaggaata 900
   gagagtgact ttttttatga agacatggag tccttgacgc agatgcttag ggccttggca 960
   acagatggaa ataaacaccg ggccaaagtg gacaagagaa agcagcggtc agttttcaga 1020
   gatgtcctga gggcagtgga ggaacgggat tttccaacag aaaccattaa atttggtcct 1080
   gaacgcatgt atattgattg ctgggtaaaa aaacacacct atgacacctt taaggaggtt 1140
   cttggatcag ggatgcagta ccacttgcag tcaaatgaat tccttcgaaa tgtatttgaa 1200
   cttggacccc cagtgatgct tgatgctgca acgcttaaaa cgatgaagat ttctcgtttc 1260
   gaaaggcatt tatataactc tgcagccttc aaagctcgaa ccaaagctag aagcaaatgt 1320
   cgagataaga gagcagatgt tggagaattc ttctag 1356
<210> 69
   <211> 1524
   <212> DNA
   <213> Rattus norvegicus
<400> 69
   atggggcgga agaaaataca aatcacacgc ataatggatg aaaggaaccg acaggtcact 60
   tttacaaaga gaaagtttgg attaatgaag aaagcctatg aacttagtgt gctctgtgac 120
   tgtgaaatag cactcatcat tttcaacagc tctaacaaac tgtttcaata tgctagcact 180
   gatatggaca aagttcttct caagtataca gaatataatg aacctcatga aagcagaacc 240
   aactcggata ttgttgaggc tctgaacaag aaggaacaca gagggtgcga cagcccagac 300
   cctgatactt catatgtgct aactccacat acagaagaaa aatataaaaa aattaatgag 360
   gaatttgata atatgatgcg gaatcataaa atcgcacctg gtctgccacc tcagaacttt 420
   tcaatgtctg tcacagttcc agtgaccagc cccaatgctt tgtcctacac taacccaggg 480
   agttcactgg tgtccccatc tttggcagcc agctcaacgt taacagattc aagcatgctc 540
   tctccacctc aaaccacatt acatagaaat gtgtctcctg gagctcctca gagaccacca 600
   agtactggca atgcaggtgg gatgttgagc actacagacc tcacagtgcc aaatggagct 660
   ggaagcagtc cagtggggaa tggatttgta aactcaagag cttctccaaa tttgattgga 720
   gctactggtg caaatagctt aggcaaagtc atgcctacaa agtctccccc tccaccaggt 780
   ggtggtaatc ttggaatgaa cagtaggaaa ccagatcttc gagttgtcat ccccccttca 840
   agcaagggca tgatgcctcc actatcggag gaagaggaat tggagttgaa cacccaaagg 900
   atcagtagtt ctcaagccac tcaacctctt gctaccccag tcgtgtctgt gacaacccca 960
   agcttgcctc cgcaaggact tgtgtactca gcaatgccga ctgcctacaa cactgattat 1020
   tcactgacca gcgctgacct gtcagccctt caaggcttca actcgccagg aatgctgtcg 1080
   ctgggacagg tgtcggcctg gcagcagcac cacctaggac aagcagccct cagctctctt 1140
   gttgctggag ggcagttatc tcagggttcc aatttatcca ttaataccaa ccaaaacatc 1200
   agcatcaagt ccgaaccgat ttcacctcct cgggatcgta tgaccccatc gggcttccag 1260
   cagcagcagc agcagcagca gcagcagcag ccgccgccac caccgcagcc ccagccacaa 1320
   cccccgcagc cccagccccg acaggaaatg ggcgctcccc ctgtggacag tctgagcagc 1380
   tctagtagct cctatgatgg cagtgatcgg gaggatccac ggggcgactt ccattctcca 1440
   attgtgcttg gccgaccccc aaacactgag gacagagaaa gcccttctgt aaagcgaatg 1500
   aggatggacg cgtgggtgac ctaa 1524
<210> 70
   <211> 675
   <212> DNA
   <213> Homo sapiens
<400> 70
   atggagctgt atgagacatc cccctacttc taccaggaac cccgcttcta tgatggggaa 60
   aactacctgc ctgtccacct ccagggcttc gaaccaccag gctacgagcg gacggagctc 120
   accctgagcc ccgaggcccc agggcccctt gaggacaagg ggctggggac ccccgagcac 180
   tgtccaggcc agtgcctgcc gtgggcgtgt aaggtgtgta agaggaagtc ggtgtccgtg 240
   gaccggcggc gggcggccac actgagggag aagcgcaggc tcaagaaggt gaatgaggcc 300
   ttcgaggccc tgaagagaag caccctgctc aaccccaacc agcggctgcc caaggtggag 360
   atcctgcgca gtgccatcca gtacatcgag cgcctccagg ccctgctcag ctccctcaac 420
   caggaggagc gtgacctccg ctaccggggc gggggcgggc cccagccagg ggtgcccagc 480
   gaatgcagct ctcacagcgc ctcctgcagt ccagagtggg gcagtgcact ggagttcagc 540
   gccaacccag gggatcatct gctcacggct gaccctacag atgcccacaa cctgcactcc 600
   ctcacctcca tcgtggacag catcacagtg gaagatgtgt ctgtggcctt cccagatgaa 660
   accatgccca actga 675
<210> 71
   <211> 822
   <212> DNA
   <213> Homo sapiens
<400> 71
   atgtcgctga ccaacacaaa gacggggttt tcggtcaagg acatcttaga cctgccggac 60
   accaacgatg aggagggctc tgtggccgaa ggtccggagg aagagaacga ggggcccgag 120
   ccagccaaga gggccgggcc gctggggcag ggcgccctgg acgcggtgca gagcctgccc 180
   ctgaagaacc ccttctacga cagcagcgac aacccgtaca cgcgctggct ggccagcacc 240
   gagggccttc agtactccct gcacggtctg gctgccgggg cgccccctca ggactcaagc 300
   tccaagtccc cggagccctc ggccgacgag tcaccggaca atgacaagga gaccccgggc 360
   ggcggggggg acgccggcaa gaagcgaaag cggcgagtgc ttttctccaa ggcgcagacc 420
   tacgagctgg agcggcgctt tcggcagcag cggtacctgt cggcgcccga gcgcgaacac 480
   ctggccagcc tcatccgcct cacgcccacg caggtcaaga tctggttcca gaaccaccgc 540
   tacaagatga agcgcgcccg ggccgagaaa ggtatggagg tgacgcccct gccctcgccg 600
   cgccgggtgg ccgtgcccgt cttggtcagg gacggcaaac catgtcacgc gctcaaagcc 660
   caggacctgg cagccgccac cttccaggcg ggcattccct tttctgccta cagcgcgcag 720
   tcgctgcagc acatgcagta caacgcccag tacagctcgg ccagcacccc ccagtacccg 780
   acagcacacc ccctggtcca ggcccagcag tggacttggt ga 822
<210> 72
   <211> 7668
   <212> DNA
   <213> Homo sapiens
<400> 72
<210> 73
   <211> 6966
   <212> DNA
   <213> Homo sapiens
<400> 73
   atggggccgg gggcccgtgg ccgccgccgc cgccgtcgcc cgatgtcgcc gccaccgcca 60
   ccgccacccg tgcgggcgct gcccctgctg ctgctgctag cggggccggg ggctgcagcc 120
   cccccttgcc tggacggaag cccgtgtgca aatggaggtc gttgcaccca gctgccctcc 180
   cgggaggctg cctgcctgtg cccgcctggc tgggtgggtg agcggtgtca gctggaggac 240
   ccctgtcact caggcccctg tgctggccgt ggtgtctgcc agagttcagt ggtggctggc 300
   accgcccgat tctcatgccg gtgcccccgt ggcttccgag gccctgactg ctccctgcca 360
   gatccctgcc tcagcagccc ttgtgcccac ggtgcccgct gctcagtggg gcccgatgga 420
   cgcttcctct gctcctgccc acctggctac cagggccgca gctgccgaag cgacgtggat 480
   gagtgccggg tgggtgagcc ctgccgccat ggtggcacct gcctcaacac acctggctcc 540
   ttccgctgcc agtgtccagc tggctacaca gggccactat gtgagaaccc cgcggtgccc 600
   tgtgcaccct caccatgccg taacgggggc acctgcaggc agagtggcga cctcacttac 660
   gactgtgcct gtcttcctgg gtttgagggt cagaattgtg aagtgaacgt ggacgactgt 720
   ccaggacacc gatgtctcaa tggggggaca tgcgtggatg gcgtcaacac ctataactgc 780
   cagtgccctc ctgagtggac aggccagttc tgcacggagg acgtggatga gtgtcagctg 840
   cagcccaacg cctgccacaa tgggggtacc tgcttcaaca cgctgggtgg ccacagctgc 900
   gtgtgtgtca atggctggac aggcgagagc tgcagtcaga atatcgatga ctgtgccaca 960
   gccgtgtgct tccatggggc cacctgccat gaccgcgtgg cttctttcta ctgtgcctgc 1020
   cccatgggca agactggcct cctgtgtcac ctggatgacg cctgtgtcag caacccctgc 1080
   cacgaggatg ctatctgtga cacaaatccg gtgaacggcc gggccatttg cacctgtcct 1140
   cccggcttca cgggtggggc atgtgaccag gatgtggacg agtgctctat cggcgccaac 1200
   ccctgcgagc acttgggcag gtgcgtgaac acgcagggct ccttcctgtg ccagtgcggt 1260
   cgtggctaca ctggacctcg ctgtgagacc gatgtcaacg agtgtctgtc ggggccctgc 1320
   cgaaaccagg ccacgtgcct cgaccgcata ggccagttca cctgtatctg tatggcaggc 1380
   ttcacaggaa cctattgcga ggtggacatt gacgagtgtc agagtagccc ctgtgtcaac 1440
   ggtggggtct gcaaggaccg agtcaatggc ttcagctgca cctgcccctc gggcttcagc 1500
   ggctccacgt gtcagctgga cgtggacgaa tgcgccagca cgccctgcag gaatggcgcc 1560
   aaatgcgtgg accagcccga tggctacgag tgccgctgtg ccgagggctt tgagggcacg 1620
   ctgtgtgatc gcaacgtgga cgactgctcc cctgacccat gccaccatgg tcgctgcgtg 1680
   gatggcatcg ccagcttctc atgtgcctgt gctcctggct acacgggcac acgctgcgag 1740
   agccaggtgg acgaatgccg cagccagccc tgccgccatg gcggcaaatg cctagacctg 1800
   gtggacaagt acctctgccg ctgcccttct gggaccacag gtgtgaactg cgaagtgaac 1860
   attgacgact gtgccagcaa cccctgcacc tttggagtct gccgtgatgg catcaaccgc 1920
   tacgactgtg tctgccaacc tggcttcaca gggccccttt gtaacgtgga gatcaatgag 1980
   tgtgcttcca gcccatgcgg cgagggaggt tcctgtgtgg atggggaaaa tggcttccgc 2040
   tgcctctgcc cgcctggctc cttgccccca ctctgcctcc ccccgagcca tccctgtgcc 2100
   catgagccct gcagtcacgg catctgctat gatgcacctg gcgggttccg ctgtgtgtgt 2160
   gagcctggct ggagtggccc ccgctgcagc cagagcctgg cccgagacgc ctgtgagtcc 2220
   cagccgtgca gggccggtgg gacatgcagc agcgatggaa tgggtttcca ctgcacctgc 2280
   ccgcctggtg tccagggacg tcagtgtgaa ctcctctccc cctgcacccc gaacccctgt 2340
   gagcatgggg gccgctgcga gtctgcccct ggccagctgc ctgtctgctc ctgcccccag 2400
   ggctggcaag gcccacgatg ccagcaggat gtggacgagt gtgctggccc cgcaccctgt 2460
   ggccctcatg gtatctgcac caacctggca gggagtttca gctgcacctg ccatggaggg 2520
   tacactggcc cttcctgcga tcaggacatc aatgactgtg accccaaccc atgcctgaac 2580
   ggtggctcgt gccaagacgg cgtgggctcc ttttcctgct cctgcctccc tggtttcgcc 2640
   ggcccacgat gcgcccgcga tgtggatgag tgcctgagca acccctgcgg cccgggcacc 2700
   tgtaccgacc acgtggcctc cttcacctgc acctgcccgc caggctacgg aggcttccac 2760
   tgcgaacagg acctgcccga ctgcagcccc agctcctgct tcaatggcgg gacctgtgtg 2820
   gacggcgtga actcgttcag ctgcctgtgc cgtcccggct acacaggagc ccactgccaa 2880
   catgaggcag acccctgcct ctcgcggccc tgcctacacg gggcgtctgc cagcgccgcc 2940
   caccctggct tccgctgcac ctgcctcgag agcttcacgg gcccgcagtg ccagacgctg 3000
   gtggattggt gcagccgcca gccttgtcaa aacgggggtc gctgcgtcca gactggggcc 3060
   tattgccttt gtccccctgg atggagcgga cgcctctgtg acatccgaag cttgccctgc 3120
   agggaggccg cagcccagat cggggtgcgg ctggagcagc tgtgtcaggc gggtgggcag 3180
   tgtgtggatg aagacagctc ccactactgc gtgtgcccag agggccgtac tggtagccac 3240
   tgtgagcagg aggtggaccc ctgcttggcc cagccctgcc agcatggggg gacctgccgt 3300
   ggctatatgg ggggctacat gtgtgagtgt cttcctggct acaatggtga taactgtgag 3360
   gacgacgtgg acgagtgtgc ctcccagccc tgccagcacg gggttcatgc cattgacctc 3420
   gtggcccgct atctctgctc ctgtccccca ggaacgctgg gggtgctctg cgagattaat 3480
   gaggatgact gcggcccagg cccaccgctg gactcagggc cccggtgcct acacaatggc 3540
   acctgcgtgg acctggtggg tggtttccgc tgcacctgtc ccccaggata cactggtttg 3600
   cgctgcgagg cagacatcaa tgagtgtcgc tcaggtgcct gccacgcggc acacacccgg 3660
   gactgcctgc aggacccagg cggaggtttc cgttgccttt gtcatgctgg cttctcaggt 3720
   cctcgctgtc agactgtcct gtctccctgc gagtcccagc catgccagca tggaggccag 3780
   tgccgtccta gcccgggtcc tgggggtggg ctgaccttca cctgtcactg tgcccagccg 3840
   ttctggggtc cgcgttgcga gcgggtggcg cgctcctgcc gggagctgca gtgcccggtg 3900
   ggcgtcccat gccagcagac gccccgcggg ccgcgctgcg cctgcccccc agggttgtcg 3960
   ggaccctcct gccgcagctt cccggggtcg ccgccggggg ccagcaacgc cagctgcgcg 4020
   gccgccccct gtctccacgg gggctcctgc cgccccgcgc cgctcgcgcc cttcttccgc 4080
   tgcgcttgcg cgcagggctg gaccgggccg cgctgcgagg cgcccgccgc ggcacccgag 4140
   gtctcggagg agccgcggtg cccgcgcgcc gcctgccagg ccaagcgcgg ggaccagcgc 4200
   tgcgaccgcg agtgcaacag cccaggctgc ggctgggacg gcggcgactg ctcgctgagc 4260
   gtgggcgacc cctggcggca atgcgaggcg ctgcagtgct ggcgcctctt caacaacagc 4320
   cgctgcgacc ccgcctgcag ctcgcccgcc tgcctctacg acaacttcga ctgccacgcc 4380
   ggtggccgcg agcgcacttg caacccggtg tacgagaagt actgcgccga ccactttgcc 4440
   gacggccgct gcgaccaggg ctgcaacacg gaggagtgcg gctgggatgg gctggattgt 4500
   gccagcgagg tgccggccct gctggcccgc ggcgtgctgg tgctcacagt gctgctgccg 4560
   ccagaggagc tactgcgttc cagcgccgac tttctgcagc ggctcagcgc catcctgcgc 4620
   acctcgctgc gcttccgcct ggacgcgcac ggccaggcca tggtcttccc ttaccaccgg 4680
   cctagtcctg gctccgaacc ccgggcccgt cgggagctgg cccccgaggt gatcggctcg 4740
   gtagtaatgc tggagattga caaccggctc tgcctgcagt cgcctgagaa tgatcactgc 4800
   ttccccgatg cccagagcgc cgctgactac ctgggagcgt tgtcagcggt ggagcgcctg 4860
   gacttcccgt acccactgcg ggacgtgcgg ggggagccgc tggagcctcc agaacccagc 4920
   gtcccgctgc tgccactgct agtggcgggc gctgtcttgc tgctggtcat tctcgtcctg 4980
   ggtgtcatgg tggcccggcg caagcgcgag cacagcaccc tctggttccc tgagggcttc 5040
   tcactgcaca aggacgtggc ctctggtcac aagggccggc gggaacccgt gggccaggac 5100
   gcgctgggca tgaagaacat ggccaagggt gagagcctga tgggggaggt ggccacagac 5160
   tggatggaca cagagtgccc agaggccaag cggctaaagg tagaggagcc aggcatgggg 5220
   gctgaggagg ctgtggattg ccgtcagtgg actcaacacc atctggttgc tgctgacatc 5280
   cgcgtggcac cagccatggc actgacacca ccacagggcg acgcagatgc tgatggcatg 5340
   gatgtcaatg tgcgtggccc agatggcttc accccgctaa tgctggcttc cttctgtggg 5400
   ggggctctgg agccaatgcc aactgaagag gatgaggcag atgacacatc agctagcatc 5460
   atctccgacc tgatctgcca gggggctcag cttggggcac ggactgaccg tactggcgag 5520
   actgctttgc acctggctgc ccgttatgcc cgtgctgatg cagccaagcg gctgctggat 5580
   gctggggcag acaccaatgc ccaggaccac tcaggccgca ctcccctgca cacagctgtc 5640
   acagccgatg cccagggtgt cttccagatt ctcatccgaa accgctctac agacttggat 5700
   gcccgcatgg cagatggctc aacggcactg atcctggcgg cccgcctggc agtagagggc 5760
   atggtggaag agctcatcgc cagccatgct gatgtcaatg ctgtggatga gcttgggaaa 5820
   tcagccttac actgggctgc ggctgtgaac aacgtggaag ccactttggc cctgctcaaa 5880
   aatggagcca ataaggacat gcaggatagc aaggaggaga cccccctatt cctggccgcc 5940
   cgcgagggca gctatgaggc tgccaagctg ctgttggacc actttgccaa ccgtgagatc 6000
   accgaccacc tggacaggct gccgcgggac gtagcccagg agagactgca ccaggacatc 6060
   gtgcgcttgc tggatcaacc cagtgggccc cgcagccccc ccggtcccca cggcctgggg 6120
   cctctgctct gtcctccagg ggccttcctc cctggcctca aagcggcaca gtcggggtcc 6180
   aagaagagca ggaggccccc cgggaaggcg gggctggggc cgcaggggcc ccgggggcgg 6240
   ggcaagaagc tgacgctggc ctgcccgggc cccctggctg acagctcggt cacgctgtcg 6300
   cccgtggact cgctggactc cccgcggcct ttcggtgggc cccctgcttc ccctggtggc 6360
   ttcccccttg aggggcccta tgcagctgcc actgccactg cagtgtctct ggcacagctt 6420
   ggtggcccag gccgggcggg tctagggcgc cagccccctg gaggatgtgt actcagcctg 6480
   ggcctgctga accctgtggc tgtgcccctc gattgggccc ggctgccccc acctgcccct 6540
   ccaggcccct cgttcctgct gccactggcg ccgggacccc agctgctcaa cccagggacc 6600
   cccgtctccc cgcaggagcg gcccccgcct tacctggcag tcccaggaca tggcgaggag 6660
   tacccggcgg ctggggcaca cagcagcccc ccaaaggccc gcttcctgcg ggttcccagt 6720
   gagcaccctt acctgacccc atcccccgaa tcccctgagc actgggccag cccctcacct 6780
   ccctccctct cagactggtc cgaatccacg cctagcccag ccactgccac tggggccatg 6840
   gccaccacca ctggggcact gcctgcccag ccacttccct tgtctgttcc cagctccctt 6900
   gctcaggccc agacccagct ggggccccag ccggaagtta cccccaagag gcaagtgttg 6960
   gcctga 6966
<210> 74
   <211> 1797
   <212> DNA
   <213> Homo sapiens
<400> 74
   atgccttgtg ttcaggcgca gtatgggtcc tcgcctcaag gagccagccc cgcttctcag 60
   agctacagtt accactcttc gggagaatac agctccgatt tcttaactcc agagtttgtc 120
   aagtttagca tggacctcac caacactgaa atcactgcca ccacttctct ccccagcttc 180
   agtaccttta tggacaacta cagcacaggc tacgacgtca agccaccttg cttgtaccaa 240
   atgcccctgt ccggacagca gtcctccatt aaggtagaag acattcagat gcacaactac 300
   cagcaacaca gccacctgcc cccccagtct gaggagatga tgccgcactc cgggtcggtt 360
   tactacaagc cctcctcgcc cccgacgccc accaccccgg gcttccaggt gcagcacagc 420
   cccatgtggg acgacccggg atctctccac aacttccacc agaactacgt ggccactacg 480
   cacatgatcg agcagaggaa aacgccagtc tcccgcctct ccctcttctc ctttaagcaa 540
   tcgccccctg gcaccccggt gtctagttgc cagatgcgct tcgacgggcc cctgcacgtc 600
   cccatgaacc cggagcccgc cggcagccac cacgtggtgg acgggcagac cttcgctgtg 660
   cccaacccca ttcgcaagcc cgcgtccatg ggcttcccgg gcctgcagat cggccacgcg 720
   tctcagctgc tcgacacgca ggtgccctca ccgccgtcgc ggggctcccc ctccaacgag 780
   gggctgtgcg ctgtgtgtgg ggacaacgcg gcctgccaac actacggcgt gcgcacctgt 840
   gagggctgca aaggcttctt taagcgcaca gtgcaaaaaa atgcaaaata cgtgtgttta 900
   gcaaataaaa actgcccagt ggacaagcgt cgccggaatc gctgtcagta ctgccgattt 960
   cagaagtgcc tggctgttgg gatggtcaaa gaagtggttc gcacagacag tttaaaaggc 1020
   cggagaggtc gtttgccctc gaaaccgaag agcccacagg agccctctcc cccttcgccc 1080
   ccggtgagtc tgatcagtgc cctcgtcagg gcccatgtcg actccaaccc ggctatgacc 1140
   agcctggact attccaggtt ccaggcgaac cctgactatc aaatgagtgg agatgacacc 1200
   cagcatatcc agcaattcta tgatctcctg actggctcca tggagatcat ccggggctgg 1260
   gcagagaaga tccctggctt cgcagacctg cccaaagccg accaagacct gctttttgaa 1320
   tcagctttct tagaactgtt tgtccttcga ttagcataca ggtccaaccc agtggagggt 1380
   aaactcatct tttgcaatgg ggtggtcttg cacaggttgc aatgcgttcg tggctttggg 1440
   gaatggattg attccattgt tgaattctcc tccaacttgc agaatatgaa catcgacatt 1500
   tctgccttct cctgcattgc tgccctggct atggtcacag agagacacgg gctcaaggaa 1560
   cccaagagag tggaagaact gcaaaacaag attgtaaatt gtctcaaaga ccacgtgact 1620
   ttcaacaatg gggggttgaa ccgccccaat tatttgtcca aactgttggg gaagctccca 1680
   gaacttcgta ccctttgcac acaggggcta cagcgcattt tctacctgaa attggaagac 1740
   ttggtgccac cgccagcaat aattgacaaa cttttcctgg acactttacc tttctaa 1797
<210> 75
   <211> 1074
   <212> DNA
   <213> Homo sapiens
<400> 75
   atgcagagtg tgcagagcac gagcttttgt ctccgaaagc agtgcctttg cctgaccttc 60
   ctgcttctcc atctcctggg acaggtcgct gcgactcagc gctgccctcc ccagtgcccg 120
   ggccggtgcc ctgcgacgcc gccgacctgc gcccccgggg tgcgcgcggt gctggacggc 180
   tgctcatgct gtctggtgtg tgcccgccag cgtggcgaga gctgctcaga tctggagcca 240
   tgcgacgaga gcagtggcct ctactgtgat cgcagcgcgg accccagcaa ccagactggc 300
   atctgcacgg cggtagaggg agataactgt gtgttcgatg gggtcatcta ccgcagtgga 360
   gagaaatttc agccaagctg caaattccag tgcacctgca gagatgggca gattggctgt 420
   gtgccccgct gtcagctgga tgtgctactg cctgagccta actgcccagc tccaagaaaa 480
   gttgaggtgc ctggagagtg ctgtgaaaag tggatctgtg gcccagatga ggaggattca 540
   ctgggaggcc ttacccttgc agcttacagg ccagaagcca ccctaggagt agaagtctct 600
   gactcaagtg tcaactgcat tgaacagacc acagagtgga cagcatgctc caagagctgt 660
   ggtatggggt tctccacccg ggtcaccaat aggaaccgtc aatgtgagat gctgaaacag 720
   actcggctct gcatggtgcg gccctgtgaa caagagccag agcagccaac agataagaaa 780
   ggaaaaaagt gtctccgcac caagaagtca ctcaaagcca tccacctgca gttcaagaac 840
   tgcaccagcc tgcacaccta caagcccagg ttctgtgggg tctgcagtga tggccgctgc 900
   tgcactcccc acaataccaa aaccatccag gcagagtttc agtgctcccc agggcaaata 960
   gtcaagaagc cagtgatggt cattgggacc tgcacctgtc acaccaactg tcctaagaac 1020
   aatgaggcct tcctccagga gctggagctg aagactacca gagggaaaat gtaa 1074
<210> 76
   <211> 768
   <212> DNA
   <213> Homo sapiens
<400> 76
   atgctgcggc cacagcggcc cggagacttg cagctcgggg cctccctcta cgagctggtg 60
   ggctacaggc agccgccctc ctcctcctcc tcctccacct cctccacctc ctccacttcc 120
   tcctcctcca cgacggcccc cctcctcccc aaggctgcgc gcgagaagcc ggaggcgccg 180
   gccgagcctc caggccccgg gcccgggtca ggcgcgcacc cgggcggcag cgcccggccg 240
   gacgccaagg aggagcagca gcagcagctg cggcgcaaga tcaacagccg cgagcggaag 300
   cgcatgcagg acctgaacct ggccatggac gccctgcgcg aggtcatcct gccctactca 360
   gcggcgcact gccagggcgc gcccggccgc aagctctcca agatagccac gctgctgctc 420
   gcccgcaact acatcctact gctgggcagc tcgctgcagg agctgcgccg cgcgctgggc 480
   gagggcgccg ggcccgccgc gccgcgcctg ctgctggccg ggctgcccct gctcgccgcc 540
   gcgcccggct ccgtgttgct ggcgcccggc gccgtaggac cccccgacgc gctgcgcccc 600
   gccaagtacc tgtcgctggc gctggacgag ccgccgtgcg gccagttcgc tctccccggc 660
   ggcggcgcag gcggccccgg cctctgcacc tgcgccgtgt gcaagttccc gcacctggtc 720
   ccggccagcc tgggcctggc cgccgtgcag gcgcaattct ccaagtga               768
<210> 77
   <211> 972
   <212> DNA
   <213> Homo sapiens
<400> 77
   atggactcgg acgccagcct ggtgtccagc cgcccgtcgt cgccagagcc cgatgacctt 60
   tttctgccgg cccggagtaa gggcagcagc ggcagcgcct tcactggggg caccgtgtcc 120
   tcgtccaccc cgagtgactg cccgccggag ctgagcgccg agctgcgcgg cgctatgggc 180
   tctgcgggcg cgcatcctgg ggacaagcta ggaggcagtg gcttcaagtc atcctcgtcc 240
   agcacctcgt cgtctacgtc gtcggcggct gcgtcgtcca ccaagaagga caagaagcaa 300
   atgacagagc cggagctgca gcagctgcgt ctcaagatca acagccgcga gcgcaagcgc 360
   atgcacgacc tcaacatcgc catggatggc ctccgcgagg tcatgccgta cgcacacggc 420
   ccttcggtgc gcaagctttc caagatcgcc acgctgctgc tggcgcgcaa ctacatcctc 480
   atgctcacca actcgctgga ggagatgaag cgactggtga gcgagatcta cgggggccac 540
   cacgctggct tccacccgtc ggcctgcggc ggcctggcgc actccgcgcc cctgcccgcc 600
   gccaccgcgc acccggcagc agcagcgcac gccgcacatc accccgcggt gcaccacccc 660
   atcctgccgc ccgccgccgc agcggctgct gccgccgctg cagccgcggc tgtgtccagc 720
   gcctctctgc ccggatccgg gctgccgtcg gtcggctcca tccgtccacc gcacggccta 780
   ctcaagtctc cgtctgctgc cgcggccgcc ccgctggggg gcgggggcgg cggcagtggg 840
   gcgagcgggg gcttccagca ctggggcggc atgccctgcc cctgcagcat gtgccaggtg 900
   ccgccgccgc accaccacgt gtcggctatg ggcgccggca gcctgccgcg cctcacctcc 960
   gacgccaagt ga                                                     972
<210> 78
   <211> 852
   <212> DNA
   <213> Homo sapiens
<400> 78
   atgaacggcg aggagcagta ctacgcggcc acgcagcttt acaaggaccc atgcgcgttc 60
   cagcgaggcc cggcgccgga gttcagcgcc agcccccctg cgtgcctgta catgggccgc 120
   cagcccccgc cgccgccgcc gcacccgttc cctggcgccc tgggcgcgct ggagcagggc 180
   agccccccgg acatctcccc gtacgaggtg ccccccctcg ccgacgaccc cgcggtggcg 240
   caccttcacc accacctccc ggctcagctc gcgctccccc acccgcccgc cgggcccttc 300
   ccggagggag ccgagccggg cgtcctggag gagcccaacc gcgtccagct gcctttccca 360
   tggatgaagt ctaccaaagc tcacgcgtgg aaaggccagt gggcaggcgg cgcctacgct 420
   gcggagccgg aggagaacaa gcggacgcgc acggcctaca cgcgcgcaca gctgctagag 480
   ctggagaagg agttcctatt caacaagtac atctcacggc cgcgccgggt ggagctggct 540
   gtcatgttga acttgaccga gagacacatc aagatctggt tccaaaaccg ccgcatgaag 600
   tggaaaaagg aggaggacaa gaagcgcggc ggcgggacag ctgtcggggg tggcggggtc 660
   gcggagcctg agcaggactg cgccgtgacc tccggcgagg agcttctggc gctgccgccg 720
   ccgccgcccc ccggaggtgc tgtgccgccc gctgcccccg ttgccgcccg agagggccgc 780
   ctgccgcctg gccttagcgc gtcgccacag ccctccagcg tcgcgcctcg gcggccgcag 840
   gaaccacgat ga                                                     852
<210> 79
   <211> 717
   <212> DNA
   <213> Homo sapiens
<400> 79
   atgagacaga gcggcgcctc ccagcccctg ctgatcaaca tgtacctgcc agatcccgtc 60
   ggagacggtc tcttcaagga cgggaagaac ccgagctggg ggccgctgag ccccgcggtt 120
   cagaaaggca gcggacagat ccagctgtgg cagtttctgc tggagctgct ggctgaccgc 180
   gcgaacgccg gctgcatcgc gtgggagggc ggtcacggcg agttcaagct cacggacccg 240
   gacgaggtgg cgcggcggtg gggcgagcgc aagagcaagc ccaacatgaa ctacgacaag 300
   ctgagccgcg ccctgcgcta ctactacgac aagaacatca tgagcaaggt gcatggcaag 360
   cgctacgcct accgcttcga cttccagggc ctggcgcagg cctgccagcc gccgcccgcg 420
   cacgctcatg ccgccgccgc agctgctgcc gccgccgcgg ccgcccagga cggcgcgctc 480
   tacaagctgc ccgccggcct cgccccgctg cccttccccg gcctctccaa actcaacctc 540
   atggccgcct cggccggggt cgcgcccgcc ggcttctcct actggccggg cccgggcccc 600
   gccgccaccg ctgccgccgc caccgccgcg ctctacccca gtcccagctt gcagcccccg 660
   cccgggccct tcggggccgt ggccgcagcc tcgcacttgg ggggccatta ccactag    717
<210> 80
   <211> 855
   <212> DNA
   <213> Homo sapiens
<400> 80
   atggactact cctacctcaa ttcgtacgac tcgtgcgtgg cggccatgga ggcgtccgcc 60
   tacggcgact ttggcgcctg cagccagccc ggcggcttcc aatacagccc cctgcggccc 120
   gctttccccg cggcagggcc gccctgcccc gcgctcggct cctccaactg cgcacttggc 180
   gccctacgcg accaccagcc cgcgccctac tcggcagtgc cctacaagtt cttcccagag 240
   ccatccggcc tgcacgagaa gcgcaagcag cggcgcatcc gcaccacgtt caccagcgcg 300
   cagctcaagg agctggagcg cgttttcgct gagacccact accccgacat ttacacgcgt 360
   gaggagctgg cgctcaagat cgacctcact gaggctcgcg tgcaggtctg gttccagaac 420
   cgccgggcca agttccgcaa acaggagcgc gcggccagcg ccaagggcgc ggcgggcgcg 480
   gcgggcgcca aaaagggcga ggcgcgctgc tcctccgagg acgacgattc caaggagtcc 540
   acgtgcagcc ccacgcccga tagcaccgcc tcgctgccgc cgccgcctgc gcccggcctg 600
   gccagcccgc gcctgagccc cagcccgctg cccgtcgcac tgggctccgg gccgggacct 660
   gggccggggc cacagccgct caagggcgca ctgtgggccg gtgtggcggg cggtgggggc 720
   ggcgggcctg gcgcgggagc ggccgaacta cttaaggctt ggcagccggc ggagtccggc 780
   cccgggccct tctccggggt tctgtcctcc tttcaccgga agcccggccc cgccctgaag 840
   accaatctct tctag                                                  855
<210> 81
   <211> 945
   <212> DNA
   <213> Homo sapiens
<400> 81
   atgtataaaa tggaatattc ttacctcaat tcctctgcct acgagtcctg tatggctggg 60
   atggacacct cgagcctggc ttcagcctat gctgacttca gttcctgcag ccaggccagt 120
   ggcttccagt ataacccgat aaggaccact tttggggcca cgtccggctg cccttccctc 180
   acgccgggat cctgcagcct gggcaccctc agggaccacc agagcagtcc gtacgccgca 240
   gttccttaca aactcttcac ggaccacggc ggcctcaacg agaagcgcaa gcagcggcgc 300
   atccgcacca ctttcaccag tgcccagctc aaagagctgg aaagggtctt cgcggagact 360
   cactaccccg acatctacac tcgggaggag ctggccctga agatcgacct cacagaggcg 420
   cgagtccagg tgtggttcca gaaccgccgc gccaagtttc gcaagcagga gcgcgcagcg 480
   gcagccgcag cggccgcggc caagaacggc tcctcgggca aaaagtctga ctcttccagg 540
   gacgacgaga gcaaagaggc caagagcact gacccggaca gcactggggg cccaggtccc 600
   aatcccaacc ccacccccag ctgcggggcg aatggaggcg gcggcggcgg gcccagcccg 660
   gctggagctc cgggggcggc ggggcccggg ggcccgggag gcgaacccgg caagggcggc 720
   gcagcagcag cggcggcggc cgcggcagcg gcggcggcgg cagcggcagc ggcggcagct 780
   ggaggcctgg ctgcggctgg gggccctgga caaggctggg ctcccggccc cggccccatc 840
   acctccatcc cggattcgct tgggggtccc ttcgccagcg tcctatcttc gctccaaaga 900
   cccaacggtg ccaaagccgc cttagtgaag agcagtatgt tctga                 945
<210> 82
   <211> 876
   <212> DNA
   <213> Homo sapiens
<400> 82
   atgagttgcc aagcttttac ttcggctgat acctttatac ctctgaattc tgacgcctct 60
   gcaactctgc ctctgataat gcatcacagt gctgccgagt gtctaccagt ctccaaccat 120
   gccaccaatg tgatgtctac agcaacagga cttcattatt ctgttccttc ctgtcattat 180
   ggaaaccagc catcaaccta tggagtgatg gcaggtagtt taaccccttg tctttataaa 240
   tttcctgacc acaccttgag tcatggattt cctcctatac accagcctct tctggcagag 300
   gaccccacag ctgctgattt caagcaggaa ctcaggcgga aaagtaaatt ggtggaagag 360
   ccaatagaca tggattctcc agaaatcaga gaacttgaaa agtttgccaa tgaatttaaa 420
   gtgagacgaa ttaaattagg atacacccag acaaatgttg gggaggccct ggcagctgtg 480
   catggctctg aattcagtca aacaacaatc tgccgatttg aaaatctgca gctcagcttt 540
   aaaaatgcat gcaaactgaa agcaatatta tccaaatggc tggaggaagc tgagcaagta 600
   ggagctttgt acaatgaaaa agtgggagca aatgaaagga aaagaaaacg aagaacaact 660
   ataagcattg ctgctaaaga tgctctggag agacactttg gagaacagaa taaaccttct 720
   tctcaagaga tcatgaggat ggctgaagaa ctgaatctgg agaaagaagt agtaagagtt 780
   tggttttgca accggaggca gagagaaaaa cgggtgaaaa caagtctgaa tcagagttta 840
   ttttctattt ctaaggaaca tcttgagtgc agataa                           876
<210> 83
   <211> 909
   <212> DNA
   <213> Homo sapiens
<400> 83
   atggagttcg gcctgctcag cgaggcagag gcccggagcc ctgccctgtc gctgtcagac 60
   gctggcactc cgcaccccca gctcccagag cacggctgca agggccagga gcacagcgac 120
   tcagaaaagg cctcggcttc gctgcccggc ggctccccag aggacggttc gctgaaaaag 180
   aagcagcggc ggcagcgcac gcacttcacc agccagcagc tacaggagct agaggcgacc 240
   ttccagagga accgctaccc cgacatgagc acgcgcgagg agatcgccgt gtggaccaac 300
   ctcaccgagg cccgcgtgcg ggtgtggttc aagaaccggc gcgccaaatg gcggaagcgc 360
   gagcgcagcc agcaggccga gctatgcaaa ggcagcttcg cggcgccgct cggggggctg 420
   gtgccgccct acgaggaggt gtaccccggc tactcgtacg gcaactggcc gcccaaggct 480
   cttgccccgc cgctcgccgc caagaccttt ccattcgcct tcaactcggt caacgtgggg 540
   cctctggctt cgcagcccgt cttctcgcca cccagctcca tcgccgcctc catggtgccc 600
   tccgccgcgg ctgccccggg caccgtgcca gggcctgggg ccctgcaggg cctgggcggg 660
   ggcccccccg ggctggctcc ggccgccgtg tcctccgggg ccgtgtcctg cccttatgcc 720
   tcggccgccg ccgccgccgc ggctgccgcc tcttccccct acgtctatcg ggacccgtgt 780
   aactcgagcc tggccagcct gcggctcaaa gccaaacagc acgcctcctt cagctacccc 840
   gctgtgcacg ggccgccccc ggcagccaac cttagtccgt gccagtacgc cgtggaaagg 900
   cccgtatga                                                         909
<210> 84
   <211> 1362
   <212> DNA
   <213> Homo sapiens
<400> 84
   atgcgtatcc ccgtagatgc cagcacgagc cgccgcttca cgccgccttc caccgcgctg 60
   agcccaggca agatgagcga ggcgttgccg ctgggcgccc cggacgccgg cgctgccctg 120
   gccggcaagc tgaggagcgg cgaccgcagc atggtggagg tgctggccga ccacccgggc 180
   gagctggtgc gcaccgacag ccccaacttc ctctgctccg tgctgcctac gcactggcgc 240
   tgcaacaaga ccctgcccat cgctttcaag gtggtggccc taggggatgt tccagatggc 300
   actctggtca ctgtgatggc tggcaatgat gaaaactact cggctgagct gagaaatgct 360
   accgcagcca tgaagaacca ggttgcaaga tttaatgacc tcaggtttgt cggtcgaagt 420
   ggaagaggga aaagcttcac tctgaccatc actgtcttca caaacccacc gcaagtcgcc 480
   acctaccaca gagccatcaa aatcacagtg gatgggcccc gagaacctcg aagacatcgg 540
   cagaaactag atgatcagac caagcccggg agcttgtcct tttccgagcg gctcagtgaa 600
   ctggagcagc tgcggcgcac agccatgagg gtcagcccac accacccagc ccccacgccc 660
   aaccctcgtg cctccctgaa ccactccact gcctttaacc ctcagcctca gagtcagatg 720
   caggatacaa ggcagatcca accatcccca ccgtggtcct acgatcagtc ctaccaatac 780
   ctgggatcca ttgcctctcc ttctgtgcac ccagcaacgc ccatttcacc tggacgtgcc 840
   agcggcatga caaccctctc tgcagaactt tccagtcgac tctcaacggc acccgacctg 900
   acagcgttca gcgacccgcg ccagttcccc gcgctgccct ccatctccga cccccgcatg 960
   cactatccag gcgccttcac ctactccccg acgccggtca cctcgggcat cggcatcggc 1020
   atgtcggcca tgggctcggc cacgcgctac cacacctacc tgccgccgcc ctaccccggc 1080
   tcgtcgcaag cgcagggagg cccgttccaa gccagctcgc cctcctacca cctgtactac 1140
   ggcgcctcgg ccggctccta ccagttctcc atggtgggcg gcgagcgctc gccgccgcgc 1200
   atcctgccgc cctgcaccaa cgcctccacc ggctccgcgc tgctcaaccc cagcctcccg 1260
   aaccagagcg acgtggtgga ggccgagggc agccacagca actcccccac caacatggcg 1320
   ccctccgcgc gcctggagga ggccgtgtgg aggccctact ga                    1362
<210> 85
   <211> 1704
   <212> DNA
   <213> Homo sapiens
<400> 85
   atgcttcatt cgcctcacaa acaaccacag aaccacaagt gcggtgcaaa ctttctccag 60
   gaggacagca agaagtctct ggtttttaaa tggttaatct ccgcaggtca ctaccagcca 120
   ccgagaccaa cagagtcatt taaggctgca agcagtattt acaacagagg gtacaagttc 180
   tatctgaaaa aaaaaggagg gactatggca tcaaacagcc tcttcagcac agtgacacca 240
   tgtcagcaaa acttcttttg ggatccgagc accagccggc gcttcagccc cccctccagc 300
   agcctgcagc ccggcaaaat gagcgacgtg agcccggtgg tggctgcgca acagcagcag 360
   caacagcagc agcagcaaca gcagcagcag cagcagcaac agcagcagca gcagcaggag 420
   gcggcggcgg cggctgcggc ggcggcggcg gctgcggcgg cggcagctgc agtgccccgg 480
   ttgcggccgc cccacgacaa ccgcaccatg gtggagatca tcgccgacca cccggccgaa 540
   ctcgtccgca ccgacagccc caacttcctg tgctcggtgc tgccctcgca ctggcgctgc 600
   aacaagaccc tgcccgtggc cttcaaggtg gtagccctcg gagaggtacc agatgggact 660
   gtggttactg tcatggcggg taacgatgaa aattattctg ctgagctccg gaatgcctct 720
   gctgttatga aaaaccaagt agcaaggttc aacgatctga gatttgtggg ccggagtgga 780
   cgaggcaaga gtttcacctt gaccataacc gtcttcacaa atcctcccca agtagctacc 840
   tatcacagag caattaaagt tacagtagat ggacctcggg aacccagaag gcacagacag 900
   aagcttgatg actctaaacc tagtttgttc tctgaccgcc tcagtgattt agggcgcatt 960
   cctcatccca gtatgagagt aggtgtcccg cctcagaacc cacggccctc cctgaactct 1020
   gcaccaagtc cttttaatcc acaaggacag agtcagatta cagaccccag gcaggcacag 1080
   tcttccccgc cgtggtccta tgaccagtct tacccctcct acctgagcca gatgacgtcc 1140
   ccgtccatcc actctaccac cccgctgtct tccacacggg gcactgggct tcctgccatc 1200
   accgatgtgc ctaggcgcat ttcaggtgct tcagaactgg gccctttttc agaccccagg 1260
   cagttcccaa gcatttcatc cctcactgag agccgcttct ccaacccacg aatgcactat 1320
   ccagccacct ttacttacac cccgccagtc acctcaggca tgtccctcgg tatgtccgcc 1380
   accactcact accacaccta cctgccacca ccctaccccg gctcttccca aagccagagt 1440
   ggacccttcc agaccagcag cactccatat ctctactatg gcacttcgtc aggatcctat 1500
   cagtttccca tggtgccggg gggagaccgg tctccttcca gaatgcttcc gccatgcacc 1560
   accacctcga atggcagcac gctattaaat ccaaatttgc ctaaccagaa tgatggtgtt 1620
   gacgctgatg gaagccacag cagttcccca actgttttga attctagtgg cagaatggat 1680
   gaatctgttt ggcgaccata ttga                                       1704
<210> 86
   <211> 1389
   <212> DNA
   <213> Homo sapiens
<400> 86
   atgctgctgc tggcgagatg tctgctgcta gtcctcgtct cctcgctgct ggtatgctcg 60
   ggactggcgt gcggaccggg cagggggttc gggaagagga ggcaccccaa aaagctgacc 120
   cctttagcct acaagcagtt tatccccaat gtggccgaga agaccctagg cgccagcgga 180
   aggtatgaag ggaagatctc cagaaactcc gagcgattta aggaactcac ccccaattac 240
   aaccccgaca tcatatttaa ggatgaagaa aacaccggag cggacaggct gatgactcag 300
   aggtgtaagg acaagttgaa cgctttggcc atctcggtga tgaaccagtg gccaggagtg 360
   aaactgcggg tgaccgaggg ctgggacgaa gatggccacc actcagagga gtctctgcac 420
   tacgagggcc gcgcagtgga catcaccacg tctgaccgcg accgcagcaa gtacggcatg 480
   ctggcccgcc tggcggtgga ggccggcttc gactgggtgt actacgagtc caaggcacat 540
   atccactgct cggtgaaagc agagaactcg gtggcggcca aatcgggagg ctgcttcccg 600
   ggctcggcca cggtgcacct ggagcagggc ggcaccaagc tggtgaagga cctgagcccc 660
   ggggaccgcg tgctggcggc ggacgaccag ggccggctgc tctacagcga cttcctcact 720
   ttcctggacc gcgacgacgg cgccaagaag gtcttctacg tgatcgagac gcgggagccg 780
   cgcgagcgcc tgctgctcac cgccgcgcac ctgctctttg tggcgccgca caacgactcg 840
   gccaccgggg agcccgaggc gtcctcgggc tcggggccgc cttccggggg cgcactgggg 900
   cctcgggcgc tgttcgccag ccgcgtgcgc ccgggccagc gcgtgtacgt ggtggccgag 960
   cgtgacgggg accgccggct cctgcccgcc gctgtgcaca gcgtgaccct aagcgaggag 1020
   gccgcgggcg cctacgcgcc gctcacggcc cagggcacca ttctcatcaa ccgggtgctg 1080
   gcctcgtgct acgcggtcat cgaggagcac agctgggcgc accgggcctt cgcgcccttc 1140
   cgcctggcgc acgcgctcct ggctgcactg gcgcccgcgc gcacggaccg cggcggggac 1200
   agcggcggcg gggaccgcgg gggcggcggc ggcagagtag ccctaaccgc tccaggtgct 1260
   gccgacgctc cgggtgcggg ggccaccgcg ggcatccact ggtactcgca gctgctctac 1320
   caaataggca cctggctcct ggacagcgag gccctgcacc cgctgggcat ggcggtcaag 1380
   tccagctga                                                    1389
<210> 87
   <211> 1530
   <212> DNA
   <213> Homo sapiens
<400> 87
   atgaatctcc tggacccctt catgaagatg accgacgagc aggagaaggg cctgtccggc 60
   gcccccagcc ccaccatgtc cgaggactcc gcgggctcgc cctgcccgtc gggctccggc 120
   tcggacaccg agaacacgcg gccccaggag aacacgttcc ccaagggcga gcccgatctg 180
   aagaaggaga gcgaggagga caagttcccc gtgtgcatcc gcgaggcggt cagccaggtg 240
   ctcaaaggct acgactggac gctggtgccc atgccggtgc gcgtcaacgg ctccagcaag 300
   aacaagccgc acgtcaagcg gcccatgaac gccttcatgg tgtgggcgca ggcggcgcgc 360
   aggaagctcg cggaccagta cccgcacttg cacaacgccg agctcagcaa gacgctgggc 420
   aagctctgga gacttctgaa cgagagcgag aagcggccct tcgtggagga ggcggagcgg 480
   ctgcgcgtgc agcacaagaa ggaccacccg gattacaagt accagccgcg gcggaggaag 540
   tcggtgaaga acgggcaggc ggaggcagag gaggccacgg agcagacgca catctccccc 600
   aacgccatct tcaaggcgct gcaggccgac tcgccacact cctcctccgg catgagcgag 660
   gtgcactccc ccggcgagca ctcggggcaa tcccagggcc caccgacccc acccaccacc 720
   cccaaaaccg acgtgcagcc gggcaaggct gacctgaagc gagaggggcg ccccttgcca 780
   gaggggggca gacagccccc tatcgacttc cgcgacgtgg acatcggcga gctgagcagc 840
   gacgtcatct ccaacatcga gaccttcgat gtcaacgagt ttgaccagta cctgccgccc 900
   aacggccacc cgggggtgcc ggccacgcac ggccaggtca cctacacggg cagctacggc 960
   atcagcagca ccgcggccac cccggcgagc gcgggccacg tgtggatgtc caagcagcag 1020
   gcgccgccgc cacccccgca gcagccccca caggccccgc cggccccgca ggcgcccccg 1080
   cagccgcagg cggcgccccc acagcagccg gcggcacccc cgcagcagcc acaggcgcac 1140
   acgctgacca cgctgagcag cgagccgggc cagtcccagc gaacgcacat caagacggag 1200
   cagctgagcc ccagccacta cagcgagcag cagcagcact cgccccaaca gatcgcctac 1260
   agccccttca acctcccaca ctacagcccc tcctacccgc ccatcacccg ctcacagtac 1320
   gactacaccg accaccagaa ctccagctcc tactacagcc acgcggcagg ccagggcacc 1380
   ggcctctact ccaccttcac ctacatgaac cccgctcagc gccccatgta cacccccatc 1440
   gccgacacct ctggggtccc ttccatcccg cagacccaca gcccccagca ctgggaacaa 1500
   cccgtctaca cacagctcac tcgaccttga                                  1530
<210> 88
   <211> 1245
   <212> DNA
   <213> Homo sapiens
<400> 88
   atgagcagcc cggatgcggg atacgccagt gacgaccaga gccagaccca gagcgcgctg 60
   cccgcggtga tggccgggct gggcccctgc ccctgggccg agtcgctgag ccccatcggg 120
   gacatgaagg tgaagggcga ggcgccggcg aacagcggag caccggccgg ggccgcgggc 180
   cgagccaagg gcgagtcccg tatccggcgg ccgatgaacg ctttcatggt gtgggctaag 240
   gacgagcgca agcggctggc gcagcagaat ccagacctgc acaacgccga gttgagcaag 300
   atgctgggca agtcgtggaa ggcgctgacg ctggcggaga agcggccctt cgtggaggag 360
   gcagagcggc tgcgcgtgca gcacatgcag gaccacccca actacaagta ccggccgcgg 420
   cggcgcaagc aggtgaagcg gctgaagcgg gtggagggcg gcttcctgca cggcctggct 480
   gagccgcagg cggccgcgct gggccccgag ggcggccgcg tggccatgga cggcctgggc 540
   ctccagttcc ccgagcaggg cttccccgcc ggcccgccgc tgctgcctcc gcacatgggc 600
   ggccactacc gcgactgcca gagtctgggc gcgcctccgc tcgacggcta cccgttgccc 660
   acgcccgaca cgtccccgct ggacggcgtg gaccccgacc cggctttctt cgccgccccg 720
   atgcccgggg actgcccggc ggccggcacc tacagctacg cgcaggtctc ggactacgct 780
   ggccccccgg agcctcccgc cggtcccatg cacccccgac tcggcccaga gcccgcgggt 840
   ccctcgattc cgggcctcct ggcgccaccc agcgcccttc acgtgtacta cggcgcgatg 900
   ggctcgcccg gggcgggcgg cgggcgcggc ttccagatgc agccgcaaca ccagcaccag 960
   caccagcacc agcaccaccc cccgggcccc ggacagccgt cgccccctcc ggaggcactg 1020
   ccctgccggg acggcacgga ccccagtcag cccgccgagc tcctcgggga ggtggaccgc 1080
   acggaatttg aacagtatct gcacttcgtg tgcaagcctg agatgggcct cccctaccag 1140
   gggcatgact ccggtgtgaa tctccccgac agccacgggg ccatttcctc ggtggtgtcc 1200
   gacgccagct ccgcggtata ttactgcaac tatcctgacg tgtga                 1245
<210> 89
   <211> 987
   <212> DNA
   <213> Homo sapiens
<400> 89
   atgactggag tctttgacag tctagtggct gatatgcact cgacccagat cgccgcctcc 60
   agcacgtacc accagcacca gcagcccccg agcggcggcg gcgccggccc gggtggcaac 120
   agcagcagca gcagcagcct ccacaagccc caggagtcgc ccacccttcc ggtgtccacc 180
   gccaccgaca gcagctacta caccaaccag cagcacccgg cgggcggcgg cggcggcggg 240
   ggctcgccct acgcgcacat gggttcctac cagtaccaag ccagcggcct caacaacgtc 300
   ccttactccg ccaagagcag ctatgacctg ggctacaccg ccgcctacac ctcctacgct 360
   ccctatggaa ccagttcgtc cccagccaac aacgagcctg agaaggagga ccttgagcct 420
   gaaattcgga tagtgaacgg gaagccaaag aaagtccgga aaccccgcac catctactcc 480
   agtttccagc tggcggctct tcagcggcgt ttccaaaaga ctcaatactt ggccttgccg 540
   gagcgagccg agctggcggc ctctctgggc ctcacccaga ctcaggtcaa aatctggttc 600
   cagaaccgcc ggtccaagtt caagaagatg tggaaaagtg gtgagatccc ctcggagcag 660
   caccctgggg ccagcgcttc tccaccttgt gcttcgccgc cagtctcagc gccggcctcc 720
   tgggactttg gtgtgccgca gcggatggcg ggcggcggtg gtccgggcag tggcggcagc 780
   ggcgccggca gctcgggctc cagcccgagc agcgcggcct cggcttttct gggcaactac 840
   ccctggtacc accagacctc gggatccgcc tcacacctgc aggccacggc gccgctgctg 900
   caccccactc agaccccgca gccgcatcac caccaccacc atcacggcgg cgggggcgcc 960
   ccggtgagcg cggggacgat tttctaa                                      987
<210> 90
   <211> 870
   <212> DNA
   <213> Homo sapiens
<400> 90
   atgacaggag tgtttgacag aagggtcccc agcatccgat ccggcgactt ccaagctccg 60
   ttccagacgt ccgcagctat gcaccatccg tctcaggaat cgccaacttt gcccgagtct 120
   tcagctaccg attctgacta ctacagccct acgggggagg ccccgcacgg ctactgctct 180
   cctacctcgg cttcctatgg caaagctctc aacccctacc agtatcagta tcacggcgtg 240
   aacggctccg ccgggagcta cccagccaaa gcttatgccg actatagcta cgctagctcc 300
   taccaccagt acggcggcgc ctacaaccgc gtcccaagcg ccaccaacca gccagagaaa 360
   gaagtgaccg agcccgaggt gagaatggtg aatggcaaac caaagaaagt tcgtaaaccc 420
   aggactattt attccagctt tcagctggcc gcattacaga gaaggtttca gaagactcag 480
   tacctcgcct tgccggaacg cgccgagctg gccgcctcgc tgggattgac acaaacacag 540
   gtgaaaatct ggtttcagaa caaaagatcc aagatcaaga agatcatgaa aaacggggag 600
   atgcccccgg agcacagtcc cagctccagc gacccaatgg cgtgtaactc gccgcagtct 660
   ccagcggtgt gggagcccca gggctcgtcc cgctcgctca gccaccaccc tcatgcccac 720
   cctccgacct ccaaccagtc cccagcgtcc agctacctgg agaactctgc atcctggtac 780
   acaagtgcag ccagctcaat caattcccac ctgccgccgc cgggctcctt acagcacccg 840
   ctggcgctgg cctccgggac actctattag                                  870
<210> 91
   <211> 843
   <212> DNA
   <213> Homo sapiens
<400> 91
   atgccagctg atataatgga gaaaaattcc tcgtccccgg tggctgctac cccagccagt 60
   gtcaacacga caccggataa accaaagaca gcatctgagc acagaaagtc atcaaagcct 120
   attatggaga aaagacgaag agcaagaata aatgaaagtc tgagccagct gaaaacactg 180
   attttggatg ctctgaagaa agatagctcg cggcattcca agctggagaa ggcggacatt 240
   ctggaaatga cagtgaagca cctccggaac ctgcagcggg cgcagatgac ggctgcgctg 300
   agcacagacc caagtgtgct ggggaagtac cgagccggct tcagcgagtg catgaacgag 360
   gtgacccgct tcctgtccac gtgcgagggc gttaataccg aggtgcgcac tcggctgctc 420
   ggccacctgg ccaactgcat gacccagatc aatgccatga cctaccccgg gcagccgcac 480
   cccgccttgc aggcgccgcc accgccccca ccgggacccg gcggccccca gcacgcgccg 540
   ttcgcgccgc cgccgccact cgtgcccatc cccgggggcg cggcgccccc tcccggcggc 600
   gccccctgca agctgggcag ccaggctgga gaggcggcta aggtgtttgg aggcttccag 660
   gtggtaccgg ctcccgatgg ccagtttgct ttcctcattc ccaacggggc cttcgcgcac 720
   agcggccctg tcatccccgt ctacaccagc aacagcggca cctccgtggg ccccaacgca 780
   gtgtcacctt ccagcggccc ctcgcttacg gcggactcca tgtggaggcc gtggcggaac 840
   tga                                                               843
<210> 92
   <211> 648
   <212> DNA
   <213> Homo sapiens
<400> 92
   atgggcagcc cccgctccgc gctgagctgc ctgctgttgc acttgctggt cctctgcctc 60
   caagcccagg taactgttca gtcctcacct aattttacac agcatgtgag ggagcagagc 120
   ctggtgacgg atcagctcag ccgccgcctc atccggacct accaactcta cagccgcacc 180
   agcgggaagc acgtgcaggt cctggccaac aagcgcatca acgccatggc agaggacggc 240
   gaccccttcg caaagctcat cgtggagacg gacacctttg gaagcagagt tcgagtccga 300
   ggagccgaga cgggcctcta catctgcatg aacaagaagg ggaagctgat cgccaagagc 360
   aacggcaaag gcaaggactg cgtcttcacg gagattgtgc tggagaacaa ctacacagcg 420
   ctgcagaatg ccaagtacga gggctggtac atggccttca cccgcaaggg ccggccccgc 480
   aagggctcca agacgcggca gcaccagcgt gaggtccact tcatgaagcg gctgccccgg 540
   ggccaccaca ccaccgagca gagcctgcgc ttcgagttcc tcaactaccc gcccttcacg 600
   cgcagcctgc gcggcagcca gaggacttgg gcccccgagc cccgatag              648
<210> 93
   <211> 975
   <212> DNA
   <213> Homo sapiens
<400> 93
   atgaactgca tgaaaggccc gcttcacttg gagcaccgag cagcggggac caagctgtcg 60
   gccgtctcct catcttcctg tcaccatccc cagccgttag ccatggcttc ggttctggct 120
   cccggtcagc cccggtcgct ggactcctcc aagcacaggc tggaggtgca caccatctcc 180
   gacacctcca gcccggaggc cgcagagaaa gataaaagcc agcaggggaa gaatgaggac 240
   gtgggcgccg aggacccgtc taagaagaag cggcaaaggc ggcagcggac tcactttacc 300
   agccagcagc tccaggagct ggaggccact ttccagagga accgctaccc ggacatgtcc 360
   acacgcgaag aaatcgctgt gtggaccaac cttacggaag cccgagtccg ggtttggttc 420
   aagaatcgtc gggccaaatg gagaaagagg gagcgcaacc agcaggccga gctatgcaag 480
   aatggcttcg ggccgcagtt caatgggctc atgcagccct acgacgacat gtacccaggc 540
   tattcctaca acaactgggc cgccaagggc cttacatccg cctccctatc caccaagagc 600
   ttccccttct tcaactctat gaacgtcaac cccctgtcat cacagagcat gttttcccca 660
   cccaactcta tctcgtccat gagcatgtcg tccagcatgg tgccctcagc agtgacaggc 720
   gtcccgggct ccagtctcaa cagcctgaat aacttgaaca acctgagtag cccgtcgctg 780
   aattccgcgg tgccgacgcc tgcctgtcct tacgcgccgc cgactcctcc gtatgtttat 840
   agggacacgt gtaactcgag cctggccagc ctgagactga aagcaaagca gcactccagc 900
   ttcggctacg ccagcgtgca gaacccggcc tccaacctga gtgcttgcca gtatgcagtg 960
   gaccggcccg tgtga                                                  975
<210> 94
   <211> 987
   <212> DNA
   <213> Mus musculus
<400> 94
   atggccaccc aggtgatggg gcagtcttct ggaggaggca gtctcttcaa caacagtgcc 60
   aacatgggca tggccttaac caacgacatg tacgacctgc acgagctctc gaaagctgaa 120
   ctggcagccc ctcagctcat catgttagcc aacgtggccc tgacggggga ggcaagcggc 180
   agctgctgcg attacctggt cggtgaagag aggcagatgg ccgaattgat gcccgtggga 240
   gacaaccact tctcagaaag tgaaggagaa ggcctggaag agtcggctga cctcaaaggg 300
   ctggaaaaca tggaactggg aagtttggag ctaagtgctg tagaacccca gcccgtattt 360
   gaagcctcag ctgccccaga aatatacagc gccaataaag atcccgctcc agaaacaccc 420
   gtggcggaag acaaatgcag gagttctaag gccaagccct tccggtgtaa gccttgccag 480
   tacgaagccg aatctgaaga gcagtttgtg catcacatcc ggattcacag cgctaagaag 540
   ttctttgtgg aggaaagtgc agagaaacag gccaaagcct gggagtcggg gtcgtctccg 600
   gccgaagagg gcgagttctc caaaggcccc atccgctgtg accgctgtgg ctacaatacc 660
   aaccggtatg accactacat ggcacacctg aagcaccacc tgcgagctgg cgagaacgag 720
   cgcatctaca agtgcatcat ctgcacgtac acgacggtca gcgagtacca ctggaggaaa 780
   cacctgagaa accatttccc caggaaagtc tacacctgca gcaagtgcaa ctacttctca 840
   gacagaaaaa ataactacgt tcagcacgtg cgaactcaca caggagaacg cccgtataaa 900
   tgtgaacttt gtccttactc aagctctcag aagactcatc taacgcgaca catgcggact 960
   cattcagagt gtgatctagc tgggtga                                     987
<210> 95
   <211> 1025
   <212> DNA
   <213> Homo sapiens
<400> 95
   atgaccatgg aatctggagc cgagaaccag cagagtggag atgcagctgt aacagaagct 60
   gaaaaccaac aaatgacagt tcaagcccag ccacagattg ccacattagc ccaggtatct 120
   atgccagcag ctcatgcaac atcatctgct cccaccgtaa ctctagtaca gctgcccaat 180
   gggcagacag ttcaagtcca tggagtcatt caggcggccc agccatcagt tattcagtct 240
   ccacaagtcc aaacagttca gtcttcctgt aaggacttaa aaagactttt ctccggaaca 300
   cagatttcaa ctattgcaga aagtgaagat tcacaggagt cagtggatag tgtaactgat 360
   tcccaaaagc gaagggaaat tctttcaagg aggccttcct acaggaaaat tttgaatgac 420
   ttatcttctg atgcaccagg agtgccaagg attgaagaag agaagtctga agaggagact 480
   tcagcacctg ccatcaccac tgtaacggtg ccaactccaa tttaccaaac tagcagtgga 540
   cagtatattg ccattaccca gggaggagca atacagctgg ctaacaatgg taccgatggg 600
   gtacagggcc tgcaaacatt aaccatgacc aatgcagcag ccactcagcc gggtactacc 660
   attctacagt atgcacagac cactgatgga cagcagatct tagtgcccag caaccaagtt 720
   gttgttcaag ctgcctctgg agacgtacaa acataccaga ttcgcacagc acccactagc 780
   actattgccc ctggagttgt tatggcatcc tccccagcac ttcctacaca gcctgctgaa 840
   gaagcagcac gaaagagaga ggtccgtcta atgaagaaca gggaagcagc tcgagagtgt 900
   cgtagaaaga agaaagaata tgtgaaatgt ttagaaaaca gagtggcagt gcttgaaaat 960
   caaaacaaga cattgattga ggagctaaaa gcacttaagg acctttactg ccacaaatca 1020
   gatta                                                             1025
<210> 96
   <211> 963
   <212> DNA
   <213> Mus musculus
<400> 96
   atggctgagg gcaaaggggc tcctctgagg ccttcagttg agaagagatg gaagctcatg 60
   gaacccaagc agacccaggc agggatgttc aagaaaatga gccttgtgga ctctgacact 120
   gctgcaggaa agggtagcca agatgaggcc tatactgaac tgagcctgcc aacagcaccg 180
   aacaagcctc gactggacag gcctcgggcc tgcaaggcat acacagagca gaggcacaat 240
   accttcacag agctatcatg tctccaggag aggccagggg acatccaggc ccagacgagg 300
   aagctggaga acccagaagg ccagctcggc cctcagcagc tgccctcgag tttcctcaga 360
   gcctcaggtg atggcacagt gtgttcagca tggccaggtg ccccccggag tgagcagaaa 420
   agtgctttca gcaagccagc caaacgccca gcagagaaac ctaagcgctc tcccatgctt 480
   ctggctggtg gaagtgcaga gggctcatgg gagctctcag gactcatcac cactgtggac 540
   atcccatatt gggctcatct gtcaactttc aagttcatgg gtgatttctg gaaattgcac 600
   acattgtcac agaacattct cctctgcaat gctttccagg gggctcccac accatggctg 660
   gagcataccc aggtacaagc ccccacatcc tcagctcctt cctccacagc ctcccggcct 720
   ctcttgccgc ccacactctc ctccttgggc ttgtctactc agaactggtg tgcgaagtgc 780
   aacctagcct ttcgcctgac agctgacctg gtcttccaca tgcggtcaca tcacaaaagg 840
   gaacacgtgg gccctgaccc acattctaag aaacgaagag aggaagttct cacttgcccc 900
   gtttgccacg agtacttccg ggagcgccac catctgtcca ggcatatggc ttcacatagt 960
   tag                                                               963
<210> 97
   <211> 1374
   <212> DNA
   <213> Homo sapiens
<400> 97
   atgctgggag cggtgaagat ggaagggcac gagccgtccg actggagcag ctactatgca 60
   gagcccgagg gctactcctc cgtgagcaac atgaacgccg gcctggggat gaacggcatg 120
   aacacgtaca tgagcatgtc ggcggccgcc atgggcagcg gctcgggcaa catgagcgcg 180
   ggctccatga acatgtcgtc gtacgtgggc gctggcatga gcccgtccct ggcggggatg 240
   tcccccggcg cgggcgccat ggcgggcatg ggcggctcgg ccggggcggc cggcgtggcg 300
   ggcatggggc cgcacttgag tcccagcctg agcccgctcg gggggcaggc ggccggggcc 360
   atgggcggcc tggcccccta cgccaacatg aactccatga gccccatgta cgggcaggcg 420
   ggcctgagcc gcgcccgcga ccccaagacc tacaggcgca gctacacgca cgcaaagccg 480
   ccctactcgt acatctcgct catcaccatg gccatccagc agagccccaa caagatgctg 540
   acgctgagcg agatctacca gtggatcatg gacctcttcc ccttctaccg gcagaaccag 600
   cagcgctggc agaactccat ccgccactcg ctctccttca acgactgttt cctgaaggtg 660
   ccccgctcgc ccgacaagcc cggcaagggc tccttctgga ccctgcaccc tgactcgggc 720
   aacatgttcg agaacggctg ctacctgcgc cgccagaagc gcttcaagtg cgagaagcag 780
   ctggcgctga aggaggccgc aggcgccgcc ggcagcggca agaaggcggc cgccggagcc 840
   caggcctcac aggctcaact cggggaggcc gccgggccgg cctccgagac tccggcgggc 900
   accgagtcgc ctcactcgag cgcctccccg tgccaggagc acaagcgagg gggcctggga 960
   gagctgaagg ggacgccggc tgcggcgctg agccccccag agccggcgcc ctctcccggg 1020
   cagcagcagc aggccgcggc ccacctgctg ggcccgcccc accacccggg cctgccgcct 1080
   gaggcccacc tgaagccgga acaccactac gccttcaacc acccgttctc catcaacaac 1140
   ctcatgtcct cggagcagca gcaccaccac agccaccacc accaccaacc ccacaaaatg 1200
   gacctcaagg cctacgaaca ggtgatgcac taccccggct acggttcccc catgcctggc 1260
   agcttggcca tgggcccggt cacgaacaaa acgggcctgg acgcctcgcc cctggccgca 1320
   gatacctcct actaccaggg ggtgtactcc cggcccatta tgaactcctc ttaa       1374
<210> 98
   <211> 1221
   <212> DNA
   <213> Homo sapiens
<400> 98
   atggatggat ggagaaggat gcctcgctgg ggactgctgc tgctgctctg gggctcctgt 60
   acctttggtc tcccgacaga caccaccacc tttaaacgga tcttcctcaa gagaatgccc 120
   tcaatccgag aaagcctgaa ggaacgaggt gtggacatgg ccaggcttgg tcccgagtgg 180
   agccaaccca tgaagaggct gacacttggc aacaccacct cctccgtgat cctcaccaac 240
   tacatggaca cccagtacta tggcgagatt gggatcggga ccccacccca aaccttcaaa 300
   gtcgtctttg acactggttc gtccaatgtt tgggtgccct cctccaagtg cagccgtctc 360
   tacactgcct gtgtgtatca caagctcttc gatgcttcgg attcctccag ctacaagcac 420
   aatggaacag aactcaccct ccgctattca acagggacag tcagtggctt tctcagccag 480
   gacatcatca ccgtgggtgg aatcacggtg acacagatgt ttggagaggt cacggagatg 540
   cccgccttac ccttcatgct ggccgagttt gatggggttg tgggcatggg cttcattgaa 600
   caggccattg gcagggtcac ccctatcttc gacaacatca tctcccaagg ggtgctaaaa 660
   gaggacgtct tctctttcta ctacaacaga gattccgaga attcccaatc gctgggagga 720
   cagattgtgc tgggaggcag cgacccccag cattacgaag ggaatttcca ctatatcaac 780
   ctcatcaaga ctggtgtctg gcagattcaa atgaaggggg tgtctgtggg gtcatccacc 840
   ttgctctgtg aagacggctg cctggcattg gtagacaccg gtgcatccta catctcaggt 900
   tctaccagct ccatagagaa gctcatggag gccttgggag ccaagaagag gctgtttgat 960
   tatgtcgtga agtgtaacga gggccctaca ctccccgaca tctctttcca cctgggaggc 1020
   aaagaataca cgctcaccag cgcggactat gtatttcagg aatcctacag tagtaaaaag 1080
   ctgtgcacac tggccatcca cgccatggat atcccgccac ccactggacc cacctgggcc 1140
   ctgggggcca ccttcatccg aaagttctac acagagtttg atcggcgtaa caaccgcatt 1200
   ggcttcgcct tggcccgctg a                                           1221
<210> 99
   <211> 654
   <212> DNA
   <213> Homo sapiens
<400> 99
   atgagtctgg taggtggttt tccccaccac ccggtggtgc accacgaggg ctacccgttt 60
   gccgccgccg ccgccgcagc tgccgccgcc gccgccagcc gctgcagcca tgaggagaac 120
   ccctacttcc atggctggct catcggccac cccgagatgt cgccccccga ctacagcatg 180
   gccctgtcct acagccccga gtatgccagc ggcgccgccg gcctggacca ctcccattac 240
   gggggggtgc cgccgggcgc cgggcccccg ggcctggggg ggccgcgccc ggtgaagcgc 300
   cgaggcaccg ccaaccgcaa ggagcggcgc aggactcaga gcatcaacag cgccttcgcc 360
   gaactgcgcg agtgcatccc caacgtaccc gccgacacca aactctccaa aatcaagacc 420
   ctgcgcctgg ccaccagcta catcgcctac ctcatggacc tgctggccaa ggacgaccag 480
   aatggcgagg cggaggcctt caaggcagag atcaagaaga ccgacgtgaa agaggagaag 540
   aggaagaagg agctgaacga aatcttgaaa agcacagtga gcagcaacga caagaaaacc 600
   aaaggccgga cgggctggcc gcagcacgtc tgggccctgg agctcaagca gtga       654
<210> 100
   <211> 942
   <212> DNA
   <213> Homo sapiens
<400> 100
   atgacttctt gtcacattgc tgaagaacat atacaaaagg ttgctatctt tggaggaacc 60
   catgggaatg agctaaccgg agtatttctg gttaagcatt ggctagagaa tggcgctgag 120
   attcagagaa cagggctgga ggtaaaacca tttattacta accccagagc agtgaagaag 180
   tgtaccagat atattgactg tgacctgaat cgcatttttg accttgaaaa tcttggcaaa 240
   aaaatgtcag aagatttgcc atatgaagtg agaagggctc aagaaataaa tcatttattt 300
   ggtccaaaag acagtgaaga ttcctatgac attatttttg accttcacaa caccacctct 360
   aacatggggt gcactcttat tcttgaggat tccaggaata actttttaat tcagatgttt 420
   cattacatta agacttctct ggctccacta ccctgctacg tttatctgat tgagcatcct 480
   tccctcaaat atgcgaccac tcgttccata gccaagtatc ctgtgggtat agaagttggt 540
   cctcagcctc aaggggttct gagagctgat atcttggatc aaatgagaaa aatgattaaa 600
   catgctcttg attttataca tcatttcaat gaaggaaaag aatttcctcc ctgcgccatt 660
   gaggtctata aaattataga gaaagttgat tacccccggg atgaaaatgg agaaattgct 720
   gctatcatcc atcctaatct gcaggatcaa gactggaaac cactgcatcc tggggatccc 780
   atgtttttaa ctcttgatgg gaagacgatc ccactgggcg gagactgtac cgtgtacccc 840
   gtgtttgtga atgaggccgc atattacgaa aagaaagaag cttttgcaaa gacaactaaa 900
   ctaacgctca atgcaaaaag tattcgctgc tgtttacatt ag                    942
<210> 101
   <211> 1590
   <212> DNA
   <213> Homo sapiens
<400> 101
   atgacaagct ccaggctttg gttttcgctg ctgctggcgg cagcgttcgc aggacgggcg 60
   acggccctct ggccctggcc tcagaacttc caaacctccg accagcgcta cgtcctttac 120
   ccgaacaact ttcaattcca gtacgatgtc agctcggccg cgcagcccgg ctgctcagtc 180
   ctcgacgagg ccttccagcg ctatcgtgac ctgcttttcg gttccgggtc ttggccccgt 240
   ccttacctca cagggaaacg gcatacactg gagaagaatg tgttggttgt ctctgtagtc 300
   acacctggat gtaaccagct tcctactttg gagtcagtgg agaattatac cctgaccata 360
   aatgatgacc agtgtttact cctctctgag actgtctggg gagctctccg aggtctggag 420
   acttttagcc agcttgtttg gaaatctgct gagggcacat tctttatcaa caagactgag 480
   attgaggact ttccccgctt tcctcaccgg ggcttgctgt tggatacatc tcgccattac 540
   ctgccactct ctagcatcct ggacactctg gatgtcatgg cgtacaataa attgaacgtg 600
   ttccactggc atctggtaga tgatccttcc ttcccatatg agagcttcac ttttccagag 660
   ctcatgagaa aggggtccta caaccctgtc acccacatct acacagcaca ggatgtgaag 720
   gaggtcattg aatacgcacg gctccggggt atccgtgtgc ttgcagagtt tgacactcct 780
   ggccacactt tgtcctgggg accaggtatc cctggattac tgactccttg ctactctggg 840
   tctgagccct ctggcacctt tggaccagtg aatcccagtc tcaataatac ctatgagttc 900
   atgagcacat tcttcttaga agtcagctct gtcttcccag atttttatct tcatcttgga 960
   ggagatgagg ttgatttcac ctgctggaag tccaacccag agatccagga ctttatgagg 1020
   aagaaaggct tcggtgagga cttcaagcag ctggagtcct tctacatcca gacgctgctg 1080
   gacatcgtct cttcttatgg caagggctat gtggtgtggc aggaggtgtt tgataataaa 1140
   gtaaagattc agccagacac aatcatacag gtgtggcgag aggatattcc agtgaactat 1200
   atgaaggagc tggaactggt caccaaggcc ggcttccggg cccttctctc tgccccctgg 1260
   tacctgaacc gtatatccta tggccctgac tggaaggatt tctacgtagt ggaacccctg 1320
   gcatttgaag gtacccctga gcagaaggct ctggtgattg gtggagaggc ttgtatgtgg 1380
   ggagaatatg tggacaacac aaacctggtc cccaggctct ggcccagagc aggggctgtt 1440
   gccgaaaggc tgtggagcaa caagttgaca tctgacctga catttgccta tgaacgtttg 1500
   tcacacttcc gctgtgagtt gctgaggcga ggtgtccagg cccaacccct caatgtaggc 1560
   ttctgtgagc aggagtttga acagacctga                                  1590
<210> 102
   <211> 657
   <212> DNA
   <213> Homo sapiens
<400> 102
   atggcgaccc gcagccctgg cgtcgtgatt agtgatgatg aaccaggtta tgaccttgat 60
   ttattttgca tacctaatca ttatgctgag gatttggaaa gggtgtttat tcctcatgga 120
   ctaattatgg acaggactga acgtcttgct cgagatgtga tgaaggagat gggaggccat 180
   cacattgtag ccctctgtgt gctcaagggg ggctataaat tctttgctga cctgctggat 240
   tacatcaaag cactgaatag aaatagtgat agatccattc ctatgactgt agattttatc 300
   agactgaaga gctattgtaa tgaccagtca acaggggaca taaaagtaat tggtggagat 360
   gatctctcaa ctttaactgg aaagaatgtc ttgattgtgg aagatataat tgacactggc 420
   aaaacaatgc agactttgct ttccttggtc aggcagtata atccaaagat ggtcaaggtc 480
   gcaagcttgc tggtgaaaag gaccccacga agtgttggat ataagccaga ctttgttgga 540
   tttgaaattc cagacaagtt tgttgtagga tatgcccttg actataatga atacttcagg 600
   gatttgaatc atgtttgtgt cattagtgaa actggaaaag caaaatacaa agcctaa    657
<210> 103
   <211> 1956
   <212> DNA
   <213> Homo sapiens
<400> 103
   atggcccggg ggtcggcggt tgcctgggcg gcgctcgggc cgttgttgtg gggctgcgcg 60
   ctggggctgc agggcgggat gctgtacccc caggagagcc cgtcgcggga gtgcaaggag 120
   ctggacggcc tctggagctt ccgcgccgac ttctctgaca accgacgccg gggcttcgag 180
   gagcagtggt accggcggcc gctgtgggag tcaggcccca ccgtggacat gccagttccc 240
   tccagcttca atgacatcag ccaggactgg cgtctgcggc attttgtcgg ctgggtgtgg 300
   tacgaacggg aggtgatcct gccggagcga tggacccagg acctgcgcac aagagtggtg 360
   ctgaggattg gcagtgccca ttcctatgcc atcgtgtggg tgaatggggt cgacacgcta 420
   gagcatgagg ggggctacct ccccttcgag gccgacatca gcaacctggt ccaggtgggg 480
   cccctgccct cccggctccg aatcactatc gccatcaaca acacactcac ccccaccacc 540
   ctgccaccag ggaccatcca atacctgact gacacctcca agtatcccaa gggttacttt 600
   gtccagaaca catattttga ctttttcaac tacgctggac tgcagcggtc tgtacttctg 660
   tacacgacac ccaccaccta catcgatgac atcaccgtca ccaccagcgt ggagcaagac 720
   agtgggctgg tgaattacca gatctctgtc aagggcagta acctgttcaa gttggaagtg 780
   cgtcttttgg atgcagaaaa caaagtcgtg gcgaatggga ctgggaccca gggccaactt 840
   aaggtgccag gtgtcagcct ctggtggccg tacctgatgc acgaacgccc tgcctatctg 900
   tattcattgg aggtgcagct gactgcacag acgtcactgg ggcctgtgtc tgacttctac 960
   acactccctg tggggatccg cactgtggct gtcaccaaga gccagttcct catcaatggg 1020
   aaacctttct atttccacgg tgtcaacaag catgaggatg cggacatccg agggaagggc 1080
   ttcgactggc cgctgctggt gaaggacttc aacctgcttc gctggcttgg tgccaacgct 1140
   ttccgtacca gccactaccc ctatgcagag gaagtgatgc agatgtgtga ccgctatggg 1200
   attgtggtca tcgatgagtg tcccggcgtg ggcctggcgc tgccgcagtt cttcaacaac 1260
   gtttctctgc atcaccacat gcaggtgatg gaagaagtgg tgcgtaggga caagaaccac 1320
   cccgcggtcg tgatgtggtc tgtggccaac gagcctgcgt cccacctaga atctgctggc 1380
   tactacttga agatggtgat cgctcacacc aaatccttgg acccctcccg gcctgtgacc 1440
   tttgtgagca actctaacta tgcagcagac aagggggctc cgtatgtgga tgtgatctgt 1500
   ttgaacagct actactcttg gtatcacgac tacgggcacc tggagttgat tcagctgcag 1560
   ctggccaccc agtttgagaa ctggtataag aagtatcaga agcccattat tcagagcgag 1620
   tatggagcag aaacgattgc agggtttcac caggatccac ctctgatgtt cactgaagag 1680
   taccagaaaa gtctgctaga gcagtaccat ctgggtctgg atcaaaaacg cagaaaatac 1740
   gtggttggag agctcatttg gaattttgcc gatttcatga ctgaacagtc accgacgaga 1800
   gtgctgggga ataaaaaggg gatcttcact cggcagagac aaccaaaaag tgcagcgttc 1860
   cttttgcgag agagatactg gaagattgcc aatgaaacca ggtatcccca ctcagtagcc 1920
   aagtcacaat gtttggaaaa cagcccgttt acttga                         1956
<210> 104
   <211> 1671
   <212> DNA
   <213> Homo sapiens
<400> 104
   atggagctgt gcgggctggg gctgccccgg ccgcccatgc tgctggcgct gctgttggcg 60
   acactgctgg cggcgatgtt ggcgctgctg actcaggtgg cgctggtggt gcaggtggcg 120
   gaggcggctc gggccccgag cgtctcggcc aagccggggc cggcgctgtg gcccctgccg 180
   ctctcggtga agatgacccc gaacctgctg catctcgccc cggagaactt ctacatcagc 240
   cacagcccca attccacggc gggcccctcc tgcaccctgc tggaggaagc gtttcgacga 300
   tatcatggct atatttttgg tttctacaag tggcatcatg aacctgctga attccaggct 360
   aaaacccagg ttcagcaact tcttgtctca atcacccttc agtcagagtg tgatgctttc 420
   cccaacatat cttcagatga gtcttatact ttacttgtga aagaaccagt ggctgtcctt 480
   aaggccaaca gagtttgggg agcattacga ggtttagaga cctttagcca gttagtttat 540
   caagattctt atggaacttt caccatcaat gaatccacca ttattgattc tccaaggttt 600
   tctcacagag gaattttgat tgatacatcc agacattatc tgccagttaa gattattctt 660
   aaaactctgg atgccatggc ttttaataag tttaatgttc ttcactggca catagttgat 720
   gaccagtctt tcccatatca gagcatcact tttcctgagt taagcaataa aggaagctat 780
   tctttgtctc atgtttatac accaaatgat gtccgtatgg tgattgaata tgccagatta 840
   cgaggaattc gagtcctgcc agaatttgat acccctgggc atacactatc ttggggaaaa 900
   ggtcagaaag acctcctgac tccatgttac agtagacaaa acaagttgga ctcttttgga 960
   cctataaacc ctactctgaa tacaacatac agcttcctta ctacattttt caaagaaatt 1020
   agtgaggtgt ttccagatca attcattcat ttgggaggag atgaagtgga atttaaatgt 1080
   tgggaatcaa atccaaaaat tcaagatttc atgaggcaaa aaggctttgg cacagatttt 1140
   aagaaactag aatctttcta cattcaaaag gttttggata ttattgcaac cataaacaag 1200
   ggatccattg tctggcagga ggtttttgat gataaagcaa agcttgcgcc gggcacaata 1260
   gttgaagtat ggaaagacag cgcatatcct gaggaactca gtagagtcac agcatctggc 1320
   ttccctgtaa tcctttctgc tccttggtac ttagatttga ttagctatgg acaagattgg 1380
   aggaaatact ataaagtgga acctcttgat tttggcggta ctcagaaaca gaaacaactt 1440
   ttcattggtg gagaagcttg tctatgggga gaatatgtgg atgcaactaa cctcactcca 1500
   agattatggc ctcgggcaag tgctgttggt gagagactct ggagttccaa agatgtcaga 1560
   gatatggatg acgcctatga cagactgaca aggcaccgct gcaggatggt cgaacgtgga 1620
   atagctgcac aacctcttta tgctggatat tgtaaccatg agaacatgta a          1671
<210> 105
   <211> 1290
   <212> DNA
   <213> Homo sapiens
<400> 105
   atgcagctga ggaacccaga actacatctg ggctgcgcgc ttgcgcttcg cttcctggcc 60
   ctcgtttcct gggacatccc tggggctaga gcactggaca atggattggc aaggacgcct 120
   accatgggct ggctgcactg ggagcgcttc atgtgcaacc ttgactgcca ggaagagcca 180
   gattcctgca tcagtgagaa gctcttcatg gagatggcag agctcatggt ctcagaaggc 240
   tggaaggatg caggttatga gtacctctgc attgatgact gttggatggc tccccaaaga 300
   gattcagaag gcagacttca ggcagaccct cagcgctttc ctcatgggat tcgccagcta 360
   gctaattatg ttcacagcaa aggactgaag ctagggattt atgcagatgt tggaaataaa 420
   acctgcgcag gcttccctgg gagttttgga tactacgaca ttgatgccca gacctttgct 480
   gactggggag tagatctgct aaaatttgat ggttgttact gtgacagttt ggaaaatttg 540
   gcagatggtt ataagcacat gtccttggcc ctgaatagga ctggcagaag cattgtgtac 600
   tcctgtgagt ggcctcttta tatgtggccc tttcaaaagc ccaattatac agaaatccga 660
   cagtactgca atcactggcg aaattttgct gacattgatg attcctggaa aagtataaag 720
   agtatcttgg actggacatc ttttaaccag gagagaattg ttgatgttgc tggaccaggg 780
   ggttggaatg acccagatat gttagtgatt ggcaactttg gcctcagctg gaatcagcaa 840
   gtaactcaga tggccctctg ggctatcatg gctgctcctt tattcatgtc taatgacctc 900
   cgacacatca gccctcaagc caaagctctc cttcaggata aggacgtaat tgccatcaat 960
   caggacccct tgggcaagca agggtaccag cttagacagg gagacaactt tgaagtgtgg 1020
   gaacgacctc tctcaggctt agcctgggct gtagctatga taaaccggca ggagattggt 1080
   ggacctcgct cttataccat cgcagttgct tccctgggta aaggagtggc ctgtaatcct 1140
   gcctgcttca tcacacagct cctccctgtg aaaaggaagc tagggttcta tgaatggact 1200
   tcaaggttaa gaagtcacat aaatcccaca ggcactgttt tgcttcagct agaaaataca 1260
   atgcagatgt cattaaaaga cttactttaa                                 1290
<210> 106
   <211> 1611
   <212> DNA
   <213> Homo sapiens
<400> 106
<210> 107
   <211> 642
   <212> DNA
   <213> Homo sapiens
<400> 107
   atgccccgga gggcggagaa ctgggacgag gccgaggtag gcgcggagga ggcaggcgtc 60
   gaagagtacg gccctgaaga agacggcggg gaggagtcgg gcgccgagga gtccggcccg 120
   gaagagtccg gcccggagga actgggcgcc gaggaggaga tggaggccgg gcggccgcgg 180
   cccgtgctgc gctcggtgaa ctcgcgcgag ccctcccagg tcatcttctg caatcgcagt 240
   ccgcgcgtcg tgctgcccgt atggctcaac ttcgacggcg agccgcagcc ctacccaacg 300
   ctgccgcctg gcacgggccg ccgcatccac agctaccgag gtcacctttg gctcttcaga 360
   gatgcaggga cacacgatgg gcttctggtt aaccaaactg aattatttgt gccatctctc 420
   aatgttgacg gacagcctat ttttgccaat atcacactgc cagtgtatac tctgaaagag 480
   cgatgcctcc aggttgtccg gagcctagtc aagcctgaga attacaggag actggacatc 540
   gtcaggtcgc tctacgaaga tctggaagac cacccaaatg tgcagaaaga cctggagcgg 600
   ctgacacagg agcgcattgc acatcaacgg atgggagatt ga                   642
<210> 108
   <211> 444
   <212> DNA
   <213> Homo sapiens
<400> 108
   atggtgcatc tgactcctga ggagaagtct gccgttactg ccctgtgggg caaggtgaac 60
   gtggatgaag ttggtggtga ggccctgggc aggctgctgg tggtctaccc ttggacccag 120
   aggttctttg agtcctttgg ggatctgtcc actcctgatg ctgttatggg caaccctaag 180
   gtgaaggctc atggcaagaa agtgctcggt gcctttagtg atggcctggc tcacctggac 240
   aacctcaagg gcacctttgc cacactgagt gagctgcact gtgacaagct gcacgtggat 300
   cctgagaact tcaggctcct gggcaacgtg ctggtctgtg tgctggccca tcactttggc 360
   aaagaattca ccccaccagt gcaggctgcc tatcagaaag tggtggctgg tgtggctaat 420
   gccctggccc acaagtatca ctaa                                       444
<210> 109
   <211> 951
   <212> DNA
   <213> Homo sapiens
<400> 109
   atgatagtgt ttgtcaggtt caactccagc catggtttcc cagtggaggt cgattctgac 60
   accagcatct tccagctcaa ggaggtggtt gctaagcgac agggggttcc ggctgaccag 120
   ttgcgtgtga ttttcgcagg gaaggagctg aggaatgact ggactgtgca ggaatttttc 180
   tttaaatgtg gagcacaccc cacctctgac aaggaaacat cagtagcttt gcacctgatc 240
   gcaacaaata gtcggaacat cacttgcatt acgtgcacag acgtcaggag ccccgtcctg 300
   gttttccagt gcaactcccg ccacgtgatt tgcttagact gtttccactt atactgtgtg 360
   acaagactca atgatcggca gtttgttcac gaccctcaac ttggctactc cctgccttgt 420
   gtggctggct gtcccaactc cttgattaaa gagctccatc acttcaggat tctgggagaa 480
   gagcagtaca accggtacca gcagtatggt gcagaggagt gtgtcctgca gatggggggc 540
   gtgttatgcc cccgccctgg ctgtggagcg gggctgctgc cggagcctga ccagaggaaa 600
   gtcacctgcg aagggggcaa tggcctgggc tgtgggtttg ccttctgccg ggaatgtaaa 660
   gaagcgtacc atgaagggga gtgcagtgcc gtatttgaag cctcaggaac aactactcag 720
   gcctacagag tcgatgaaag agccgccgag caggctcgtt gggaagcagc ctccaaagaa 780
   accatcaaga aaaccaccaa gccctgtccc cgctgccatg taccagtgga aaaaaatgga 840
   ggctgcatgc acatgaagtg tccgcagccc cagtgcaggc tcgagtggtg ctggaactgt 900
   ggctgcgagt ggaaccgcgt ctgcatgggg gaccactggt tcgacgtgta g          951

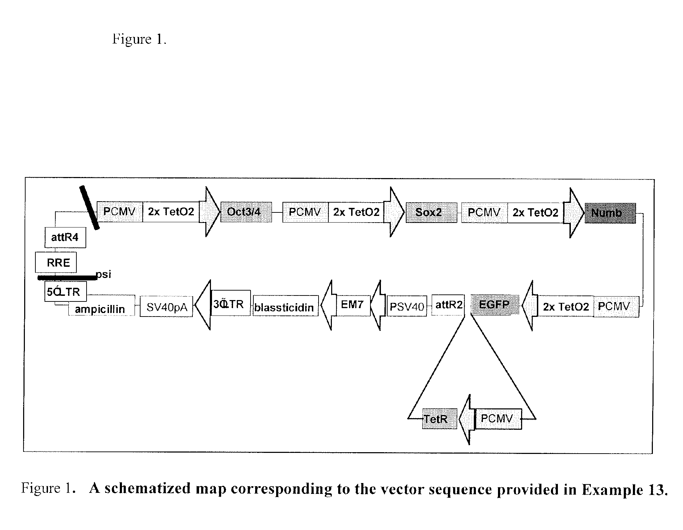

## Claims

1. A method for producing multipotent, pluripotent, and/or self-renewing cells from the selected cells, comprising:
transfecting selected cells obtained by selecting cells from the group of somatic cells with nucleotide sequences encoding the "long" (PRR insert +) isoform(s) of the mammalian numb protein and with an additional nucleotide sequence encoding Notch; and
growing the selected cells in a growth medium comprising cytokines selected from EGF, bFGF, LIF, steel factor, IL-6, hyper IL-6, IL-7, oncostatin-M, cardiotrophin-1 and other growth enhancing cytokines effective that the selected cells expand at a rate, wherein said rate is determined from the doubling times of the selected cells in said growth culture medium; and maintaining the cells under these culture conditions until the desired cell number is achieved.

## Patentansprüche

1. Verfahren zur Herstellung von multipotenten, pluripotenten und/oder sich selbst erneuernden Zellen aus die ausgewählten Zellen, umfassend:
Transfektion von ausgewählten Zellen, die durch die Auswahl von Zellen aus der Gruppe von Körperzellen mit Nukleotidsequenzen, die die "lange" ("langen") (PRR Einsatz +) Isoform(en) von einem NUMB-Säugerprotein kodieren, und mit einer weiteren Nukleotidsequenz, die Notch kodiert, erhalten sind; und
Züchtung der ausgewählten Zellen in einem Züchtungsmedium, umfassend Zytokine, die aus EGF, bFGF, LIF, Stahlfaktor, IL-6, Hyper-IL-6, IL-7, Onkostatin-M, Kardiotrofin-1 und anderen Züchtung erhöhenden Zytokinen gewählt sind und dazu beitragen, dass die ausgewählten Zellen bei einer Geschwindigkeit expandieren, wobei diese Geschwindigkeit aufgrund der Verdopplungszeit der ausgewählten Zellen im Züchtungsmedium definiert ist; und Verweilen von den Zellen unter diesen Kulturbedingungen bis zum Erhalten der gewünschten Zellenanzahl.

## Revendications

1. Procédé de production des cellules multipotentes, pluripotentes et / ou auto-renouvellantes à partir de cellules sélectionnées, comprenant:
transfection des cellules sélectionnées obtenues en sélectionnant les cellules parmi un groupe de cellules somatiques avec des séquences de nucléotides codant pour une ou plusieurs isoformes "longues" (PRR insert +) de la protéine Numb de mammifère et avec des séquences de nucléotides additionnelles codant pour Notch; et
cultivation les cellules sélectionnées dans un milieu de culture comprenant des cytokines sélectionnées parmi le EGF, le bFGF, le LIF, le Steel facteur, l'IL-6, l'hyper IL-6, IL-7, la oncostatine-M, la cardiotrophine-1 et d'autres cytokines favorisant la cultivation efficace que les cellules sélectionnées se propagent à une certaine vitesse, où ladite vitesse est déterminée à partir des temps de doublement des cellules sélectionnées dans ledit milieu de culture; et
maintien des cellules dans ces conditions de culture jusqu'à ce que le nombre de cellules désiré soit atteint.
